# EUROPEAN PATENT APPLICATION

(11) **EP 3 141 258 A1**
(43) Date of publication of application: **15.03.2017**
(21) Application number: 15184199.6
(22) Date of filing: 08.09.2015
(51) Int. Cl.: A61K 38/16, C07K 14/415, C07K 16/16

(54) **SOYBEAN ALLERGY RELATED EPITOPES**

(71) Applicant: Fraunhofer-Gesellschaft zur Förderung der angewandten Forschung e.V., 80686 München (DE)
(72) Inventor: Kern, Karolin, 06114 Halle (DE); Spiegel, Holger, 52074 Aachen (DE); Havenith, Heide, 52064 Aachen (DE); Szardenings, Michael, 04299 Leipzig (DE)
(74) Representative: PATERIS Patentanwälte PartmbB

(57) **Abstract**

The invention relates to a compilation comprising at least five different peptides, each peptide comprising at least one sequence element corresponding to an epitope selected from the group consisting of SEQ ID NO.: 1 - 354. The invention further relates to an *in vitro* method for determining a patient's immune status to soybean allergens, to a method for detecting at least one soybean allergen in a substance and to a method for determining the allergenicity of a soybean variety. Additionally, the invention relates to a kit comprising at least one composition containing a compound comprising at least two identical and/or different sequence elements each corresponding to an epitope selected from the group consisting of SEQ ID NO.: 1 - 354. Furthermore, the invention relates to the use of a peptide comprising a sequence element corresponding to an epitope for providing a molecule binding to a protein or peptide comprising the epitope.

## Description

### Field of the Invention

The invention relates to a compilation comprising at least five different peptides, each peptide comprising at least one sequence element corresponding to an epitope selected from the group consisting of SEQ ID NO.: 1 - 354. The invention further relates to an *in vitro* method for determining a patient's immune status to soybean allergens, to a method for detecting at least one soybean allergen in a substance and to a method for determining the allergenicity of a soybean variety. Additionally, the invention relates to a kit comprising at least one composition containing a compound comprising at least two identical and/or different sequence elements each corresponding to an epitope selected from the group consisting of SEQ ID NO.: 1 - 354. Furthermore, the invention relates to the use of a peptide comprising a sequence element corresponding to an epitope for providing a molecule binding to a protein or peptide comprising the epitope.

### Background of the Invention

Within the last decades, soybean seeds have become an important source of protein in many countries of the world. In developing countries, soybean is increasingly used to provide sufficient nutrition to the population, whereas in industrial countries it has become a major ingredient of processed food products. Moreover, soybean food products are specifically promoted for having health benefits and are believed to lower incidences of high plasma cholesterol, cancer, diabetes mellitus and obesity. Indeed, soybean products provide all essential amino acids and are rich in vitamins and minerals, making them an interesting source for protein rich nutrition. Despite these advantages, soybean is one of the most allergenic foods. Soybean allergies usually arise spontaneously during childhood with symptoms such as atopic dermatitis, enterocolitis and other IgE-mediated multisystem reactions (Masilamani et al., 2012). Moreover, they often occur together with allergies to other food products, in particular peanut and milk protein (Masilamani et al., 2012). Thus, affected patients not only have to cope with the risk of allergic reactions for almost their entire life but need to be particularly careful about their diet in general.

Although the reaction threshold of soybean protein (400 mg) is rather high compared to peanut (0.1 mg), most sensitive persons need to avoid soybean proteins completely to prevent allergic reaction (Masilamani et al., 2012). Unfortunately, obviating soybean protein containing food products is increasingly difficult, since soybean protein became an ubiquitous ingredient of vegetable as well as meat containing foods. In particular processed or partially processed food products usually contain at least traces of at least some soybean proteins. To increase security for persons suffering from allergies, governments, in particular within the European Union and the United States of America, implement increasing requirements regarding the identification of food ingredients. To meet these requirements, food producing companies need comprehensive and reliable methods for analyzing the content of their food products.

Additionally, patients need to know about the primary cause and extent of their allergic reaction to be able to benefit from the increasing information provided about food product ingredients.

Therefore, an unmet need exists for improved products and methods to determine the sensitivity of patients to soybean protein as well as to identify the presence of soybean proteins and their allergenic potential in food and other products.

### Summary of the Invention

In a first aspect, the invention relates to a compilation comprising at least five different peptides, each peptide comprising at least one sequence element corresponding to an epitope selected from the group consisting of SEQ ID NO.: 1 - 354.

In a further aspect, the invention relates to an *in vitro* method for determining a patient's immune status to soybean allergens, comprising the steps providing at least five different peptides, each peptide comprising at least one sequence element corresponding to an epitope selected from the group consisting of SEQ ID NO.: 1 - 354, contacting each peptide with a sample comprising antibodies derived from the patient, and detecting an interaction of each peptide with the sample.

In a further aspect, the invention relates to a kit comprising at least one composition containing a compound comprising at least two identical and/or different sequence elements each corresponding to an epitope selected from the group consisting of SEQ ID NO.: 1 - 354.

In a further aspect, the invention relates to a method for detecting at least one soybean allergen in a substance, comprising the steps providing at least two different peptides, each peptide comprising at least one sequence element corresponding to an epitope selected from the group consisting of SEQ ID NO.: 1 - 354, raising at least one antibody against each peptide, contacting the substance with the antibodies, and determining binding of the antibodies to the substance.

In a further aspect, the invention relates to a method for producing an immunoassay product, comprising the steps providing at least two different peptides, each peptide comprising at least one sequence element corresponding to an epitope selected from the group consisting of SEQ ID NO.: 1 - 354, raising at least one antibody against each peptide, and compiling the antibodies to provide the immunoassay product.

In a further aspect, the invention relates to a method for determining the allergenicity of a soybean variety by detecting the presence of at least two epitopes in a sample of the variety, wherein the epitopes are selected from the group consisting of SEQ ID NO.: 1 - 354.

In a further aspect, the invention relates to the use of a peptide comprising a sequence element corresponding to an epitope selected from the group consisting of SEQ ID NO.: 1 - 354 for providing a molecule binding to a protein or peptide comprising the epitope.

In a further aspect, the invention relates to a method for generating a variant of an allergenic soybean protein having a reduced allergenic potential, comprising the steps providing an amino acid sequence of at least one epitope selected from the group consisting of SEQ ID NO.: 1 - 354, altering the amino acid sequence of the at least one epitope as to eliminating the structure of the epitope, and generating a protein or peptide comprising the altered amino acid sequence.

### Brief Description of the Drawings

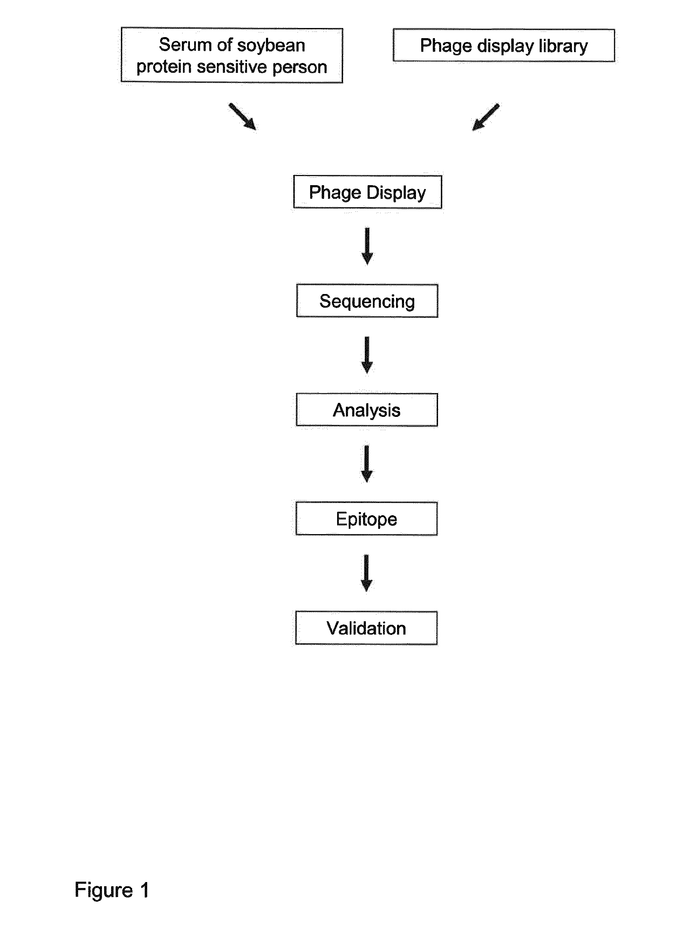

Figure 1 depicts the procedure of identifying epitopes responsible for immunologic reactions to soybean protein.

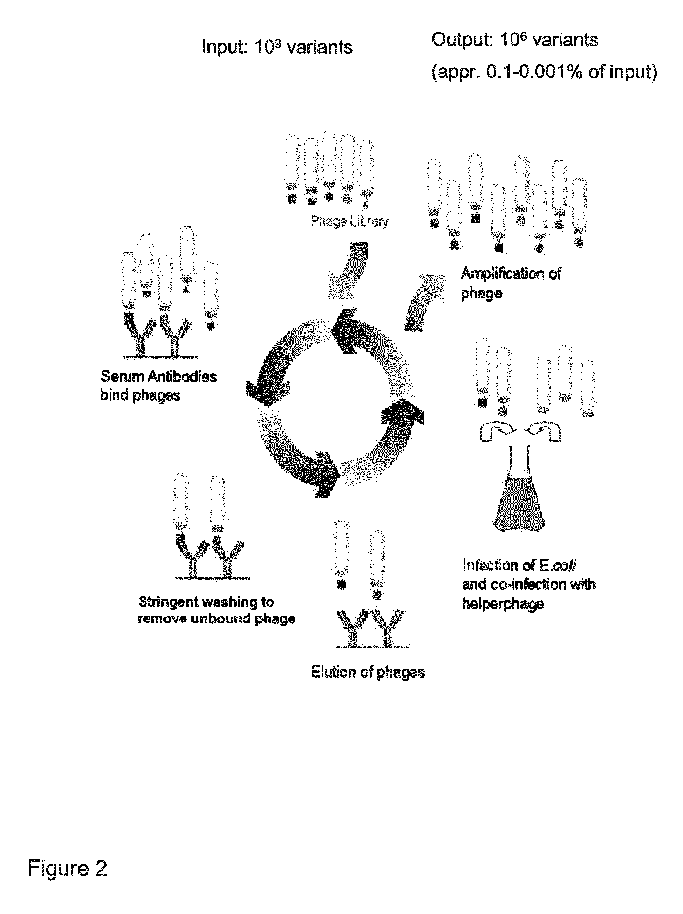

Figure 2 depicts the phage display procedure used for identifying epitopes.

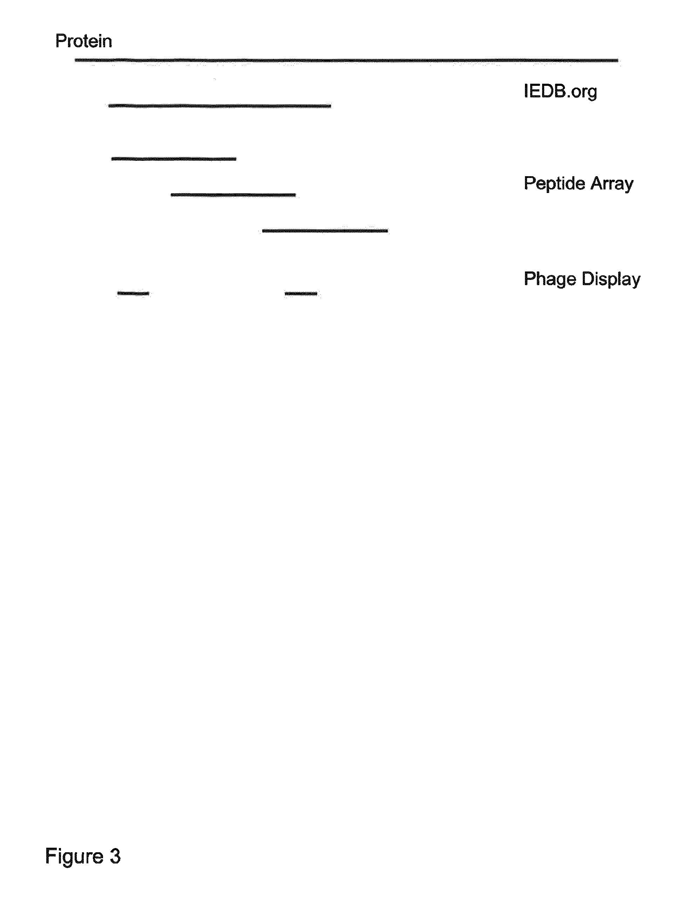

Figure 3 depicts the size of allergy relevant sequences of soybean proteins identified by various techniques in relation to the size of an entire soybean protein. Whereas the free epitope data base (www.IEDB.org) and arrays of synthetic peptides for screening protein sequences provide sequences of about 10 to 20 and even more amino acids, specific core sequences for anti-Allergen Antibody binding peptides of usually 4 to 6 amino acids were identified by phage display.

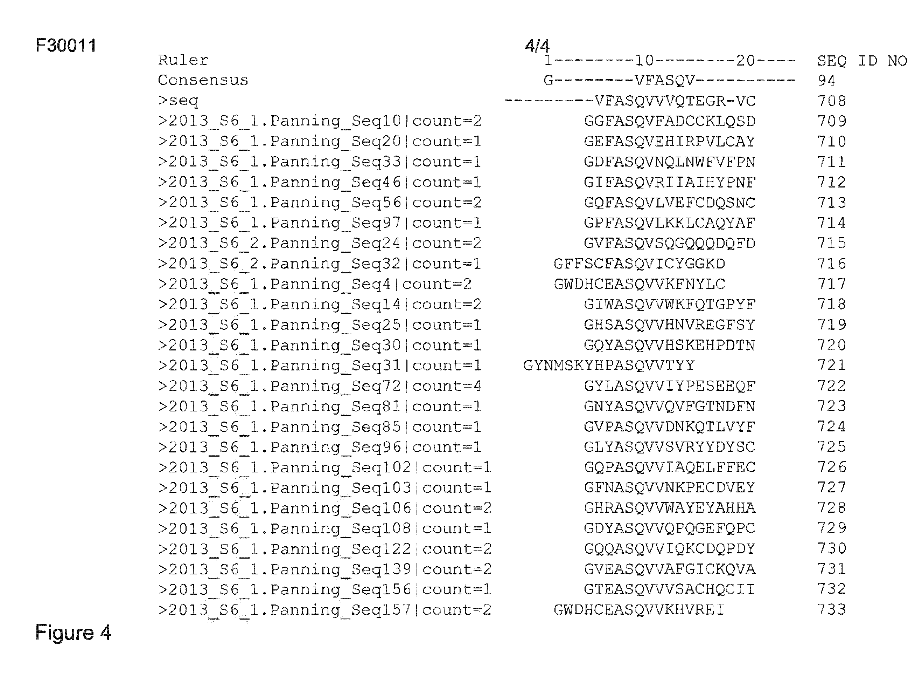

Figure 4 shows the identification of an epitope. "Seq" refers to the original sequence tested in PEPper-PRINT Array. All sequences identified from Serum 21 (only a subset shown) share the common motive "VFASQV (SEQ ID NO.: 94). The motifs variability is shown by different sequences bound by serum 21.

### Detailed Description of the Invention

In a first aspect, the invention relates to a compilation comprising at least five different peptides, each peptide comprising at least one sequence element corresponding to an epitope selected from the group consisting of SEQ ID NO.: 1 - 354.

Several proteins of soybean seeds have been found to trigger immunoglobulin E (IgE) mediated allergic responses in sensitive persons. Strong immunodominant allergic proteins, such as P34 thiol protease (Gly m Bd 30K), are known to induce allergic reactions, whereas other soybean proteins, although no potent inducers of a primary allergic response, can nevertheless trigger effector responses due to their binding by IgE. Interestingly, affected persons show varying sensitivity to different soybean proteins. For example, only 65 % of soybean sensitive individuals carry IgE recognizing P34 thiol protease (Gly m Bd 30K), which is considered one of the strongest allergenic soybean proteins (Yang et al., 2011). Finally, as antibodies usually do not recognize an entire protein but interact with distinct structures of limited numbers of amino acids within a molecule, sensitivity between patients varies distinctly due to their genetic background and different results of antibody maturation. To identify many specific epitopes, i.e. amino acid sequences of soybean proteins, recognized by antibodies of sensitive patients, with high resolution down to the individual amino acids involved, the inventors applied a novel approach using a specific random phage library (EP 14 166 662.8). This phage library is generated using triplet codons with specific limitations, such that most amino acids are regularly represented throughout the entire library. In consequence, the library covers an unusually broad range of sequence variability and allows particular reliable statistical analysis. The library was contacted with serum samples derived from a biobank of 50 different soybean protein sensitive patients. After phage selection, the amino acid sequences that were bound by the patients' antibodies (anti-[allergen antibody] amino acid sequence) were identified by next generation sequencing and the results statistically analyzed. To confirm the allergy relevance of the identified amino acid sequences, their reactivity with patient IgE was determined by peptide arrays. Thereby, the inventors identified more than 300 potentially allergenic epitopes from different soybean proteins. The identified epitopes could be assigned to a total of 23 soybean proteins (Table 1) including P34 thiol protease (Gly m Bd 30K), β-conglycinin (Gly m Bd60 K) and Gly m Bd 28K, which are considered major soybean allergenic proteins (Yang et al., 2011).

**Table 1: Individual Epitopes**

| Soybean allergen (ID) | Epitop NO | Epitop sequence | SEQ ID NO. |
|---|---|---|---|
| beta conglycinin alpha chain (X17698) | 6.04.001 | SNRFE | 1 |
| | 6.04.002 | FKNQYG | 2 |
| | 6.04.003 | FNSKPN | 3 |
| | 6.04.004 | VNPDNN | 4 |
| | 6.04.005 | LAIPV | 5 |
| | 6.04.006 | NXPGRFES | 6 |
| | 6.04.007 | EASYD | 7 |
| | 6.04.008 | EQQQGE | 8 |
| | 6.04.009 | QESVI | 9 |
| | 6.04.010 | KSSSRK | 10 |
| | 6.04.011 | PEKNPQ | 11 |
| | 6.04.012 | FVIPAG | 12 |
| | 6.04.013 | AFPGS | 13 |
| | 6.04.014 | CLQSCNS | 14 |
| | 6.04.015 | NSERD | 15 |
| | 6.04.016 | ERDSYR | 16 |
| | 6.04.017 | RNQACHA | 17 |
| | 6.04.018 | QACHAR | 18 |
| | 6.04.019 | KEECEEG | 19 |
| | 6.04.020 | EIPRPR | 20 |
| | 6.04.021 | KEECEE | 21 |
| | 6.04.022 | RPQHPEREP | 22 |
| | 6.04.023 | REPQQPGE | 23 |
| | 6.04.024 | HPEREP | 24 |
| | 6.04.025 | QQPGEKE | 25 |
| | 6.04.026 | EDEDEQPRP | 26 |
| | 6.04.027 | IPFPRPQP | 27 |
| | 6.04.028 | PRQEEE | 28 |
| | 6.04.029 | EEHEQREEQ | 29 |
| | 6.04.030 | GEKGSEE | 30 |
| | 6.04.031 | RNEEEDE | 31 |
| | 6.04.032 | EDEEQQ | 32 |
| | 6.04.033 | EEQQRES | 33 |
| | 6.04.034 | RESEES | 34 |
| | 6.04.035 | VLFSREEG | 35 |
| | 6.04.036 | EEGQQQG | 36 |
| | 6.04.037 | LRSRDPIY | 37 |
| | 6.04.038 | LSIVD | 38 |
| | 6.04.039 | LKEQQQE | 39 |
| | 6.04.040 | EQQQE | 40 |
| | 6.04.041 | EEQPLE | 41 |
| | 6.04.042 | NQRESYFV | 42 |
| | 6.04.043 | FVDAQP | 43 |
| beta conglycinin alpha' chain (M13759.1) | 7.03.001 | GVVFL | 44 |
| | 7.03.002 | RFQTL | 45 |
| | 7.03.003 | PQLRD | 46 |
| | 7.03.005 | QPHQK | 47 |
| | 7.03.007 | DRDSY | 48 |
| | 7.03.008 | LRVPA | 49 |
| | 7.03.009 | KVEEEE | 50 |
| | 7.03.010 | PERERQ | 51 |
| | 7.03.011 | QQHGEK | 52 |
| | 7.03.012 | EDEGEQP | 53 |
| | 7.03.013 | EGEQPRPF | 54 |
| | 7.03.014 | FPRPRQP | 55 |
| | 7.03.015 | QPHQEE | 56 |
| | 7.03.016 | EQKEEH | 57 |
| | 7.03.017 | KEEHE | 58 |
| | 7.03.018 | SEEEQDERE | 59 |
| | 7.03.019 | HKQEKH | 60 |
| | 7.03.020 | ESEEE | 61 |
| | 7.03.021 | EEEDQDE | 62 |
| | 7.03.022 | EEDQDEDEEQ | 63 |
| | 7.03.023 | ESQESEGSE | 64 |
| | 7.03.024 | ESQREP | 65 |
| | 7.03.025 | NKRSQQLQ | 66 |
| | 7.03.026 | LPHHAD | 67 |
| | 7.03.027 | GREEGQ | 68 |
| | 7.03.028 | VLVINE | 69 |
| | 7.03.029 | IELVGI | 70 |
| | 7.03.030 | QDIFVIP | 71 |
| | 7.03.031 | DAQPQQ | 72 |
| | 7.03.032 | LEVRKYR | 73 |
| | 7.03.033 | ESYFVD | 74 |
| | 7.03.034 | FVDAQP | 75 |
| beta conglycinin beta chain (S44893) | 8.04.001 | FLASV | 76 |
| | 8.04.002 | LSGRAI | 77 |
| | 8.04.005 | HQNLK | 78 |
| | 8.04.006 | KPGRYD | 79 |
| | 8.04.007 | ELSKEQ | 80 |
| | 8.04.010 | IERQVQ | 81 |
| | 8.04.011 | PQLENL | 82 |
| | 8.04.012 | DSYNLH | 83 |
| | 8.04.013 | PGDAQ | 84 |
| | 8.04.014 | SHNILE | 85 |
| | 8.04.015 | SFHSEFEE | 86 |
| | 8.04.016 | EEINRV | 87 |
| | 8.04.017 | RVLFGE | 88 |
| | 8.04.018 | QRQQE | 89 |
| | 8.04.019 | QEGVIV | 90 |
| | 8.04.020 | ILVINE | 91 |
| | 08.04.2008 | PHFNSK | 92 |
| | 08.04.2009 | EEEPLE | 93 |
| Defensin (Z13956.1) | 10.00.01 | VFASQV | 94 |
| | 10.00.02 | VVVQTE | 95 |
| | 10.00.03 | TEGRVC | 96 |
| | 10.00.04 | RVCESQS | 97 |
| | 10.00.05 | QSHGF | 98 |
| | 10.00.06 | SQSHGFH | 99 |
| | 10.00.07 | HGLCNRDHN | 100 |
| | 10.00.08 | RDHNCALVC | 101 |
| | 10.00.09 | LVCRNE | 102 |
| | 10.00.10 | SRRCF | 103 |
| Gly m BD 28K (AB046874.2) | 11.00.01 | QEEDEE | 104 |
| Glycinin G1 (AB113349.1) | 12.00.01 | QCAGVA | 105 |
| | 12.00.02 | PRGSQS | 106 |
| | 12.00.02 | VPHYNLNA | 107 |
| | 12.00.03 | LIQVVN | 108 |
| | 12.00.04 | AARSQ | 109 |
| | 12.00.05 | DNFEY | 110 |
| | 12.00.06 | SREQPQ | 111 |
| | 12.00.07 | CCFAFS | 112 |
| | 12.00.08 | PQQNEC | 113 |
| | 12.00.09 | GPQEIYIQQ | 114 |
| | 12.00.10 | RPSYTN | 115 |
| | 12.00.11 | CPSTFEE | 116 |
| | 12.00.12 | IYPGCPS | 117 |
| | 12.00.13 | PQQPQQR | 118 |
| | 12.00.14 | QRGQS | 119 |
| | 12.00.15 | QSSRPQ | 120 |
| | 12.00.16 | PQDRHQ | 121 |
| | 12.00.17 | QKIYNF | 122 |
| | 12.00.18 | REGDL | 123 |
| | 12.00.19 | VPTGVAW | 124 |
| | 12.00.20 | NQLDQ | 125 |
| | 12.00.21 | QEQEF | 126 |
| | 12.00.22 | KYQQEQ | 127 |
| | 12.00.23 | HQSQK | 128 |
| | 12.00.24 | KHQQEEEN | 129 |
| | 12.00.25 | KNLQGE | 130 |
| | 12.00.26 | TDEQQQ | 131 |
| | 12.00.27 | QQRPQE | 132 |
| | 12.00.28 | EKPQCK | 133 |
| | 12.00.29 | DKHCQR | 134 |
| | 12.00.30 | FVPHY | 135 |
| | 12.00.31 | KFLVPPQE | 136 |
| | 12.00.32 | PQESQK | 137 |
| Glycinin G2 (D00216.1) | 13.00.01 | CPSTYN | 138 |
| | 13.00.02 | QGGSQS | 139 |
| | 13.00.03 | SGAIV | 140 |
| | 13.00.03 | GGLRVT | 141 |
| | 13.00.04 | RLRQN | 142 |
| | 13.00.05 | LKLSAQ | 143 |
| | 13.00.06 | VAAKSQ | 144 |
| | 13.00.07 | PSIGNL | 145 |
| | 13.00.08 | FSFLVPP | 146 |
| | 13.00.09 | REQAQQN | 147 |
| | 13.00.10 | QKLNA | 148 |
| | 13.00.11 | RIESEG | 149 |
| | 13.00.12 | TYQEPQ | 150 |
| | 13.00.13 | PQESQ | 151 |
| | 13.00.14 | ESQQRG | 152 |
| | 13.00.15 | GRSQR | 153 |
| | 13.00.16 | QDRHQK | 154 |
| | 13.00.17 | VHRFRE | 155 |
| | 13.00.18 | EEENEG | 156 |
| | 13.00.19 | QIVRNL | 157 |
| | 13.00.20 | RKPQQE | 158 |
| | 13.00.21 | EEEQPQ | 159 |
| | 13.00.22 | DIYNPQ | 160 |
| | 13.00.23 | ERVFD | 161 |
| | 13.00.24 | ELQEG | 162 |
| Glycinin G3 (X15123.1) | 14.00.01 | FREQPQQN | 163 |
| | 14.00.02 | QPQKQQ | 164 |
| | 14.00.03 | RQIVRK | 165 |
| | 14.00.04 | RRQQAR | 166 |
| | 14.00.05 | ECQIQR | 167 |
| | 14.00.06 | IQQGSGI | 168 |
| | 14.00.07 | QQKGQS | 169 |
| | 14.00.08 | IYHFRE | 170 |
| | 14.00.09 | AVPTGF | 171 |
| | 14.00.10 | NSFQN | 172 |
| | 14.00.11 | PTEEQ | 173 |
| | 14.00.12 | QQQRPE | 174 |
| | 14.00.13 | PDCDEK | 175 |
| | 14.00.14 | DKHCQS | 176 |
| | 14.00.15 | CQSQSR | 177 |
| | 14.00.16 | QSQSRN | 178 |
| | 14.00.17 | HNIGQT | 179 |
| Glycinin G4 (X05652) | 15.03.2001 | QGKGA | 180 |
| | 15.03.2002 | VLSGFS | 181 |
| | 15.03.2003 | LNECQL | 182 |
| | 15.03.2004 | PDHRVE | 183 |
| | 15.03.2005 | RNGLHS | 184 |
| | 15.03.2006 | YSPYPR | 185 |
| | 15.03.2007 | VAIPGC | 186 |
| | 15.03.2008 | ETFEEP | 187 |
| | 15.03.2009 | QQLQDS | 188 |
| | 15.03.2010 | IRHFNE | 189 |
| | 15.03.2011 | DIEYPET | 190 |
| | 15.03.2012 | KQGQHQ | 191 |
| | 15.03.2013 | DIAEKL | 192 |
| | 15.03.2014 | KLESPD | 193 |
| | 15.03.2015 | SVISPK | 194 |
| | 15.03.2016 | QQDED | 195 |
| | 15.03.2017 | KREQD | 196 |
| | 15.03.2018 | HPPRRP | 197 |
| | 15.03.2019 | REQDED | 198 |
| | 15.03.2020 | GQDEDE | 199 |
| | 15.03.2021 | TQPRRP | 200 |
| | 15.03.2022 | PRQEEP | 201 |
| | 15.03.2023 | EEPRER | 202 |
| Glycinin G5 (M10962.1) | 16.02.2001 | NSQHPE | 203 |
| | 16.02.2002 | HPELQC | 204 |
| | 16.02.2003 | QCAGVT | 205 |
| | 16.02.2004 | TVSKR | 206 |
| | 16.02.2005 | KGAIGF | 207 |
| | 16.02.2006 | GSRSQ | 208 |
| | 16.02.2007 | VTVEG | 209 |
| | 16.02.2008 | RTPSYPP | 210 |
| | 16.02.2009 | VEENICT | 211 |
| | 16.02.2010 | PSRADF | 212 |
| | 16.02.2011 | NCQGN | 213 |
| | 16.02.2012 | RRGQLL | 214 |
| | 16.02.2013 | PAVAE | 215 |
| | 16.02.2014 | IPSEVL | 216 |
| | 16.02.2015 | NSYNL | 217 |
| | 16.02.2016 | CQLNN | 218 |
| | 16.02.2017 | RVESEG | 219 |
| | 16.02.2018 | YLPYPQ | 220 |
| | 16.02.2019 | SHLPSY | 221 |
| | 16.02.2020 | IGFAFP | 222 |
| | 16.02.2021 | FPGCPE | 223 |
| | 16.02.2022 | VIPLGV | 224 |
| | 16.02.2023 | DEPWA | 225 |
| | 16.02.2024 | RVFYL | 226 |
| | 16.02.2025 | GNPDIE | 227 |
| | 16.02.2026 | IEHPET | 228 |
| | 16.02.2027 | QGQHR | 229 |
| | 16.02.2028 | HRQQE | 230 |
| | 16.02.2029 | QQEEEG | 231 |
| | 16.02.1930 | DTAEK | 232 |
| | 16.02.1931 | EGGLS | 233 |
| | 16.02.1932 | KWQEQE | 234 |
| | 16.02.1933 | QEQEDE | 235 |
| | 16.02.1934 | DEEYGR | 236 |
| | 16.02.1935 | GKHEDD | 237 |
| | 16.02.1936 | EEEDQP | 238 |
| | 16.02.1937 | PRPDHP | 239 |
| | 16.02.1938 | HPPQRP | 240 |
| | 16.02.1939 | QRPSRP | 241 |
| | 16.02.1940 | PEQQEP | 242 |
| | 16.02.1941 | QQEPRG | 243 |
| | 16.02.1942 | LRRGQ | 244 |
| | 16.02.1943 | GNAVFD | 245 |
| | 16.02.1944 | THHNAV | 246 |
| | 16.02.1945 | SNSYNL | 247 |
| | 16.02.1946 | LGQSQV | 248 |
| | 16.02.1947 | PLVNP | 249 |
| | 16.02.1948 | QGNSG | 250 |
| Hydrophobic Seed Protein (A29385) | 18.00.01 | LGGSL | 251 |
| | 18.00.02 | DDCCAL | 252 |
| | 18.00.03 | NSCGRS | 253 |
| Kunitz trypsin inhibitor (S45092.1) | 19.13.01 | GNERC | 254 |
| | 19.13.02 | RAAPTG | 255 |
| | 19.13.03 | LCVGIP | 256 |
| | 19.13.04 | TEWSV | 257 |
| p34 thiol protease (J05560.1) | 23.01.2001 | VITQVK | 258 |
| | 23.01.2002 | KYQGGC | 259 |
| Profilin (AJ223981.1) | 26.00.01 | KPEEI | 260 |
| | 26.00.02 | FPQFKP | 261 |
| | 26.00.03 | EPGSL | 262 |
| | 26.00.04 | QGEPGAV | 263 |
| | 26.00.05 | ERLGDY | 264 |
| | 26.00.06 | AYVDDH | 265 |
| | 26.00.07 | NHLTHAAI | 266 |
| | 26.00.08 | IIGQDG | 267 |
| | 26.00.09 | GSVWLQ | 268 |
| | 26.00.10 | TPGQC | 269 |
| | 26.00.11 | IYDEP | 270 |
| Profilin (AJ223982.1) | 26.01.2001 | CDIEGN | 271 |
| | 26.01.2002 | GSVWAQ | 272 |
| | 26.01.2003 | VVERP | 273 |
| | 26.01.2004 | IDQGY | 274 |
| PR10 (X60043.1) | 25.00.01 | SIDEA | 275 |
| | 25.00.02 | NLGYSY | 276 |
| | 25.00.03 | VENVEGN | 277 |
| | 25.00.04 | EDEINS | 278 |
| | 25.00.05 | NSPVAP | 279 |
| | 25.00.06 | PDTAEK | 280 |
| | 25.00.07 | EKITF | 281 |
| | 25.00.08 | GSAGKLT | 282 |
| | 25.00.09 | ETKGD | 283 |
| 18 kD Seed Maturation Protein (AJ574791.1) | 2.00.001 | IGASA | 284 |
| | 2.00.002 | ATVQEKAER | 285 |
| | 2.00.003 | ARDPVQ | 286 |
| | 2.00.004 | ELATQK | 287 |
| | 2.00.005 | QHNTA | 288 |
| | 2.00.006 | GHGHHT | 289 |
| | 2.00.007 | GEYGQP | 290 |
| | 2.00.008 | HQTSA | 291 |
| | 2.00.009 | HGTGQPTGH | 292 |
| | 2.00.010 | HVTEGV | 293 |
| | 2.00.011 | VGSHPIG | 294 |
| 2s Albumin (AAB71140.1) | 1.00.001 | LFCIAHTCS | 295 |
| | 1.00.002 | SASKWQH | 296 |
| | 1.00.004 | QQDSCR | 297 |
| | 1.00.005 | SCRKQL | 298 |
| | 1.00.006 | KQLQGVN | 299 |
| | 1.00.007 | NLTPCEK | 300 |
| | 1.00.009 | QGRGD | 301 |
| | 1.00.011 | EDEEEEG | 302 |
| | 1.00.012 | QKCCT | 303 |
| | 1.00.013 | TEMSEL | 304 |
| | 1.00.015 | CKALQK | 305 |
| | 1.00.016 | NQSEELEEK | 306 |
| | 1.00.017 | MCRFGP | 307 |
| | 1.00.018 | IQCDLS | 308 |
| 2s Albumin (BT091363.1) | 1.01.002 | CASKWQQH | 309 |
| | 1.01.003 | QHQQES | 310 |
| | 1.01.005 | ESCREQL | 311 |
| | 1.01.007 | NPCEHI | 312 |
| | 1.01.008 | EKIQAG | 313 |
| | 1.01.009 | DGSDEDH | 314 |
| | 1.01.010 | EGKEE | 315 |
| | 1.01.011 | KEEEEE | 316 |
| | 1.01.013 | SEMSE | 317 |
| | 1.01.014 | SPICQCK | 318 |
| | 1.01.016 | NQSEQLEGKE | 319 |
| | 1.01.017 | AIRCRL | 320 |
| | 1.01.018 | IGCDL | 321 |
| Albumin 1 (AJ574791.1) | 3.00.001 | NGACSPFE | 322 |
| | 3.00.002 | PPCRSRD | 323 |
| | 3.00.003 | RDCRC | 324 |
| | 3.00.004 | RCVPIGL | 325 |
| | 3.00.005 | AGFCIH | 326 |
| | 3.00.006 | GLSSVA | 327 |
| | 3.00.008 | HPNLCQSD | 328 |
| | 3.00.009 | QSDDEC | 329 |
| | 3.00.010 | GNFCARYP | 330 |
| Albumin 1 (AJ223037.1) | 3.01.002 | PPCRSS | 331 |
| | 3.01.007 | VDEHP | 332 |
| | 3.01.011 | SDSEA | 333 |
| Albumin 1 (AJ223037.1) | 3.03.001 | EGTSSAKLT | 334 |
| | 3.03.002 | RCVPIA | 335 |
| Lectin (K00821.1) | 21.05.2001 | LAPIDT | 336 |
| | 21.05.2002 | TKPQT | 337 |
| | 21.05.2003 | KPQTHA | 338 |
| | 21.05.2004 | DPPNPHI | 339 |
| | 21.05.2005 | DTFRN | 340 |
| | 21.05.2006 | NLPHAS | 341 |
| Seed biotinylated protein (U59626.1) | 27.01.2001 | EIHVEK | 342 |
| | 27.01.2002 | KHRVPK | 343 |
| | 27.01.2003 | DHAGKA | 344 |
| | 27.01.2004 | KESQRE | 345 |
| | 27.01.2005 | ANWG | 346 |
| | 27.01.2006 | VAEKGR | 347 |
| | 27.01.2007 | GRETE | 348 |
| | 27.01.2008 | AHVVEG | 349 |
| | 27.01.2009 | EYTAK | 350 |
| | 27.01.2010 | EAQRELE | 351 |
| | 27.01.2011 | QPQEAE | 352 |
| | 27.01.2012 | GESEG | 353 |
| | 27.01.2013 | RAKHEEG | 354 |

| | | | |
|---|---|---|---|
| "X" indicating any amino acid | | | |

The epitopes identified by the inventors provide the most comprehensive data on potentially allergenic proteins and peptides derived from soybean that are available so far. These data, i.e. the identified epitopes, provide the essential basis for assessing both, the immune status of an individual to soybean protein and the allergenic potential of food and other products suspected of containing soybean protein. The compilation of the invention provides a collection of peptides of different amino acid sequences with each peptide comprising a short element of about three to six amino acids (sequence element), which corresponds to one of the identified epitopes (table 1). Thus, each peptide presents a potential epitope for a soybean-allergen antibody (IgE). The term "corresponding" refers to the exact reproduction of the epitope sequence as well as variants thereof differing from the identified epitope in one or two amino acids. The variation may be due to one or two missing amino acids and/or to the exchange of one or two amino acids in comparison to the identified epitope. It is well established in the art that the secondary structure and the physical-chemical properties of a peptide (e.g. its antibody binding properties due to size, charge and/or polarity) may be maintained despite of minor changes to the amino acid sequence. In particular, single amino acids with side chains of little polarity or other reactivity, may be omitted or replaced by other amino acids of similar reactivity (e.g. -lysine - arginine or leucine - isoleucine). The possibility of minor amino acid changes within the amino acid sequence corresponding to the epitope was also confirmed by the data obtained from the phage display and the peptide arrays. The antibodies contained in the patients' sera recognized several variants of individual epitopes, e.g. overlapping epitopes such as EDEEQQ (SEQ ID NO.: 32), EEQQRES (SEQ ID NO.: 33), RESEES (SEQ ID NO.: 34), (see table 1, and SEQ ID NO.: 385) of beta conglycinin alpha chain.

Accordingly, comprehensive analysis of interactions of antibody sera derived from various patients allowed to determine variations within the epitopes sequence. Table 2 depicts the epitopes within the endogenous soybean protein sequence (epitopes underlined) and indicates the variability of the epitope (minimum amino acids / all amino acids of the epitope).

**Table 2: Epitopes within the natural soybean protein**

| Soybean protein (Genebank ID) | Epitope with adjacent amino acids according to protein sequence | SEQ ID NO. | variation |
|---|---|---|---|
| Defensin (Z13956.1) | VFASQVVVQTEGRVC | 355 | 4/6 |
| | VFASQVVVQTEGRVC | 356 | 4/6 |
| | VFASQVVVQTEGRVC | 357 | 4/6 |
| | QVVVQTEGRVCESQS | 358 | 4/7 |
| | QTEGRVCESQSHGFH | 359 | 4/5 |
| | RVCESQSHGFHGLCNR | 360 | 4/7 |
| | SQSHGFHGLCNRDHN | 361 | 4/9 |
| | GFHGLCNRDHNCALVC | 362 | 4/9 |
| | LCNRDHNCALVCRNE | 363 | 4/6 |
| | RCKRSRRCFCTRICG | 364 | 4/6 |
| beta conglycinin alpha chain (X17698.1) | PKHNKCLQSCNSERD | 365 | 4/7 |
| | PKHNKCLQSCNSERD | 366 | 4/5 |
| | SCNSERDSYRNQACH | 367 | 4/6 |
| | SCNSERDSYRNQACHA | 368 | 4/7 |
| | ERDSYRNQACHARCN | 369 | 4/6 |
| | LKVEKEECEEGEIPR | 370 | 4/7 |
| | KEECEEGEIPRPRPR | 371 | 4/6 |
| | KEECEEGEIPRPRPR | 372 | 4/6 |
| | IPRPRPRPQHPEREPQ | 373 | 4/9 |
| | RPRPQHPEREPQQPGE | 374 | 4/7 |
| | QHPEREPQQPGEKEE | 375 | 4/6 |
| | QHPEREPQQPGEKEE | 376 | 4/7 |
| | QPGEKEEDEDEQPRP | 377 | 4/9 |
| | EDEQPRPIPFPRPQP | 378 | 4/6 |
| | PRPIPFPRPQPRQEEE | 379 | 4/6 |
| | PQPRQEEEHEQREEQ | 380 | 5/8 |
| | WPRKEEKRGEKGSEE | 381 | 5/7 |
| | RPPHQKEERNEEEDE | 382 | (4/7 |
| | QKEERNEEEDEDEEQQ | 383 | 4/6 |
| | RNEEEDEDEEQQRES | 384 | 4/7 |
| | EDEDEEQQRESEESE | 385 | 4/7 |
| | KFEEINKVLFSREEG | 386 | 5/8 |
| | INKVLFSREEGQQQG | 387 | 4/7 |
| | SEDKPFNLRSRDPIY | 388 | 4/7 |
| | LSIVDMNEGALLLPHF | 389 | 4/5 |
| | ELVGLKEQQQEQQQE | 390 | 4/7 |
| | ELVGLKEQQQEQQQE | 391 | 4/7 |
| | LKEQQQEQQQEEQPLE | 392 | 4/6 |
| | LLKNQRESYFVDAQP | 393 | 5/8 |
| | LLKNQRESYFVDAQP | 394 | 4/6 |
| | PFLFGSNRFETLFKN | 395 | 4/5 |
| | RFETLFKNQYGRIRVL | 396 | 4/6 |
| | YRILEFNSKPNTLLLP | 397 | 4/6 |
| | TTYYVVNPDNNENLRL | 398 | 4/6 |
| | LRLITLAIPVNKPGR | 399 | 4/5 |
| | IPVNKPGRFESFFLSS | 400 | 4/6 |
| | SRNILEASYDTKFEE | 401 | 4/5 |
| | LFSREEGQQQGEQRLQE | 402 | 4/7 |
| | GEQRLQESVIVEISK | 403 | 4/5 |
| | LSKRAKSSSRKTISSE | 404 | 4/6 |
| | FFEITPEKNPQLRDLD | 405 | 4/6 |
| | SEQDIFVIPAGYPVVV | 406 | 4/6 |
| | QVQELAFPGSAQAVE | 407 | 4/6 |
| beta conglycinin alpha' chain (M13759.1) | RCNLLKVEEEEECEE | 408 | 4/6 |
| | QIPRPRPQHPERERQ | 409 | 4/6 |
| | PRPQHPERERQQHGEK | 410 | 4/6 |
| | ERQQHGEKEEDEGEQP | 411 | 4/7 |
| | HGEKEEDEGEQPRPF | 412 | 4/8 |
| | GEQPRPFPFPRPRQP | 413 | 4/7 |
| | RPFPFPRPRQPHQEE | 414 | 4/6 |
| | QEEEHEQKEEHEWHR | 415 | 4/6 |
| | QEEEHEQKEEHEWHR | 416 | 4/5 |
| | GGKGSEEEQDEREHP | 417 | 5/9 |
| | HKQEKHQGKESEEEEE | 418 | 4/6 |
| | HKQEKHQGKESEEEEE | 419 | 4/5 |
| | HQGKESEEEEEDQDE | 420 | 4/7 |
| | ESEEEEEDQDEDEEQ | 421 | 5/9 |
| | KESQESEGSESQREP | 422 | 4/9 |
| | KESQESEGSESQREP | 423 | 4/6 |
| | NKRSQQLQNLRDYRI | 424 | 4/8 |
| | KPNTLLLPHHADADY | 425 | 4/6 |
| | FEEINKVLFGREEGQ | 426 | 4/6 |
| | AIVVLVINEGEANIE | 427 | 4/6 |
| | NIELVGIKEQQQRQQ | 428 | 4/6 |
| | QDIFVIPAGYPVMVN | 429 | 4/7 |
| | ESYFVDAQPQQKEEG | 430 | 4/6 |
| | ESYFVDAQPQQKEEG | 431 | 4/6 |
| | ESYFVDAQPQQKEEG | 432 | 4/5 |
| | PLLLLGVVFLASVSV | 433 | 4/5 |
| | HFNSKRFQTLFKNQY | 434 | 4/5 |
| | ITQRNPQLRDLDVFL | 435 | 4/5 |
| | HPRPHQPHQKEEEKH | 436 | 4/5 |
| | LVNNDDRDSYNLQSG | 437 | 4/5 |
| | QSGDALRVPAGTTFY | 438 | 4/6 |
| beta conglycinin beta chain (S44893) | QRFNKRSPQLENLRD | 439 | 4/6 |
| | RDSYNLHPGDAQRIP | 440 | 4/5 |
| | RDSYNLHPGDAQRIP | 441 | 4/6 |
| | SHNILETSFHSEFEE | 442 | 4/8 |
| | SHNILETSFHSEFEE | 443 | 4/6 |
| | LETSFHSEFEEINRV | 444 | 4/6 |
| | NRVLFGEEEEQRQQE | 445 | 4/5 |
| | NRVLFGEEEEQRQQE | 446 | 4/6 |
| | FGEEEEQROQEGVIV | 447 | 4/6 |
| | AIVILVINEGDANIE | 448 | 4/5 |
| | LLGTVFLASVCVSLK | 449 | 4/6 |
| | FLLFVLSGRAILTLVN | 450 | 4/5 |
| | VNPHDHQNLKIIKLA | 451 | 4/6 |
| | AIPVNKPGRYDDFFLS | 452 | 4/6 |
| | EGVIVELSKEQIRQLS | 453 | 4/6 |
| | GALLLPHFNSKAIVIL | 454 | 4/6 |
| | QKQKQEEEPLEVQRYR | 455 | 4/6 |
| | NVVRQIERQVQELAFP | 456 | 4/5 |
| Glycinin G1 (AB113349.1) | FSGCCFAFSSREQPQ | 457 | 4/6 |
| | FSGCCFAFSSREQPQ | 458 | 4/5 |
| | CFAFSSREQPQQNEC | 459 | 4/9 |
| | RPSYTNGPQEIYIQQ | 460 | 4/6 |
| | RPSYTNGPQEIYIQQ | 461 | 4/7 |
| | GMIYPGCPSTFEEPQ | 462 | 4/7 |
| | GMIYPGCPSTFEEPQ | 463 | 4/8 |
| | STFEEPQQPQQRGQS | 464 | 4/5 |
| | STFEEPQQPQQRGQS | 465 | 4/6 |
| | EPQQPQQRGQSSRPQ | 466 | 4/6 |
| | PQQRGQSSRPQDRHQ | 467 | 4/6 |
| | RPQDRHQKIYNFREG | 468 | 4/5 |
| | REGDLIAVPTGVAWW | 469 | 4/5 |
| | REGDLIAVPTGVAWW | 470 | 4/5 |
| | NSLENQLDQMPRRFY | 471 | 4/5 |
| | AGNQEQEFLKYQQEQ | 472 | 4/6 |
| | AGNQEQEFLKYQQEQ | 473 | 4/5 |
| | GHQSQKGKHQQEEEN | 474 | 4/8 |
| | GHQSQKGKHQQEEEN | 475 | 4/6 |
| | KQIAKNLQGENEGED | 476 | 4/6 |
| | GLSVIKPPTDEQQQR | 477 | 4/6 |
| | IKPPTDEQQQRPQEE | 478 | 4/6 |
| | DEKPQCKGKDKHCQR | 479 | 4/6 |
| | DEKPQCKGKDKHCQR | 480 | 4/5 |
| | FGSLRKNAMFVPHYN | 481 | 4/8 |
| | NNPFKFLVPPQESQK | 482 | 4/6 |
| | NNPFKFLVPPQESQK | 483 | 4/6 |
| | NNKPFQCAGVALSRCT | 484 | 4/5 |
| | KHCQRPRGSQSKSRRN | 485 | 4/8 |
| | KNAMFVPHYNLNANSIIY | 486 | 4/6 |
| | LNGRALIQVVNCNGER | 487 | 4/5 |
| | QNFVVAARSQSDNFE | 488 | 4/5 |
| | ARSQSDNFEYVSFKT | 489 | 4/7 |
| Glycinin G2 (D00216.1) | FALREQAQQNECQIQ | 490 | 4/5 |
| | QIQKLNALKPDNRIE | 491 | 4/6 |
| | LNALKPDNRIESEGG | 492 | 4/6 |
| | FPGCPSTYQEPQESQ | 493 | 4/5 |
| | FPGCPSTYQEPQESQ | 494 | 4/6 |
| | ESQQRGRSQRPQDRH | 495 | 4/5 |
| | ESQQRGRSQRPQDRH | 496 | 4/6 |
| | QRPQDRHQKVHRFRE | 497 | 4/6 |
| | QRPQDRHQKVHRFRE | 498 | 4/6 |
| | QSQKGKQQEEENEGS | 499 | 4/6 |
| | AFGVNMQIVRNLQGE | 500 | 4/6 |
| | VTAPAMRKPQQEEDD | 501 | 4/6 |
| | PQQEEDDDDEEEQPQ | 502 | 4/6 |
| | QNIGQNSSPDIYNPQ | 503 | 4/5 |
| | NCNGERVFDGELQEG | 504 | 4/5 |
| | NCNGERVFDGELQEG | 505 | 4/6 |
| | MIFPGCPSTYQEPQES | 506 | 4/5 |
| | YQQQQQGGSQSQKGKQ | 507 | 4/5 |
| | NEEEDSGAIVTVKGG | 508 | 4/5 |
| | IVTVKGGLRVTAPAMR | 509 | 4/5 |
| | TICTMRLRQNIGQNS | 510 | 4/6 |
| | PALWLLKLSAQYGSLR | 511 | 4/6 |
| | PQNFAVAAKSQSDNFE | 512 | 4/6 |
| | KTNDRPSIGNLAGANS | 513 | 4/7 |
| | KNNNPFSFLVPPQESQR | 514 | 4/6 |
| Glycinin G3 (X15123.1) | SFREQPQQNECQIQR | 515 | 4/6 |
| | IQQGSGIFGMIFPGC | 516 | 4/6 |
| | STFEEPQQKGQSSRP | 517 | 4/6 |
| | SRPQDRHQKIYHFRE | 518 | 4/6 |
| | FREGDLIAVPTGFAY | 519 | 4/5 |
| | TNSFQNQLDQMPRRF | 520 | 4/5 |
| | LSVISPPTEEQQQRP | 521 | 4/6 |
| | EEQQQRPEEEEKPDC | 522 | 4/6 |
| | PDCDEKDKHCQSQSR | 523 | 4/6 |
| | PDCDEKDKHCQSQSR | 524 | 4/6 |
| | PDCDEKDKHCQSQSR | 525 | 4/6 |
| | HCQSQSRNGIDETIC | 526 | 4/6 |
| | HNIGQTSSPDIFNPQ | 527 | 4/8 |
| | CFAFSFREQPQQNECQIQ | 528 | 4/6 |
| | EFLQYQPQKQQGGTQS | 529 | 4/6 |
| | AFVVDRQIVRKLQGENE | 530 | 4/5 |
| | QTFNLRRQQARQVKNN | 531 | 4/6 |
| Glycinin G4 (X05652) | QLNNLNALEPDHRVE | 532 | 4/6 |
| | RNGLHSPSYSPYPRM | 533 | 4/6 |
| | RNGLHSPSYSPYPRM | 534 | 4/6 |
| | LGVAIPGCPETFEEP | 535 | 4/6 |
| | LGVAIPGCPETFEEP | 536 | 4/5 |
| | QQLQDSHQKIRHFNE | 537 | 4/6 |
| | QQLQDSHQKIRHFNE | 538 | 4/7 |
| | PDIEYPETMQQQQQQ | 539 | 4/6 |
| | QQQKSHGGRKQGQHQ | 540 | 4/6 |
| | NEDIAEKLESPDDER | 541 | 4/6 |
| | NEDIAEKLESPDDER | 542 | 4/6 |
| | LSVISPKWQEQQDED | 543 | 4/5 |
| | LSVISPKWQEQQDED | 544 | 4/5 |
| | SHPPRRPSHGKREQD | 545 | 4/6 |
| | SHPPRRPSHGKREQD | 546 | 4/6 |
| | RRPSHGKREQDEDED | 547 | 4/6 |
| | GKRNKTGQDEDEDED | 548 | 4/6 |
| | WRSKKTQPRRPRQEE | 549 | 4/6 |
| | KTQPRRPRQEEPRER | 550 | 4/6 |
| | KTQPRRPRQEEPRER | 551 | 4/6 |
| | HNAVTSYLKDVFRAI | 552 | 4/5 |
| | MIIIAQGKGALGVAI | 553 | 4/6 |
| | EEGGSVLSGFSKHFLA | 554 | 4/6 |
| | SSSKLNECQLNNLNA | 555 | 4/5 |
| Glycinin G5 (M10962.1) | CQLNNLNALEPDHRV | 556 | 4/6 |
| | NLNALEPDHRVESEG | 557 | 4/5 |
| | NRNGSHLPSYLPYPQ | 558 | 4/8 |
| | NRNGSHLPSYLPYPQ | 559 | 4/6 |
| | AIGFAFPGCPETFEK | 560 | 4/6 |
| | AIGFAFPGCPETFEK | 561 | 4/6 |
| | FNEGDVLVIPLGVPY | 562 | 4/6 |
| | TYNTGDEPVVAISPL | 563 | 4/5 |
| | RVFYLAGNPDIEHPE | 564 | 4/6 |
| | RVFYLAGNPDIEHPE | 565 | 4/6 |
| | RVFYLAGNPDIEHPET | 566 | 4/5 |
| | QQQKSHGGRKQGQHR | 567 | 4/5 |
| | SHGGRKQGQHRQQEE | 568 | 4/6 |
| | RKQGQHRQQEEEGGS | 569 | 4/5 |
| | EDTAEKLRSPDDERK | 570 | 4/5 |
| | EGGLSVISPKWQEQE | 571 | 4/6 |
| | EGGLSVISPKWQEQE | 572 | 4/6 |
| | SVISPKWQEQEDEDE | 573 | 4/6 |
| | EQEDEDEDEDEEYGR | 574 | 4/6 |
| | SHGKHEDDEDEDEEE | 575 | 4/6 |
| | HEDDEDEDEEEDQPR | 576 | 4/6 |
| | EDEDEEEDQPRPDHP | 577 | 4/6 |
| | EEEDQPRPDHPPQRP | 578 | 4/6 |
| | QPRPDHPPQRPSRPE | 579 | 4/6 |
| | DHPPQRPSRPEQQEP | 580 | 4/6 |
| | RPEQQEPRGRGCQTR | 581 | 4/5 |
| | CQGNAVFDGELRRGQ | 582 | 4/6 |
| | CQGNAVFDGELRRGQ | 583 | 4/6 |
| | THHNAVSSYIKDVFR | 584 | 4/6 |
| | VLSNSYNLGQSQVRQ | 585 | 4/6 |
| | VLSNSYNLGQSQVRQ | 586 | 4/5 |
| | KYQGNSGPLVNPGSG | 587 | 4/5 |
| | KYQGNSGPLVNPGSG | 588 | 4/6 |
| | LIETWNSQHPELQCAG | 589 | 4/6 |
| | TWNSQHPELQCAGVTV | 590 | 4/6 |
| | QHPELQCAGVTVSKRT | 591 | 4/5 |
| | QCAGVTVSKRTLNRN | 592 | 4/6 |
| | IVVQGKGAIGFAFPGC | 593 | 4/5 |
| | QSSRRGSRSQQQLQD | 594 | 4/5 |
| | ERKQIVTVEGGLSVI | 595 | 4/7 |
| | DEEYGRTPSYPPRRPSH | 596 | 4/7 |
| | QTRNGVEENICTMKLHE | 597 | 4/6 |
| | ENIARPSRADFYNPKA | 598 | 4/5 |
| | RVRVVNCQGNAVFDG | 599 | 4/6 |
| | FDGELRRGQLLVVPQN | 600 | 4/5 |
| | VVPQNPAVAEQGGEQ | 601 | 4/6 |
| | DVFRVIPSEVLSNSYN | 602 | 4/5 |
| | SEVLSNSYNLGQSQV | 603 | 4/6 |
| Profilin1 (AJ223981.1) | AYVDDHLLCGIEGNH | 604 | 4/8 |
| | GNHLTHAAIIGQDGS | 605 | 4/6 |
| | GNHLTHAAIIGQDGS | 606 | 4/6 |
| | THAAIIGQDGSVWLQ | 607 | 4/5 |
| | LIIGIYDEPMTPGQC | 608 | 4/5 |
| | DFPQFKPEEITAIMN | 609 | 4/6 |
| | LQSTDFPQFKPEEITA | 610 | 4/5 |
| | MNDFNEPGSLAPTGL | 611 | 4/7 |
| | KYMVIQGEPGAVIRGKK | 612 | 4/6 |
| | CNMVVERLGDYLIDQG | 613 | 4/5 |
| Profilin2 (AJ223982.) | MVVERPGDYLIDQGY | 614 | 4/5 |
| | MVVERPGDYLIDQGY | 615 | 4/6 |
| | DDHLLCDIEGNHLTHA | 616 | 4/6 |
| | IIGQDGSVWAQSTDFP | 617 | 4/5 |
| PR10 (X60043.1) | HKIESIDEANLGYSY | 618 | 4/6 |
| | HKIESIDEANLGYSY | 619 | 4/7 |
| | VENVEGNGGPGTIKK | 620 | 4/6 |
| | GVFTFEDEINSPVAPA | 621 | 4/6 |
| | FEDEINSPVAPATLYK | 622 | 4/6 |
| | GGAALPDTAEKITFDS | 623 | 4/5 |
| | LPDTAEKITFDSKLV | 624 | 4/7 |
| | AGPNGGSAGKLTVKYET | 625 | 4/5 |
| | LTVKYETKGDAEPNQ | 626 | 4/9 |
| 2s Albumin (AAB71140.1) | LLISLLFCIAHTCSASKWQ | 627 | 4/7 |
| | IAHTCSASKWQHQQDSC | 628 | 4/6 |
| | SKWQHQQDSCRKQLQG | 629 | 4/6 |
| | QHQQDSCRKQLQGVNL | 630 | 4/7 |
| | QDSCRKQLQGVNLTPCE | 631 | 4/7 |
| | QLQGVNLTPCEKHIMEK | 632 | 4/5 |
| | IMEKIQGRGDDDDDD | 633 | 4/7 |
| | NEGKDEDEEEEGHMQKC | 634 | 4/5 |
| | EEGHMQKCCTEMSEL | 635 | 4/6 |
| | MQKCCTEMSELRSPKC | 636 | 4/6 |
| | SPKCQCKALQKIMENQ | 637 | 4/9 |
| | QKIMENQSEELEEKQKKK | 638 | 4/6 |
| | INLATMCRFGPMIQCDL | 639 | 4/6 |
| | RFGPMIQCDLSSDD | 640 | 4/8 |
| 2s Albumin (BT091363.1) | IAHTCCASKWQQHQQES | 641 | 4/6 |
| | CCASKWQQHQQESCREQ | 642 | 4/7 |
| | QQHQQESCREQLKGINL | 643 | 4/6 |
| | KGINLNPCEHIMEKIQ | 644 | 4/6 |
| | CEHIMEKIQAGRRGED | 645 | 4/7 |
| | GRRGEDGSDEDHILIRT | 646 | 4/5 |
| | YIRKKEGKEEEEEGH | 647 | 4/6 |
| | RKKEGKEEEEEGHMQK | 648 | 4/5 |
| | MQKCCSEMSELKSPI | 649 | 4/7 |
| | MSELKSPICQCKALQKI | 650 | 4/10 |
| | QKIMDNQSEQLEGKEKKQ | 651 | 4/6 |
| | ELMNLAIRCRLGPMIG | 652 | 4/5 |
| | RLGPMIGCDLSSDD | 653 | 4/5 |
| 18 kD Seed Maturation Protein (AJ574791.1) | ETATNIGASAKAGME | 654 | 4/10 |
| | MEKTKATVQEKAERMTAR | 655 | 4/6 |
| | AERMTARDPVQKELAT | 656 | 4/6 |
| | DPVQKELATQKKEAKM | 657 | 4/5 |
| | KQAARQHNTAAKQSA | 658 | 4/6 |
| | TAGHMGHGHHTTGTGT | 659 | 4/6 |
| | TYSTTGEYGQPMGAHQ | 660 | 4/5 |
| | QPMGAHQTSAMPGHG | 661 | 4/9 |
| | SAMPGHGTGQPTGHVTE | 662 | 4/6 |
| | GQPTGHVTEGVVGSHP | 663 | 4/7 |
| | VTEGVVGSHPIGTNRGP | 664 | 4/8 |
| Albumin 1_(AJ574791.1) | EAADCNGACSPFEMPPCR | 665 | 4/7 |
| | SPFEMPPCRSRDCRCVP | 666 | 4/5 |
| | PPCRSRDCRCVPIGL | 667 | 4/7 |
| | RSRDCRCVPIGLVAGFC | 668 | 4/6 |
| | PIGLVAGFCIHPTGLS | 669 | 4/6 |
| | CIHPTGLSSVAKMIDE | 670 | 4/8 |
| | KMIDEHPNLCQSDDECMK | 671 | 4/6 |
| | HPNLCQSDDECMKKGS | 672 | 4/8 |
| | MKKGSGNFCARYPNNYID | 673 | 4/6 |
| Albumin 1 (AJ223037.1) | SPFEVPPCRSSDCRCV | 674 | 4/5 |
| | SVAKMVDEHPNLCQS | 675 | 4/5 |
| | GWCFDSDSEALKGFL | 676 | 4/9 |
| Albumin 1 (AJ223037.1_G3FGW7) | MQEGTSSAKLTTHLNK | 677 | 4/6 |
| | RSSDCRCVPIALFVGF | 678 | 4/6 |
| Seed biotinylated protein (U59626.1) | EIHVEKHRVPKMATH | 679 | 4/6 |
| | EIHVEKHRVPKMATH | 680 | 4/6 |
| | KDHAGKAMGDIGGRG | 681 | 4/6 |
| | HAAANVVGNKESQRE | 682 | 4/5 |
| | HAAANVVGNKESQRE | 683 | 4/6 |
| | ESGGQVVAEKGRETE | 684 | 4/5 |
| | ESGGQVVAEKGRETE | 685 | 4/6 |
| | AAAHVVEGAAGYAGH | 686 | 4/5 |
| | EYTAKKKEEAQRELE | 687 | 4/7 |
| | EYTAKKKEEAQRELE | 688 | 4/6 |
| | QPQEAEERPSEGIGE | 689 | 4/5 |
| | NTMGGESEGGGGKEE | 690 | |
| | VLETRVTGRAKHEEG | 691 | 4/6 |
| Lectin (K00821.1) | GLAFFLAPIDTKPQT | 692 | 4/5 |
| | GLAFFLAPIDTKPQT | 693 | 4/6 |
| | FLAPIDTKPQTHAGY | 694 | 4/7 |
| | FDTFRNSWDPPNPHI | 695 | 4/5 |
| | FDTFRNSWDPPNPHI | 696 | 4/6 |
| | ASNLPHASSNIDPLD | 697 | 4/6 |
| Gly m BD 28K (AB046874.2) | GGYVPCRQEEDEELHHKC | 698 | 4/6 |
| p34 thiol protease (J05560.1) | GVITQVKYQGGCGRG | 699 | 4/5 |
| Kunitz trypsin inhibitor (S45092.1) | AFGGIRAAPTGNERC | 700 | 4/6 |
| | AFGGIRAAPTGNERC | 701 | 4/6 |
| | | | |
| | AVIMLCVGIPTEWSV | 702 | 4/5 |
| | AVIMLCVGIPTEWSV | 703 | 4/6 |
| Hydrophobic Seed Protein (A29385) | ILGGSLGTVDDCCAL | 704 | 4/5 |
| | ILGGSLGTVDDCCAL | 705 | 4/6 |

The compilation of the invention is for example suitable for determining the immune status of a patient to soybean proteins by analysing the patient's immunological reaction to individual epitopes. As each peptide of the compilation presents a potentially allergenic soybean epitope, interaction of the patient's antibodies (IgE) with the peptides is representative for the patient's immune status to soybean allergens.

Additionally, a peptide comprising at least one sequence element corresponding to a soybean epitope is suitable for identifying or generating molecules specifically binding to the respective epitope and peptides or proteins comprising the same. The resulting molecules, e.g. antibodies, provide a significant advantage for detecting soybean proteins. Common anti-soybean antibodies or sera are raised against whole soybean proteins or even proteomes and therefore inevitably include antibodies directed against non-allergen proteins/parts of proteins. In contrast, utilizing peptides with sequence elements corresponding to specific allergenic soybean epitopes allows for the generation of antibodies distinctly directed against allergenic soybean proteins. Moreover, these antibodies specifically bind to the allergenic part (i.e epitope) of a soybean protein. Finally, such antibodies, or other binding molecules generated by using the peptides of the invention, allow the detection of allergenic remnants of soybean proteins that could be missed using antibodies binding to non-allergenic parts of a protein.

The term "compilation" refers to a collection of at least five different peptides, which includes that each peptide may be present in several or even hundreds of identical copies. Each peptide comprises at least one sequence element corresponding to an epitope, but may also comprise more, e.g. two or three sequence elements corresponding to identical and/or different epitopes. For example, the compilation may comprise at least five different peptides, wherein each peptide comprises the same sequence element (thus representing the same epitope) but different additional amino acids. In this case, the same sequence element is presented in various different molecular environments e.g. secondary structures. Likewise, each peptide may comprise multiple sequence elements corresponding to different epitopes, such that various epitopes/soybean proteins are represented in the compilation. Therefore, it is to be understood, that the compilation may comprise both, peptides comprising sequence elements corresponding to different epitopes as well as peptides comprising identical sequence elements, thus all corresponding to the same epitope, but comprising different additional amino acids.

The compilation may be provided by immobilizing the peptides on a carrier, e.g. a chip, slide, well plate or on beads. Likewise, the compilation may be provided as a multitude of compositions, each composition containing identical copies of a peptide or a mixture of different peptides. Alternatively, the compilation may be provided as a single composition containing different peptides comprising sequence elements corresponding to the same or different epitopes.

In a preferred embodiment, the compilation comprises at least 10, preferably at least 20, more preferred at least 50 different peptides. Depending on the application, the advantageous number of different peptides may vary. For example, when testing the binding specificity of an antibody to a distinct epitope, a compilation comprising five different peptides, each comprising the same sequence element corresponding to the epitope but different additional amino acids, may be used. In a simple diagnostic application, a few peptides, e.g. five to ten peptides, may be sufficient to investigate sera for the general presence of antibodies against soybean proteins. In such cases, the sequence elements are preferably selected to correspond to epitopes of the most allergenic proteins such as Gly m Bd 28K, glycinin, hydrophobic seed protein (Gly m 1 a), kunitz trypsin inhibitor, profilin (Gly m3), β-conglycinin (Gly m Bd 60K) and defensin (Gly 2m). For more detailed analyses, the compilation may comprise about 20 to 500 different peptides, preferably representing at least one epitope of each identified allergenic soybean protein. For example, such analysis could be used for providing a more personalized analysis of a patient's health condition. For such comprehensive analysis, a peptide array or a bead-based multiplex assay may be provided covering sequence elements corresponding to each of the identified epitopes.

In a preferred embodiment, the peptide consists of about 5 to 30 amino acids, preferably 8 to 20 amino acids, more preferred 8 to 15 amino acids. Besides the sequence element corresponding to the epitope, usually four, five or six amino acids, each peptide may comprise further amino acids on one or both sides of the sequence element. Such additional amino acids may be added for example for immobilizing the peptide on a substrate or for embedding the sequence element corresponding to the epitope into a specific molecular environment (secondary structure). The additional amino acids may be chosen depending on the use of the compilation. For example, to present the epitope in its natural molecular environment, the sequence element corresponding to the epitope may be flanked by amino acids as found in the naturally occurring protein sequence of the soybean protein. In case the epitope is flanked by different amino acid sequences in different soybean varieties, corresponding peptides may be used to evaluate the influence of the adjacent amino acids to the allergenic potential of the epitope. Additionally, functional molecules e.g. for coupling or readout may be added at the N- or C- terminus of the peptide.

In a preferred embodiment, each epitope is selected from one of the groups comprising group 1 (β-conglycinin) consisting of SEQ ID NO.: 1 - 93, group 2 (defensin) consisting of SEQ ID NO.: 94 - 103, group 3 (Gly m Bd 28K) consisting of SEQ ID NO.: 104, group 4 (glycinin) consisting of SEQ ID NO.: 105 - 250, group 5 (hydrophobic seed protein) consisting of SEQ ID NO.: 251 - 253, group 6 (kunitz trypsin inhibitor) consisting of SEQ ID NO.: 254 - 257, group 7 (P34 thiol protease) consisting of SEQ ID NO.:258 - 259, group 8 (profilin) consisting of SEQ ID NO.: 260 - 274, and group 9 (Pr-10) consisting of SEQ ID NO.: 275 - 283. Although the sensitivity to soybean proteins is known to vary distinctly between different patients, several proteins are considered major allergens, in particular P34 (Gly m1), β-conglycinin (Gly m5) and glycinin (Gly m6). β-conglycinin and glycinin are storage proteins and constitute 70 to 80 % of total seed protein. Both are regarded good diagnostic markers for soybean protein sensitivity (Masilamani et al., 2012). β-conglycinin comprises three individual subunits (α, α', β), which were all found to react with IgE. Interestingly, wild and cultivated soybean plants were found to vary distinctly in these subunits (Natarajan 2014). Glycinin comprises five subunits, which were also all found to be recognized by IgE from soybean protein sensitive persons. P34 is a soybean vacuole protein (hull protein) with high sequence similarity to thiol proteases of the papain family, which are known to potentially induce allergic reactions in human. In general, several soybean allergens show distinct similarity to allergens of other organisms. For example, P34 is related to DERp1 from dust mite, profilin (Gly m3) is related to the profilin family of proteins in birch pollen, Gly m5/6 is closely related to ARAh1/3 of peanut and Pr10 (Gly m4) is highly homolog to BETV1 and other major allergens of cross reactivity with birch pollen. Thus, compilations covering sequence elements corresponding to epitopes of the above mentioned groups are suitable to provide detailed information not only on soybean sensitivity but also regarding sensitivity to allergens from other organisms. In particular, when providing screening tests to identify soybean protein sensitive patients, the compilation of peptides may cover at least five, preferably all of these proteins. Accordingly, in a particularly preferred embodiment, the compilation comprises at least nine different peptides and each peptide comprising at least one sequence element corresponding to an epitope selected from each of the groups comprising group 1 (β-conglycinin) consisting of SEQ ID NO.: 1 - 93, group 2 (defensin) consisting of SEQ ID NO.: 94 - 103, group 3 (Gly m Bd 28K) consisting of SEQ ID NO.: 104, group 4 (glycinin) consisting of SEQ ID NO.: 105 - 250, group 5 (hydrophobic seed protein) consisting of SEQ ID NO.: 251 - 253, group 6 (kunitz trypsin inhibitor) consisting of SEQ ID NO.: 254 - 257, group 7 (P34 thiol protease) consisting of SEQ ID NO.:258 - 259, group 8 (profilin) consisting of SEQ ID NO.: 260 - 274, and group 9 (Pr-10) consisting of SEQ ID NO.: 275 - 283.

In a preferred embodiment, each epitope is selected from group 10 consisting of SEQ ID NO.: 18, 22, 29, 34, 37, 60, 65, 67, 79, 85, 87, 91, 99, 95, 94, 92, 103, 117, 131, 110, 126, 152, 153, 168, 173, 172, 183, 188, 200, 227, 233, 237, 250, 251, 252, 253, 254, 255, 257, 258, 262, 271, 261, 276, 281, 274, 309, 311, 319, 307, 294, 305, 298, 284, 291, 290, 328, 322, 323, 331, 332, 333, 334, 335, 336, 337, 339, 340, 345, 346 and 351.. Although the immune status to major allergenic soybean proteins varies distinctly between patients, statistic analyses enabled by the use of the specific phage display library, made it possible to identify certain epitopes that are recognized by the majority of patients (Table 3).

**Table 3: Epitopes recognized by the majority of patients:**

| Protein | Epitope | SEQ ID NO. |
|---|---|---|
| beta conglycinin alpha chain (X17698) | QACHAR | 18 |
| | RPQHPEREP | 22 |
| | EEHEQ | 29 |
| | EEQQRES | 34 |
| | EEGQQQG | 37 |
| beta conglycinin alpha' chain (M13759.1) | SEEEQDERE | 60 |
| | ESQESEGSE | 65 |
| | NKRSQQLQ | 67 |
| beta conglycinin beta chain (S44893) | IERQVQ | 79 |
| | EEINRV | 85 |
| | ELSKEQ | 87 |
| | EEEPLE | 91 |
| Defensin (Z13956.1) | HGLCNRDHN | 99 |
| | RVCESQS | 95 |
| | TEGRVC | 94 |
| | VFASQV | 92 |
| Gly m BD 28K | QEEDEE | 103 |
| Glycinin G1 (AB113349.1) | PQQPQQR | 117 |
| | QQRPQE | 131 |
| | SREQPQ | 110 |
| | KYQQEQ | 126 |
| Glycinin G2 (D00216.1) | GRSQR | 152 |
| | QDRHQK | 153 |
| | QQKGQS | 168 |
| Glycinin G3 (X15123.1) | QQQRPE | 173 |
| | PTEEQ | 172 |
| Glycinin G4 (X05652) | RNGLHS | 183 |
| | IRHFNE | 188 |
| | PRQEEP | 200 |
| Glycinin G5 (M10962.1) | IEHPET | 227 |
| | KWQEQE | 233 |
| | EEEDQP | 237 |
| Hydrophobic Seed Protein (A29385) | LGGSL | 250 |
| | DDCCAL | 251 |
| | NSCGRS | 252 |
| Kunitz trypsin inhibitor (S45092.1) | GNERC | 253 |
| | RAAPTG | 254 |
| | LCVGIP | 255 |
| p34 thiol protease (J05560.1) | VITQVK | 257 |
| | KYQGGC | 258 |
| Profilin (AJ223981.1) | QGEPGAV | 262 |
| | GSVWAQ | 271 |
| | EPGSL | 261 |
| PR10 (X60043.1) | VENVEGN | 276 |
| | GSAGKLT | 281 |
| | SIDEA | 274 |
| 2s Albumin (BT091363.1) | QHQQES | 309 |
| | ESCREQL | 311 |
| | NQSEQLEGKE | 319 |
| 2s Albumin (BT091363.1) | IQCDLS | 307 |
| | LFCIAHTCS | 294 |
| | NQSEELEEK | 305 |
| | KQLQGVN | 298 |
| 18 kD Seed Maturation Protein (AJ574791.1) | ATVQEKAER | 284 |
| | HGTGQPTGH | 291 |
| | HQTSA | 290 |
| Albumin 1_(AJ574791.1) | HPNLCQSD | 328 |
| | NGACSPFE | 322 |
| | PPCRSRD | 323 |
| Albumin 1 (AJ223037.1) | PPCRSS | 331 |
| | VDEHP | 332 |
| | SDSEA | 333 |
| Albumin 1 (AJ223037.1) | EGTSSAKLT | 334 |
| | RCVPIA | 335 |
| Lectin (K00821.1) | LAPIDT | 336 |
| | TKPQT | 337 |
| | DPPNPHI | 339 |
| | DTFRN | 340 |
| Seed biotinylated protein (U59626.1) | KESQRE | 345 |
| | ANVVG | 346 |
| | EAQRELE | 351 |

These epitopes are particularly preferred for providing a general immune status test, because it can be expected that every sensitive person will respond to at least one of these epitopes. For example the epitopes RNQACHA (SEQ ID NO.: 17), EIPRP (SEQ ID NO.: 20), NSERD (SEQ ID NO.: 15) have been recognized by 15/23 patients resp. 3/23 resp 7/23. All three epitopes have been recognized just by one patient, two epitopes (SEQ ID NO.: 15, SEQ ID NO.: 17) by 5/23 patients.

In a further aspect, the invention relates to an analysis tool comprising at least five different peptides, each peptide comprising at least one sequence element corresponding to an epitope selected from the group consisting of SEQ ID NO.: 1 - 354. The term "analysis tool" as used herein refers to means and products for chemical, biochemical or medical analysis of samples, e.g. biologic samples. Such tools include devices (e.g. microarray, multi-well plates), as well as reagents (e.g. compositions comprising free or bead-bound peptides) suitable for single or multiplex analyses. When presented on the surface of a chip or beads, the peptides can interact with substances, e.g. other molecules, brought into contact with the surface. In a peptide array, for example, multitudes of identical peptides are deposited in spots, each spot representing a different peptide. In case of a bead based analysis tool, identical peptides may be conjugated to the surface of a single bead. The peptides can be attached to a surface using hydrophilic linker moieties to avoid sterical hindrance, which might influence the interaction between the peptide and the sample. The analysis tools of the invention are for example suitable for diagnostic applications such as determining a patient's immune status to soybean proteins. Serum derived from a person suspected of being sensitive to soybean protein is brought into contact with the analysis tool to allow the IgE or other antibody classes of the patient to interact with and bind to the peptides. The binding of the antibodies can be specifically detected and analyzed. Depending on the aim of the diagnosis, the analysis tool may cover different epitopes. For example, for a general initial screening, the analysis tool may contain peptides comprising sequence elements corresponding to epitopes of some or all major allergens of soybean. Additionally, the analysis tool may contain peptides comprising sequence elements corresponding to epitopes of allergens of other organisms to provide a more comprehensive survey. For a more detailed analysis, all major soybean allergens should be represented by the analysis tool. When used in analysis tools such as microarrays or bead based assays, the peptides preferably consist of 5 to 30, more preferred of 8 to 20, most preferred of 8 to 15 amino acids.

In a further aspect, the invention relates to an *in vitro* method for determining a patient's immune status to soybean allergens, comprising the steps providing at least five different peptides, each peptide comprising at least one sequence element corresponding to an epitope selected from the group consisting of SEQ ID NO.: 1 - 354, contacting each peptide with a sample comprising antibodies derived from the patient, and detecting an interaction of each peptide with the sample. The term "immune staus" refers to the presence of antibodies in the blood of a person, in particular to the presence of antibodies directed to soybean proteins. The method allows revealing whether a patient produces antibodies recognizing soybean proteins and, thus, is expected to show allergic reactions when consuming soybean containing products and at least one of these antibodies is an IgE antibody. Moreover, the method is suitable to specifically identify the epitopes of soybean proteins, to which the patient reacts. This may provide further information about the strength of the allergy and may indicate whether the patient is likely to react to allergens from other organisms as well, since related allergenic epitopes are found in proteins of various organisms. Dependent on the distinct aim of using the method, various numbers of peptides comprising sequence elements corresponding to different epitopes may be employed. For example, to merely test whether a person in question is likely to show any allergic reaction to soybean proteins, a few peptides (e.g. five to ten) representing epitopes of at least two, preferably five major allergenic proteins (epitopes of groups 1 to 9) may be sufficient. For a more detailed analysis on the antibody status of the patient, peptides comprising sequence elements corresponding to epitopes selected from all major soybean allergens, preferably from all potentially allergenic soybean proteins, may be covered. The results of such a comprehensive analysis can help to decide about a patient's personalized treatment, e.g. form the basis for a subsequent desensitization protocol.

For carrying out the method, the peptides can be provided as a microarray or bead-based multiplex assay. For a simpler approach, the method may be used in form of a lateral flow test. In any case, the peptides are incubated with the patient's sample such that the molecules contained in the sample, in particular the patient's antibodies, can interact and bind to the peptides. Binding of the antibodies is subsequently detected, e.g. using secondary antibodies directed against human immunoglobulins. In case the secondary antibodies are fluorescently labelled, the analysis can be performed using fluorescence scanners and suitable data analysis software, which allows for a fast and standardized readout providing reliable results in reasonable time.

In a preferred embodiment, the sample is a body fluid sample, preferably a blood sample, more preferred a serum sample. For analyzing a patient's immune status to soybean allergens, antibodies derived from the patient are contacted with peptides comprising sequence elements corresponding to the identified epitopes. The antibodies may be derived from the patient through a blood or serum sample, which are usually used for testing on IgE. Serum samples are particularly preferred since they are devoid of several blood compounds that could interfere with peptide binding. Moreover, blood shows high autofluorescence and is therefore disadvantageous in case a fluorescent readout is used. In addition, samples derived from lymphocyte transformation tests may be used (Pichler and Tilch, 2004).

Alternatively to determining the patient's immune status using a body fluid sample, allergenic sensitivity is commonly tested using skin allergy tests such as skin prick test, skin scratch test, intrademic test or patch test. In all these tests the potential allergen is applied onto or beneath the patient's skin. In case the patient is sensitive to the allergen, i.e. produces antibodies recognizing the allergen, the patient will develop rash and/or urticaria in the proximity of the applied allergen. Since the peptides of the invention comprise sequence elements corresponding to epitopes that can be specifically recognized by IgE of soybean protein sensitive patients, they are suitable for respective allergy tests. Accordingly, in a further aspect, the invention relates to a method for determining a patient's sensitivity to soybean allergens, comprising the steps providing a compound comprising at least two identical and/or different sequence elements corresponding to an epitope selected from the group consisting of SEQ ID NO.: 1 - 354, contacting the patient with the compound, preferably by applying the compound to and/or beneath the patient's skin, and detecting a reaction, preferably rash and/or urticaria. Upon application, e.g. beneath the patient's skin, the epitope is recognized by IgE antibodies localized on mast cells, inducing dimerisation of the IgE receptors and subsequent degranulation of the mast cell. Thus, for application in allergy tests, at least two peptides comprising sequence elements corresponding to epitopes, preferably derived from the same allergenic protein, are combined into a single compound. This can be achieved, for example, by providing a polypeptide comprising several identical and/or different sequence elements corresponding to epitopes, e.g. a homo- or heterodimer or polymer, respectively. Alternatively, the compound may be provided by associating the peptides with a cargo molecule. Suitable cargo molecules include chemical compounds as well as surface molecules such as nanoparticles (e.g. iron oxide nanoparticles or albumin nanoparticles), liposomes, microparticles or microbubbles.

In a further aspect, the invention relates to a kit comprising at least one composition, containing a compound comprising at least two identical and/or different sequence elements corresponding to an epitope selected from the group consisting of SEQ ID NO.: 1 - 354. A composition of the kit may represent one single epitope by only containing compounds that comprise a sequence element corresponding to said epitope. Alternatively, the composition may represent various epitopes of a single soybean protein by containing compounds that comprise various sequence elements corresponding to different epitopes of the soybean protein of interest. The various sequence elements may be comprised in a single compound or allocated to different compounds. Finally, the composition may cover epitopes of several or even all allergenic soybean proteins. The kit is suitable to provide potential soybean protein allergens for various applications, in particular skin allergy tests. Accordingly, the composition may be a composition suitable for diagnostic tests such as skin prick test or patch test. The concentration, in which the peptides are provided and applied to the skin, should be according to biological standardization, e.g histamine equivalent potency (HEP/ml) or allergy units (AU/ml) as defined by the Food and Drug Administration of the United States.

In a further aspect, the invention relates to a method for detecting at least one soybean allergen in a substance, comprising the steps providing at least two different peptides, each peptide comprising at least one sequence element corresponding to an epitope selected from the group consisting of SEQ ID NO.: 1 - 354, raising at least one antibody against each peptide, contacting the substance with the antibodies, and determining binding of the antibodies to the substance. Detection of the soybean allergen may include a qualitative detection and/or a quantitative analysis of the amount of soybean protein present in the substance. In a first step, epitopes of interest are selected, preferably representing one or more of the major allergenic soybean proteins. Depending on the aim of the analysis, different epitopes of various soybean products may be covered. To gain comprehensive information on the presence of potentially allergenic proteins in a sample, e.g. of a food product, at least one epitope of each major allergenic protein, preferably of each potentially allergenic protein should be detected. To exclude the presence of any soybean remnants e.g. for baby nutrition, the method may be performed using antibodies for the most frequently recognized or each of the identified epitopes. Accordingly, depending on the number of epitopes and allergens to be covered by the method, at least 5 peptides, preferably at least 10 peptides, more preferred at least 20 peptides, most preferred at least 50 peptides are provided and antibodies raised against them. Finally, epitopes may be used that are recognized by most patients (group 10, table 3) giving a good first impression on the immune status of the patient. After selecting the epitopes, peptides are produced comprising at least one of the selected epitopes. Each peptide may consist of the epitope sequence(s) or may comprise additional amino acids on one or both sides of the epitope(s). The additional amino acids may for example correspond to the amino acids flanking the epitope in the natural protein or in the original peptide identified by the phage display. Alternatively, they may be individually selected e.g. to optimize antibody formation. For example, to avoid hindrance of interactions between the peptide and an antibody, flanking amino acids may be selected having small residues, such as glycine, alanine or serine. The size of the peptide amounts preferably to 5 to 30, more preferred to 8 to 20, most preferred to 8 to 15 amino acids. For raising antibodies, preferably monoclonal antibodies, methods well known in the art may be employed. To detect soybean proteins or protein remnants in a substance, the substance is contacted with the antibodies to allow interaction and binding, which is then determined or even quantified e.g. by use of fluorescent secondary antibodies and fluorescence scanners. In summary, the method allows determining the allergenic potential of the substance by detecting allergenic proteins or remnants of soybean proteins therein.

In a preferred embodiment, the substance is a human or animal food product, a dietary supplement or a cosmetic product. Soybean protein and preparations thereof are not only processed in human and animal food products but also in dietary supplements and cosmetics, e.g. in the form of soybean extracts or soybean oil. Since allergy reactions do not only occur upon oral ingestion but also upon contact to skin, a test for potentially allergenic proteins and protein remnants is likewise needed for cosmetic products and ingredients.

In a preferred embodiment, the soybean allergen is selected from the group consisting of profilin, P34 thiol protease, kunitz trypsin inhibitor, hydrophobic seed protein, glycinin, Gly m BD 28K, defensin, β-glycinin and PR10. These major allergenic soybean proteins are known to induce strong allergic reactions and are, thus, considered most crucial. Therefore, preferably each of these proteins is represented by at least one epitope. For testing products with particularly strict safety requirements, e.g. baby nutrition, all epitopes of the major allergenic soybean proteins or even of all potentially allergenic soybean proteins (Table 1) may be detected.

In a further aspect, the invention relates to a method for producing an immunoassay product, comprising the steps providing at least two different peptides, each peptide comprising at least one sequence element corresponding to an epitope selected from the group consisting of SEQ ID NO.: 1 - 354, raising at least one antibody against each peptide, and compiling the antibodies to provide the immunoassay product. Processed food and other industrial products contain soybean protein in various quantities and different conditions. For example, processing of soybean seeds or extracted protein leads to denaturation and modification of the soybean proteins, which can eliminate epitopes and thereby reduce the allergenic potential of the product. Therefore, methods are already used and continuously developed that aim on reducing the allergenic soybean proteins e.g. by food processing techniques (Wilson et al., 2005) or genetic modification of soybean plants (WO 2010/087888). Moreover, wild (*glycine soja*) and cultivated (*glycine max*) soybean plants as well as different varieties of *glycine max* were found to have different variants of potentially allergenic proteins. The proteins differ at least slightly in their amino acid sequences and therefore vary in their allergenic potential. This prompted the development of specific *glycine max* varieties, which express reduced amounts of potentially allergenic proteins, e.g. P34 (Joseph et al., 2006). Despite these efforts, a reliable and complete destruction or removal of all allergenic proteins from soybean products has not yet been achieved. Therefore, it is necessary to asses the remaining allergenic potential of food and other products comprising soybean proteins. So far, soybean protein tests are mainly based on antibodies raised against the complete soybean proteome. Sometimes polyclonal antibodies are even directly derived from animals or humans sensitive to soybean proteins. However, such antibodies or sera do not necessarily provide reliable results. Antibodies usually only recognize the particular protein or amino acid residues they have been raised against. Therefore, antibodies raised against native proteins may no longer recognize soybean proteins having an altered confirmation due to industrial processing techniques. Thus, the allergenic epitope, although present, may not be bound by the respective antibodies. Moreover, antibodies or sera raised against the entire proteome of a specific variety may fail to detect allergenic proteins of a different variety. In contrast, antibodies raised against individual and defined epitopes of about four to ten amino acids have a high potential to recognize allergenic proteins or amino acid residues independently of conformational changes in the molecule (e.g. due to food processing techniques) or adjacent amino acid sequences (that may vary according to soybean variety). Thus, the identified epitopes and peptides comprising sequence elements corresponding to these epitopes are particularly suitable for providing an immunoassay product for detecting potentially allergenic soybean proteins. The term "antibody" as used herein refers to natural as well as artificial immunoglobulins and derivatives thereof, including polyclonal antibodies, monoclonal antibodies, antibody fragments, antigen-binding fragments (Fab) and single chain variable fragments (scFv).

For providing an immunoassay product, the peptides and antibodies may be selected and provided as described above. The antibodies are compiled to provide the immunoassay product, which is suitable for testing a sample for the presence of soybean proteins, e.g. by contacting the sample with the antibodies and detecting possible interactions.

In a preferred embodiment compiling the antibodies comprises immobilizing them onto a solid surface, e.g a chip, a multiwell plate or beads. Thereby, they can be easily contacted with the sample to be tested and the non-bound remnants of the sample removed by washing if necessary. This allows a fast and specific detection suitable to be implied into industrial processes.

In a preferred embodiment, the immunoassay product is a microarray, a bead-based assay product, an ELISA plate or a lateral flow test. Microarrays and bead-based assay products are particularly preferred for detecting the presence of a multitude of different epitopes, because they allow the simultaneous use and specific readout of many antibodies directed against different epitopes. ELISA (enzyme-linked immunosorbent assay) plates, usually multi-well or microtiter plates, also allow for a simultaneous testing of a substantial number of different antibodies. Lateral flow tests, in contrast, provide a particular fast readout, however, usually cover only one or few different antibodies. Depending on the number of epitopes to be detected and the time available, different immunoassay products may be advantageous. All of the assays are fully established standard methods allowing a sensitive and fast readout of antibody-antigen interaction. Moreover, readout systems have been developed, in particular for microarrays and ELISA that allow a fast and fully automated analysis.

In a further aspect, the invention relates to a method for determining the allergenicity of a soybean variety by detecting the presence of at least two epitopes in a sample of the variety, wherein the epitopes are selected from the group consisting of SEQ ID NO.: 1 - 354. In addition to wild (*glycine soja*) and cultivated (*glycine max*) soybean, several varieties of *glycine max* are known. Additionally, plant varieties expressing reduced levels of allergenic proteins (e.g. P34, Joseph et al., 2006) or alternative amino acid sequences of the allergenic proteins (e.g. WO 2010/087888) were specifically developed to reduce the allergenic potential. By detecting the presence and, preferably, also the quantity of the allergenic epitopes or proteins, the allergenicity of a particular soybean variety can be determined. This is of particular importance, since the use of soybean varieties with reduced allergenicity could contribute to a total reduction of newly developed allergies.

In a preferred embodiment, the presence of the epitopes is detected by an immunoassay or mass spectrometry. Suitable immunoassays comprise, for example, ELISA and flow rate assays that may be provided by raising antibodies specific to the identified epitopes. In addition, mass spectrometry can be used to directly analyze the proteome of soybean varieties for the presence or the quantity of epitopes and the corresponding soybean proteins, respectively (e.g. Houston et al., 2011).

In a further aspect, the invention relates to the use of a peptide comprising a sequence element corresponding to an epitope selected from the group consisting of SEQ ID NO.: 1 - 354 for providing a molecule binding to a protein or peptide comprising the epitope, in particular an antibody. The term "molecule binding to a protein or peptide comprising the epitope" refers to any molecule that is able to specifically form a complex with the epitope and/or a peptide or protein containing a sequence element corresponding to the epitope due to intermolecular forces. Peptides consisting of, or comprising one or more sequence elements corresponding to the epitopes of a specific soybean protein may be used to identify molecules interacting and binding to the epitope and the original protein, e.g. by screening molecule libraries. Likewise, the peptides may be used to raise antibodies specifically recognizing and binding the epitope. Thus, in contrast to commonly used anti-soybean antibodies or sera, the molecules provided by using the peptides of the invention, specifically detect the epitope(s) of the protein responsible for allergic reactions to soybean. Accordingly, the binding molecules are particularly suitable for detecting allergenic proteins of soybean, for example in food or cosmetic products.

In a further aspect, the invention relates to a method for generating a variant of an allergenic soybean protein having a reduced allergenic potential, comprising the steps providing an amino acid sequence of at least one epitope selected from the group consisting of SEQ ID NO.: 1 - 354, altering the amino acid sequence of the at least one epitope as to eliminating the structure of the epitope, and generating a protein or peptide comprising the altered amino acid sequence. The term "variant of an allergenic soybean protein" refers to a variant of a soybean protein, which is known to induce allergenic reactions, that comprises one or more epitopes that have a different amino acid sequence compared to the natural protein. The term "a protein having reduced allergenic potential" also comprises proteins having no allergenic potential at all. The identified soybean epitopes (Tables 1 and 2) are suitable to generate variants of natural soybean proteins with an amino acid sequence that is altered in a way that binding of IgE antibodies of a soybean sensitive patient do not bind or bind to a reduced extent. This can be achieved by replacing or removing amino acids within one or more of the identified epitopes (table 1), which are essential for IgE binding. For example, to support the selection of suitable residues, epitopes from the group consisting of SEQ ID NO.: 1 - 354 can be mapped onto existing structures of the respective soy protein, or structures of homologous variants of the protein from other, favourably closely related, species available in the protein structure databases. One or more selected amino acids within the epitope's sequence may be replaced by amino acids of different physiochemical properties, such as size, charge or polarity. As a result, the modified epitope, and more importantly, proteins comprising the modified epitope are not recognized by epitope specific antibodies. In case an allergenic soybean protein was found to comprise several epitopes, one or more epitopes may be eliminated. For example, to determine the patient's antibody specificity, all epitopes found to induce cross-reactivity with antibodies against proteins of other species (e.g. birch pollen) may be eliminated

Proteins with one or more epitopes eliminated, are suitable for analysing antibody - epitope interaction, as they allow determining the participation of individual amino acids in this interaction. Additionally, they provide important negative controls for analysis tools based on epitope specific antibodies. By including respective variants of soybean proteins, antibody binding and specificity can be evaluated in detail.

In addition, the method can be employed for generating derivatives of natural soybean proteins comprising one or more modified epitopes. Such derivates are, for example, suitable for immunotherapeutic applications. Modified allergens, i.e. derivatives of allergens suitable to provoke an IgG immunoresponse, but lacking the allergenic epitopes responsible for IgE reactivity, can be used as recombinant vaccines to reduce or even prevent allergenic reactions (Valenta and Niederberger, 2007).

In a further aspect, the invention relates to a peptide or protein comprising an modified variant of at least one epitope selected from the group consisting of SEQ ID NO.: 1 - 354, wherein the original structure of the epitope was eliminated.

### Examples

### Affinity selection and planning

24 µg of serum protein from patients with soy allergy were diluted in 2.5 ml PBS and coated in protein binding Immuno^{™}Tubes with maxisorp^{™} for 1 h at 4°C at 18 rpm (negative control: PBS). The coated tubes were washed with 2.5 ml blocking buffer (5% NFDM/1xPBS) and incubated with 4 ml blocking buffer at 4°C for at least 15 min (18 rpm). The tubes were washed three times with 4 ml wash buffer I (1xPBS). 1 ml blocking buffer containing 30 µg of non-allergic serum protein and the phage library ENTE1 was added to each tube. The tubes were incubated for 2 h at 4°C (18 rpm). After incubation, the tubes were washed 5 times with 1 ml 0.1% Tween/1xPBS and subsequently with 0.5% Tween/1xPBS. Then the bound phages were eluted using 1 ml of elution buffer (0.1 M Glycin.HCl pH 2.2). The tubes were incubated 5 min and vortexed several times. The eluate was immediately neutralized with 200 µl neutralization buffer (1 M Tris.HCl pH 8.2). In the first selection round 4 x 10¹¹ cfu of the ENTE1-library were used per tube. In the second selection round 1000 x the recovered phages were inserted.

### Infection of TG1 (λ) with eluted phage

TG1 (λ) cells were grown in 250 ml baffled culture flasks in 10 ml dYT at 37°C until they reached an OD₆₀₀ about 0.5 - 1.0. The cells were incubated with eluted phage for 20 min at 37°C with 100 rpm agitation. After incubation, the solution was immediately poured on dYT-agar plates (25 x 25 cm) containing 200µg/ml ampicillin and incubated overnight at 30 4°C.

### Packaging of phage from infected cells

After overnight incubation the colonies were resuspended in 25 ml dYT containing 1 x 10¹⁰ cfu/ml M13K07. The cells were diluted in 25 ml dYT containing 500 µg/ml ampicillin and 1 x 10¹⁰ cfu/ml M13K07 using a 250 ml baffled culture flasks to obtain a cell density of OD₆₀₀ 0.5. The cells were incubated for 1 h at 37°C with 180 rpm agitation. Afterwards the culture was growing about 13 h at 30°C with 180 rpm agitation.

### Purification of phage

The infected bacteria cells were centrifuged at 20,000 x g for 20 min at 4°C. ¼ volume of cold PEG/NaCl (PEG/NaCl (20% (w/v) PEG 6000, 2.5 M NaCl) solution was added to the supernatant and incubated for at least 1 h on ice. Phages were collected by centrifugation (30 min, 4°C, 14,000 x g) and the pellet was resuspended in 1 ml 1xPBS. The solution was centrifuged at 14,000 x g for 10 min and the supernatant recovered and incubated with ¼ volume of PEG/NaCl (PEG/NaCl (20% (w/v) PEG 6000, 2.5 M NaCl) solution for at least 20 min on ice. Phages were again collected by 30 min centrifugation at 14,000 x g, 4°C and the pellet of phage particles resuspended in 1 ml PBS. The purified phage suspension was stored at 4°C for several days.

### Identification of selected epitopes

For data analysis the LibDB software was used. Due to the design of the trinucleotide based library, sequences with potential errors were removed. All valid sequences were written into a database and a database of 3-5mer motifs was generated. The observed frequency as well as statistically and theoretically expected values were provided for each motif in the database.

### Validation of epitopes using a peptide array

The peptide micro arrays were designed to represent nine predominant soybean allergens, named beta-conglycinin, glycinin, defensin, lectin, PR-10, Gly m 1, P34, Kunitz trypsin-inhibitor and profilin. Each allergen amino acid sequence was divided into 15mer fragments with 4 amino acids overlap and a chip was designed bearing duplicates of every peptide. The slides were purchased from the company PEPperPRINT, which synthesized the peptides in a cycle of synthesis, where amino acid micro particles are printed directly on a glass slide based on solid phase Fmoc chemistry.

To analyze the IgE reactivity of soybean allergic patients to the soybean antigen peptides displayed on the array, the glass slides were prepared as follows. Each slide was washed in staining buffer (PBS, 0.05% (v/v) Tween 20, 0.1% (w/v) BSA, pH 7.4) for 10 min at room temperature and afterwards blocked for 30 min in blocking buffer (PBS, 0.05% (v/v) Tween 20, 1% (w/v) BSA, pH 7.4). Incubation of the slides in patient sera diluted 1:50 in staining buffer was performed over night at 4°C. After 3 times washing in standard buffer (PBS, 0.05% (v/v) Tween 20, pH 7.4) the slides were shaken at 200 rpm for 30 min in a goat-anti human IgE antibody solution (Invitrogen; 1:5000 diluted in staining buffer). Detection was performed by incubating the array after 3 additional washing steps in a solution containing the secondary donkey-anti goat antibody labelled with Cy5 (Abcam, 1:5000 diluted in staining buffer) for another 30 min at room temperature. The fluorescence signals on the array were detected in a micro array reader at 635 nm.

Data evaluation was performed using the PepSlide Analyser software from PEPperPRINT, where signal intensity was directly connected to the peptide spot. Additionally the software in a first step performed the subtraction of the local background signal for every spot, resulting in a normalization of the different background levels for the tested sera. Further for all sera an overall threshold level from 30 fluorescence signal intensity units was set, where signal intensities above this threshold were assumed as positively recognized by the patient sera. Peptides detected by a minimum of 25% of the 16 analyzed patient sera were defined as soybean related allergenic epitopes. Peptide sequences tested positive for IgE binding (as a measure of allergy relevance) in a peptide array were entered in the software and matched/aligned with the motives in the database. A sequence was considered to represent a potential epitope, if the enrichment value of the 4mer motives was different in comparison with the naïve library. The enrichment value of the motive in the serum was higher than the enrichment value of the motive in the naïve library. All sequences with the identified motive were saved in a fastq-file, which was aligned to soybean proteins using the software MegAlign Pro 12. If the 4mer motive could be expanded to a 5mer or 6mer motive, the potential epitope was validated for inclusion into table 1.

### Identification of novel epitopes

Sequences of soybean proteins were matched with the motives in the database and the statistic listed. Then, a graph of enrichment values of the naïve library and the serum was generated in Excel. A positive difference of the values of the naïve library and the serum indicated a relevant epitope. In the second panning more than one sequence should be enriched. All sequences with the identified motive were saved in a fastq-file, which was aligned to soybean proteins using the software MegAlign Pro 12. If the 4mer motive could expand to a 5mer or 6mer motive, the potential epitope was validated.

For example, the motive "QHQQ" was identified in the first panning contained in
>2013_S1_2.Panning_seq_10|count=250: GIEPCSKYSQHQQHVQN (SEQ ID NO.: 706) and
>2013_S1_2.Panning_seq_11|count=122: GEIMCWREVVTQHQQHA (SEQ ID NO.: 707).

In the second panning, the motive was significantly enriched with a total of 825 counts in comparison to 407 counts in the first panning (Serum 1_1.Panning round: count 407, Serum 1_2.Panning round: count 825).

### Validation of an epitope using magnetic beads

200 µl Dynabeads^{®} M-270 Carboxylic Acid (Invitrogen) were washed 3 times with 1 ml PBS. The beads were activated by adding of 200 µl 0.4 M 1-ethyl-3-(3-dimethylaminopropyl) carbodiimide (EDC) and 200 µl 0.1 M N-hydroxysuccinimide (NHS). After 5 minute incubation at room temperature with agitation, the beads were washed once with 1 ml 10 mM sodium acetat, pH 4.5. 100 µg streptavidin in 100 µl 10 mM sodium acetat, pH 4.5 were added. After 30 min incubation at room temperature with agitation, beads were blocked with 500 µl 0.1 M ethanolamine pH 8.5 for 10 minutes at room temperature with agitation. The beads were washed 3 times with 1 ml PBS and resuspended in 200 µl PBS.

Potential epitopes were synthesized as peptides and coupled with 10 µl streptavidin coupled beads by adding of 3 µg (= 3 µl) peptide for 1 h at room temperature with agitation. Beads were blocked with 500 µl blocking buffer (15 mM Tris, 0.14 M NaCl, 1% BSA, 0.05% Tween) for 30 minutes at room temperature with agitation. After three washing steps with 1 ml PBS, the beads were incubated with 10 µl serum from a patient with soy allergy for 1 h at room temperature with agitation and afterwards washed 3 times with 1 ml 0.1 % Tween/PBS and resuspended in 10 µl SDS-loading Dye.

Immediately a SDS-Page with a 12% gel at 180 V for 50 minutes was performed. The western blot was performed with 0.2 µm nitrocellulose membrane and 260 mA und 200 V for 2 h at 4°C. After blotting the membrane was blocked with 0.1 % Tween/PBS for 1 hour at room temperature. The antibodies (Anti-human polyvalent immunoglobulins) were diluted 1:10000 in 10 ml 0.1% Tween/PBS. After incubation for 1 h at room temperature, three washing steps with 0.1% Tween/PBS and one with PBS were performed. Antibodies were detected using DAB-solution (10 ml 0.1m Imidazole, pH 7, 50 µl 1 % Diaminobenzidine, 5 µl 30% H₂O₂).

### References

EP 14 166 662.8
WO 2010/087888
Joseph LM, Hymowitz T, Schmidt MA, and Herman EM. Evaluation of glycine germplasm for nulls of the immunodominant allergen P34/GlymBd30k. Crop Sci. 2006 46, 1755-1763.
Houston NL, Lee DG, Stevenson SE, Ladics GS, Bannon GA, McClain S, Privalle L, Stagg N, Herouet-Guicheney C, MacIntosh SC, Thelen JJ. Quantitation of soybean allergens using tandem mass spectrometry. J Proteome Res. 2011 Feb 4;10(2):763-73.
Masilamani M, Commins S, Shreffler W. Determinants of food allergy. Immunol Allergy Clin North Am. 2012 Feb;32(1):11-33.
Natarajan S S. Analysis of Soybean Seed Proteins Using Proteomics. Journal of Data mining in Genomics and Proteomics. 2014, 5:1.
Pichler WJ, Tilch J. Allergy. 2004 Aug;59(8):809-20.
Valenta R, Niederberger V. Recombinant allergens for immunotherapy. J Allergy Clin Immunol. 2007 Apr;119(4):826-30. Epub 2007 Mar 1.
Wilson S, Blaschek K, de Mejia E. Allergenic proteins in soybean: processing and reduction of P34 allergenicity. Nutr Rev. 2005 Feb;63(2):47-58.
Yang W W, Gonzales de Mejia E, Zheng H, and Lee Y. Soybean Allergens: Presence, Detection and Methods for Mitigation in Soybean and Health, InTech 2011, ISBN 978-953-307-535-8.

## Claims

1. A compilation comprising at least five different peptides, each peptide comprising at least one sequence element corresponding to an epitope selected from the group consisting of SEQ ID NO.: 1 - 354.

2. The compilation of claim 1, wherein each of the at least five different peptides comprises a sequence element corresponding to a different epitope.

3. The compilation of claim 1 or 2, wherein the compilation comprises at least 10, preferably at least 20, more preferred at least 50 different peptides.

4. The compilation of any one of claims 1 to 3, wherein each epitope is selected from one of the groups comprising
- group 1 (β-conglycinin) consisting of SEQ ID NO.: 1 - 93,
- group 2 (defensin) consisting of SEQ ID NO.: 94 - 103,
- group 3 (Gly m Bd 28K) consisting of SEQ ID NO.: 104,
- group 4 (glycinin) consisting of SEQ ID NO.: 105 - 242509,
- group 5 (hydrophobic seed protein) consisting of SEQ ID NO.: 521 - 253,
- group 6 (kunitz trypsin inhibitor) consisting of SEQ ID NO.: 254 - 257,
- group 7 (P34 thiol protease) consisting of SEQ ID NO.:258 - 259,
- group 8 (profilin) consisting of SEQ ID NO.: 260 - 274, and
- group 9 (Pr-10) consisting of SEQ ID NO.: 275 - 283.

5. The compilation of any one of claims 1 to 4, wherein each epitope is selected from group 10 consisting of SEQ ID NO.: 18, 22, 29, 34, 37, 60, 65, 67, 79, 85, 87, 91, 99, 95, 94, 92, 103, 117, 131, 110, 126, 152, 153, 168, 173, 172, 183, 188, 200, 227, 233, 237, 250, 251, 252, 253, 254, 255, 257, 258, 262, 271, 261, 276, 281, 274, 309, 311, 319, 307, 294, 305, 298, 284, 291, 290, 328, 322, 323, 331, 332, 333, 334, 335, 336, 337, 339, 340, 345, 346 and 351.

6. An *in vitro* method for determining a patient's immune status to soybean allergens, comprising the steps
- providing at least five different peptides, each peptide comprising at least one sequence element corresponding to an epitope selected from the group consisting of SEQ ID NO.: 1 - 354,
- contacting each peptide with a sample comprising antibodies derived from the patient, and
- detecting an interaction of each peptide with the sample.

7. The method of claim 6, wherein the sample is a body fluid sample, preferably a blood sample, more preferred a serum sample.

8. A kit comprising at least one composition containing a compound comprising at least two identical and/or different sequence elements each corresponding to an epitope selected from the group consisting of SEQ ID NO.: 1 - 354.

9. A method for detecting at least one soybean allergen in a substance, comprising the steps
- providing at least two different peptides, each peptide comprising at least one sequence element corresponding to an epitope selected from the group consisting of SEQ ID NO.: 1 - 354,
- raising at least one antibody against each peptide,
- contacting the substance with the antibodies, and
- determining binding of the antibodies to the substance.

10. A method for producing an immunoassay product, comprising the steps
- providing at least two different peptides, each peptide comprising at least one sequence element corresponding to an epitope selected from the group consisting of SEQ ID NO.: 1 - 354,
- raising at least one antibody against each peptide, and
- compiling the antibodies to provide the immunoassay product.

11. The method of claim 10, wherein compiling the antibodies comprises immobilising the antibodies onto a solid surface.

12. The method of claim 10 or 11, wherein the immunoassay product is a microarray, a bead-based assay product, an ELISA plate or a lateral flow test.

13. A method for determining the allergenicity of a soybean variety by detecting the presence of at least two epitopes in a sample of the variety, wherein the epitopes are selected from the group consisting of SEQ ID NO.: 1 - 354.

14. The method of claim 13, wherein the presence of the epitopes is detected by an immunoassay or mass spectrometry.

15. Use of a peptide comprising a sequence element corresponding to an epitope selected from the group consisting of SEQ ID NO.: 1 - 354 for providing a molecule binding to a protein or peptide comprising the epitope.

16. A method for generating a variant of an allergenic soybean protein having a reduced allergenic potential, comprising the steps
- providing an amino acid sequence of at least one epitope selected from the group consisting of SEQ ID NO.: 1 - 354,
- altering the amino acid sequence of the at least one epitope as to eliminating the structure of the epitope, and
- generating a protein or peptide comprising the altered amino acid sequence.


SEQUENCE LISTING

<110> Fraunhofer-Gesellschaft zur FÃ¶rderung der angewandten Forschung e.V.

<120> Soybean allergy related epitopes

<130> F30011

<160> 733

<170> BiSSAP 1.3

<210> 1
<211> 5
<212> PRT
<213> glycine max

<400> 1
Ser Asn Arg Phe Glu
1               5

<210> 2
<211> 6
<212> PRT
<213> glycine max

<400> 2
Phe Lys Asn Gln Tyr Gly
1               5

<210> 3
<211> 6
<212> PRT
<213> glycine max

<400> 3
Phe Asn Ser Lys Pro Asn
1               5

<210> 4
<211> 6
<212> PRT
<213> glycine max

<400> 4
Val Asn Pro Asp Asn Asn
1               5

<210> 5
<211> 5
<212> PRT
<213> glycine max

<400> 5
Leu Ala Ile Pro Val
1               5

<210> 6
<211> 8
<212> PRT
<213> glycine max

<220>

<221> SITE
<222> 2..2
<223> Xaa may be any amino acid

<400> 6
Asn Xaa Pro Gly Arg Phe Glu Ser
1               5

<210> 7
<211> 5
<212> PRT
<213> glycine max

<400> 7
Glu Ala Ser Tyr Asp
1               5

<210> 8
<211> 6
<212> PRT
<213> glycine max

<400> 8
Glu Gln Gln Gln Gly Glu
1               5

<210> 9
<211> 5
<212> PRT
<213> glycine max

<400> 9
Gln Glu Ser Val Ile
1               5

<210> 10
<211> 6
<212> PRT
<213> glycine max

<400> 10
Lys Ser Ser Ser Arg Lys
1               5

<210> 11
<211> 6
<212> PRT
<213> glycine max

<400> 11
Pro Glu Lys Asn Pro Gln
1               5

<210> 12
<211> 6
<212> PRT
<213> glycine max

<400> 12
Phe Val Ile Pro Ala Gly
1               5

<210> 13

<211> 5
<212> PRT
<213> glycine max

<400> 13
Ala Phe Pro Gly Ser
1               5

<210> 14
<211> 7
<212> PRT
<213> glycine max

<400> 14
Cys Leu Gln Ser Cys Asn Ser
1               5

<210> 15
<211> 5
<212> PRT
<213> glycine max

<400> 15
Asn Ser Glu Arg Asp
1               5

<210> 16
<211> 6
<212> PRT
<213> glycine max

<400> 16
Glu Arg Asp Ser Tyr Arg
1               5

<210> 17
<211> 7
<212> PRT
<213> glycine max

<400> 17
Arg Asn Gln Ala Cys His Ala
1               5

<210> 18
<211> 6
<212> PRT
<213> glycine max

<400> 18
Gln Ala Cys His Ala Arg
1               5

<210> 19
<211> 7
<212> PRT
<213> glycine max

<400> 19
Lys Glu Glu Cys Glu Glu Gly
1               5

<210> 20

<211> 6
<212> PRT
<213> glycine max

<400> 20
Glu Ile Pro Arg Pro Arg
1               5

<210> 21
<211> 6
<212> PRT
<213> glycine max

<400> 21
Lys Glu Glu Cys Glu Glu
1               5

<210> 22
<211> 9
<212> PRT
<213> glycine max

<400> 22
Arg Pro Gln His Pro Glu Arg Glu Pro
1               5

<210> 23
<211> 8
<212> PRT
<213> glycine max

<400> 23
Arg Glu Pro Gln Gln Pro Gly Glu
1               5

<210> 24
<211> 6
<212> PRT
<213> glycine max

<400> 24
His Pro Glu Arg Glu Pro
1               5

<210> 25
<211> 7
<212> PRT
<213> glycine max

<400> 25
Gln Gln Pro Gly Glu Lys Glu
1               5

<210> 26
<211> 9
<212> PRT
<213> glycine max

<400> 26
Glu Asp Glu Asp Glu Gln Pro Arg Pro
1               5

<210> 27

<211> 8
<212> PRT
<213> glycine max

<400> 27
Ile Pro Phe Pro Arg Pro Gln Pro
1               5

<210> 28
<211> 6
<212> PRT
<213> glycine max

<400> 28
Pro Arg Gln Glu Glu Glu
1               5

<210> 29
<211> 9
<212> PRT
<213> glycine max

<400> 29
Glu Glu His Glu Gln Arg Glu Glu Gln
1               5

<210> 30
<211> 7
<212> PRT
<213> glycine max

<400> 30
Gly Glu Lys Gly Ser Glu Glu
1               5

<210> 31
<211> 7
<212> PRT
<213> glycine max

<400> 31
Arg Asn Glu Glu Glu Asp Glu
1               5

<210> 32
<211> 6
<212> PRT
<213> glycine max

<400> 32
Glu Asp Glu Glu Gln Gln
1               5

<210> 33
<211> 7
<212> PRT
<213> glycine max

<400> 33
Glu Glu Gln Gln Arg Glu Ser
1               5

<210> 34

<211> 6
<212> PRT
<213> glycine max

<400> 34
Arg Glu Ser Glu Glu Ser
1               5

<210> 35
<211> 8
<212> PRT
<213> glycine max

<400> 35
Val Leu Phe Ser Arg Glu Glu Gly
1               5

<210> 36
<211> 7
<212> PRT
<213> glycine max

<400> 36
Glu Glu Gly Gln Gln Gln Gly
1               5

<210> 37
<211> 8
<212> PRT
<213> glycine max

<400> 37
Leu Arg Ser Arg Asp Pro Ile Tyr
1               5

<210> 38
<211> 5
<212> PRT
<213> glycine max

<400> 38
Leu Ser Ile Val Asp
1               5

<210> 39
<211> 7
<212> PRT
<213> glycine max

<400> 39
Leu Lys Glu Gln Gln Gln Glu
1               5

<210> 40
<211> 5
<212> PRT
<213> glycine max

<400> 40
Glu Gln Gln Gln Glu
1               5

<210> 41

<211> 6
<212> PRT
<213> glycine max

<400> 41
Glu Glu Gln Pro Leu Glu
1               5

<210> 42
<211> 8
<212> PRT
<213> glycine max

<400> 42
Asn Gln Arg Glu Ser Tyr Phe Val
1               5

<210> 43
<211> 6
<212> PRT
<213> glycine max

<400> 43
Phe Val Asp Ala Gln Pro
1               5

<210> 44
<211> 5
<212> PRT
<213> glycine max

<400> 44
Gly Val Val Phe Leu
1               5

<210> 45
<211> 5
<212> PRT
<213> glycine max

<400> 45
Arg Phe Gln Thr Leu
1               5

<210> 46
<211> 5
<212> PRT
<213> glycine max

<400> 46
Pro Gln Leu Arg Asp
1               5

<210> 47
<211> 5
<212> PRT
<213> glycine max

<400> 47
Gln Pro His Gln Lys
1               5

<210> 48

<211> 5
<212> PRT
<213> glycine max

<400> 48
Asp Arg Asp Ser Tyr
1               5

<210> 49
<211> 5
<212> PRT
<213> glycine max

<400> 49
Leu Arg Val Pro Ala
1               5

<210> 50
<211> 6
<212> PRT
<213> glycine max

<400> 50
Lys Val Glu Glu Glu Glu
1               5

<210> 51
<211> 6
<212> PRT
<213> glycine max

<400> 51
Pro Glu Arg Glu Arg Gln
1               5

<210> 52
<211> 6
<212> PRT
<213> glycine max

<400> 52
Gln Gln His Gly Glu Lys
1               5

<210> 53
<211> 7
<212> PRT
<213> glycine max

<400> 53
Glu Asp Glu Gly Glu Gln Pro
1               5

<210> 54
<211> 8
<212> PRT
<213> glycine max

<400> 54
Glu Gly Glu Gln Pro Arg Pro Phe
1               5

<210> 55

<211> 7
<212> PRT
<213> glycine max

<400> 55
Phe Pro Arg Pro Arg Gln Pro
1               5

<210> 56
<211> 6
<212> PRT
<213> glycine max

<400> 56
Gln Pro His Gln Glu Glu
1               5

<210> 57
<211> 6
<212> PRT
<213> glycine max

<400> 57
Glu Gln Lys Glu Glu His
1               5

<210> 58
<211> 5
<212> PRT
<213> glycine max

<400> 58
Lys Glu Glu His Glu
1               5

<210> 59
<211> 9
<212> PRT
<213> glycine max

<400> 59
Ser Glu Glu Glu Gln Asp Glu Arg Glu
1               5

<210> 60
<211> 6
<212> PRT
<213> glycine max

<400> 60
His Lys Gln Glu Lys His
1               5

<210> 61
<211> 5
<212> PRT
<213> glycine max

<400> 61
Glu Ser Glu Glu Glu
1               5

<210> 62

<211> 7
<212> PRT
<213> glycine max

<400> 62
Glu Glu Glu Asp Gln Asp Glu
1               5

<210> 63
<211> 10
<212> PRT
<213> glycine max

<400> 63
Glu Glu Asp Gln Asp Glu Asp Glu Glu Gln
1               5                   10

<210> 64
<211> 9
<212> PRT
<213> glycine max

<400> 64
Glu Ser Gln Glu Ser Glu Gly Ser Glu
1               5

<210> 65
<211> 6
<212> PRT
<213> glycine max

<400> 65
Glu Ser Gln Arg Glu Pro
1               5

<210> 66
<211> 8
<212> PRT
<213> glycine max

<400> 66
Asn Lys Arg Ser Gln Gln Leu Gln
1               5

<210> 67
<211> 6
<212> PRT
<213> glycine max

<400> 67
Leu Pro His His Ala Asp
1               5

<210> 68
<211> 6
<212> PRT
<213> glycine max

<400> 68
Gly Arg Glu Glu Gly Gln
1               5

<210> 69

<211> 6
<212> PRT
<213> glycine max

<400> 69
Val Leu Val Ile Asn Glu
1               5

<210> 70
<211> 6
<212> PRT
<213> glycine max

<400> 70
Ile Glu Leu Val Gly Ile
1               5

<210> 71
<211> 7
<212> PRT
<213> glycine max

<400> 71
Gln Asp Ile Phe Val Ile Pro
1               5

<210> 72
<211> 6
<212> PRT
<213> glycine max

<400> 72
Asp Ala Gln Pro Gln Gln
1               5

<210> 73
<211> 7
<212> PRT
<213> glycine max

<400> 73
Leu Glu Val Arg Lys Tyr Arg
1               5

<210> 74
<211> 6
<212> PRT
<213> glycine max

<400> 74
Glu Ser Tyr Phe Val Asp
1               5

<210> 75
<211> 6
<212> PRT
<213> glycine max

<400> 75
Phe Val Asp Ala Gln Pro
1               5

<210> 76

<211> 5
<212> PRT
<213> glycine max

<400> 76
Phe Leu Ala Ser Val
1               5

<210> 77
<211> 6
<212> PRT
<213> glycine max

<400> 77
Leu Ser Gly Arg Ala Ile
1               5

<210> 78
<211> 5
<212> PRT
<213> glycine max

<400> 78
His Gln Asn Leu Lys
1               5

<210> 79
<211> 6
<212> PRT
<213> glycine max

<400> 79
Lys Pro Gly Arg Tyr Asp
1               5

<210> 80
<211> 6
<212> PRT
<213> glycine max

<400> 80
Glu Leu Ser Lys Glu Gln
1               5

<210> 81
<211> 6
<212> PRT
<213> glycine max

<400> 81
Ile Glu Arg Gln Val Gln
1               5

<210> 82
<211> 6
<212> PRT
<213> glycine max

<400> 82
Pro Gln Leu Glu Asn Leu
1               5

<210> 83

<211> 6
<212> PRT
<213> glycine max

<400> 83
Asp Ser Tyr Asn Leu His
1               5

<210> 84
<211> 5
<212> PRT
<213> glycine max

<400> 84
Pro Gly Asp Ala Gln
1               5

<210> 85
<211> 6
<212> PRT
<213> glycine max

<400> 85
Ser His Asn Ile Leu Glu
1               5

<210> 86
<211> 8
<212> PRT
<213> glycine max

<400> 86
Ser Phe His Ser Glu Phe Glu Glu
1               5

<210> 87
<211> 6
<212> PRT
<213> glycine max

<400> 87
Glu Glu Ile Asn Arg Val
1               5

<210> 88
<211> 6
<212> PRT
<213> glycine max

<400> 88
Arg Val Leu Phe Gly Glu
1               5

<210> 89
<211> 5
<212> PRT
<213> glycine max

<400> 89
Gln Arg Gln Gln Glu
1               5

<210> 90

<211> 6
<212> PRT
<213> glycine max

<400> 90
Gln Glu Gly Val Ile Val
1               5

<210> 91
<211> 6
<212> PRT
<213> glycine max

<400> 91
Ile Leu Val Ile Asn Glu
1               5

<210> 92
<211> 6
<212> PRT
<213> glycine max

<400> 92
Pro His Phe Asn Ser Lys
1               5

<210> 93
<211> 6
<212> PRT
<213> glycine max

<400> 93
Glu Glu Glu Pro Leu Glu
1               5

<210> 94
<211> 6
<212> PRT
<213> glycine max

<400> 94
Val Phe Ala Ser Gln Val
1               5

<210> 95
<211> 6
<212> PRT
<213> glycine max

<400> 95
Val Val Val Gln Thr Glu
1               5

<210> 96
<211> 6
<212> PRT
<213> glycine max

<400> 96
Thr Glu Gly Arg Val Cys
1               5

<210> 97

<211> 7
<212> PRT
<213> glycine max

<400> 97
Arg Val Cys Glu Ser Gln Ser
1               5

<210> 98
<211> 5
<212> PRT
<213> glycine max

<400> 98
Gln Ser His Gly Phe
1               5

<210> 99
<211> 7
<212> PRT
<213> glycine max

<400> 99
Ser Gln Ser His Gly Phe His
1               5

<210> 100
<211> 9
<212> PRT
<213> glycine max

<400> 100
His Gly Leu Cys Asn Arg Asp His Asn
1               5

<210> 101
<211> 9
<212> PRT
<213> glycine max

<400> 101
Arg Asp His Asn Cys Ala Leu Val Cys
1               5

<210> 102
<211> 6
<212> PRT
<213> glycine max

<400> 102
Leu Val Cys Arg Asn Glu
1               5

<210> 103
<211> 5
<212> PRT
<213> glycine max

<400> 103
Ser Arg Arg Cys Phe
1               5

<210> 104

<211> 6
<212> PRT
<213> glycine max

<400> 104
Gln Glu Glu Asp Glu Glu
1               5

<210> 105
<211> 6
<212> PRT
<213> glycine max

<400> 105
Gln Cys Ala Gly Val Ala
1               5

<210> 106
<211> 6
<212> PRT
<213> glycine max

<400> 106
Pro Arg Gly Ser Gln Ser
1               5

<210> 107
<211> 8
<212> PRT
<213> glycine max

<400> 107
Val Pro His Tyr Asn Leu Asn Ala
1               5

<210> 108
<211> 6
<212> PRT
<213> glycine max

<400> 108
Leu Ile Gln Val Val Asn
1               5

<210> 109
<211> 5
<212> PRT
<213> glycine max

<400> 109
Ala Ala Arg Ser Gln
1               5

<210> 110
<211> 5
<212> PRT
<213> glycine max

<400> 110
Asp Asn Phe Glu Tyr
1               5

<210> 111

<211> 6
<212> PRT
<213> glycine max

<400> 111
Ser Arg Glu Gln Pro Gln
1               5

<210> 112
<211> 6
<212> PRT
<213> glycine max

<400> 112
Cys Cys Phe Ala Phe Ser
1               5

<210> 113
<211> 6
<212> PRT
<213> glycine max

<400> 113
Pro Gln Gln Asn Glu Cys
1               5

<210> 114
<211> 9
<212> PRT
<213> glycine max

<400> 114
Gly Pro Gln Glu Ile Tyr Ile Gln Gln
1               5

<210> 115
<211> 6
<212> PRT
<213> glycine max

<400> 115
Arg Pro Ser Tyr Thr Asn
1               5

<210> 116
<211> 7
<212> PRT
<213> glycine max

<400> 116
Cys Pro Ser Thr Phe Glu Glu
1               5

<210> 117
<211> 7
<212> PRT
<213> glycine max

<400> 117
Ile Tyr Pro Gly Cys Pro Ser
1               5

<210> 118

<211> 7
<212> PRT
<213> glycine max

<400> 118
Pro Gln Gln Pro Gln Gln Arg
1               5

<210> 119
<211> 5
<212> PRT
<213> glycine max

<400> 119
Gln Arg Gly Gln Ser
1               5

<210> 120
<211> 6
<212> PRT
<213> glycine max

<400> 120
Gln Ser Ser Arg Pro Gln
1               5

<210> 121
<211> 6
<212> PRT
<213> glycine max

<400> 121
Pro Gln Asp Arg His Gln
1               5

<210> 122
<211> 6
<212> PRT
<213> glycine max

<400> 122
Gln Lys Ile Tyr Asn Phe
1               5

<210> 123
<211> 5
<212> PRT
<213> glycine max

<400> 123
Arg Glu Gly Asp Leu
1               5

<210> 124
<211> 7
<212> PRT
<213> glycine max

<400> 124
Val Pro Thr Gly Val Ala Trp
1               5

<210> 125

<211> 5
<212> PRT
<213> glycine max

<400> 125
Asn Gln Leu Asp Gln
1               5

<210> 126
<211> 5
<212> PRT
<213> glycine max

<400> 126
Gln Glu Gln Glu Phe
1               5

<210> 127
<211> 6
<212> PRT
<213> glycine max

<400> 127
Lys Tyr Gln Gln Glu Gln
1               5

<210> 128
<211> 5
<212> PRT
<213> glycine max

<400> 128
His Gln Ser Gln Lys
1               5

<210> 129
<211> 8
<212> PRT
<213> glycine max

<400> 129
Lys His Gln Gln Glu Glu Glu Asn
1               5

<210> 130
<211> 6
<212> PRT
<213> glycine max

<400> 130
Lys Asn Leu Gln Gly Glu
1               5

<210> 131
<211> 6
<212> PRT
<213> glycine max

<400> 131
Thr Asp Glu Gln Gln Gln
1               5

<210> 132

<211> 6
<212> PRT
<213> glycine max

<400> 132
Gln Gln Arg Pro Gln Glu
1               5

<210> 133
<211> 6
<212> PRT
<213> glycine max

<400> 133
Glu Lys Pro Gln Cys Lys
1               5

<210> 134
<211> 6
<212> PRT
<213> glycine max

<400> 134
Asp Lys His Cys Gln Arg
1               5

<210> 135
<211> 5
<212> PRT
<213> glycine max

<400> 135
Phe Val Pro His Tyr
1               5

<210> 136
<211> 8
<212> PRT
<213> glycine max

<400> 136
Lys Phe Leu Val Pro Pro Gln Glu
1               5

<210> 137
<211> 6
<212> PRT
<213> glycine max

<400> 137
Pro Gln Glu Ser Gln Lys
1               5

<210> 138
<211> 6
<212> PRT
<213> glycine max

<400> 138
Cys Pro Ser Thr Tyr Asn
1               5

<210> 139

<211> 6
<212> PRT
<213> glycine max

<400> 139
Gln Gly Gly Ser Gln Ser
1               5

<210> 140
<211> 5
<212> PRT
<213> glycine max

<400> 140
Ser Gly Ala Ile Val
1               5

<210> 141
<211> 6
<212> PRT
<213> glycine max

<400> 141
Gly Gly Leu Arg Val Thr
1               5

<210> 142
<211> 5
<212> PRT
<213> glycine max

<400> 142
Arg Leu Arg Gln Asn
1               5

<210> 143
<211> 6
<212> PRT
<213> glycine max

<400> 143
Leu Lys Leu Ser Ala Gln
1               5

<210> 144
<211> 6
<212> PRT
<213> glycine max

<400> 144
Val Ala Ala Lys Ser Gln
1               5

<210> 145
<211> 6
<212> PRT
<213> glycine max

<400> 145
Pro Ser Ile Gly Asn Leu
1               5

<210> 146

<211> 7
<212> PRT
<213> glycine max

<400> 146
Phe Ser Phe Leu Val Pro Pro
1               5

<210> 147
<211> 7
<212> PRT
<213> glycine max

<400> 147
Arg Glu Gln Ala Gln Gln Asn
1               5

<210> 148
<211> 5
<212> PRT
<213> glycine max

<400> 148
Gln Lys Leu Asn Ala
1               5

<210> 149
<211> 6
<212> PRT
<213> glycine max

<400> 149
Arg Ile Glu Ser Glu Gly
1               5

<210> 150
<211> 6
<212> PRT
<213> glycine max

<400> 150
Thr Tyr Gln Glu Pro Gln
1               5

<210> 151
<211> 5
<212> PRT
<213> glycine max

<400> 151
Pro Gln Glu Ser Gln
1               5

<210> 152
<211> 6
<212> PRT
<213> glycine max

<400> 152
Glu Ser Gln Gln Arg Gly
1               5

<210> 153

<211> 5
<212> PRT
<213> glycine max

<400> 153
Gly Arg Ser Gln Arg
1               5

<210> 154
<211> 6
<212> PRT
<213> glycine max

<400> 154
Gln Asp Arg His Gln Lys
1               5

<210> 155
<211> 6
<212> PRT
<213> glycine max

<400> 155
Val His Arg Phe Arg Glu
1               5

<210> 156
<211> 6
<212> PRT
<213> glycine max

<400> 156
Glu Glu Glu Asn Glu Gly
1               5

<210> 157
<211> 6
<212> PRT
<213> glycine max

<400> 157
Gln Ile Val Arg Asn Leu
1               5

<210> 158
<211> 6
<212> PRT
<213> glycine max

<400> 158
Arg Lys Pro Gln Gln Glu
1               5

<210> 159
<211> 6
<212> PRT
<213> glycine max

<400> 159
Glu Glu Glu Gln Pro Gln
1               5

<210> 160

<211> 6
<212> PRT
<213> glycine max

<400> 160
Asp Ile Tyr Asn Pro Gln
1               5

<210> 161
<211> 5
<212> PRT
<213> glycine max

<400> 161
Glu Arg Val Phe Asp
1               5

<210> 162
<211> 5
<212> PRT
<213> glycine max

<400> 162
Glu Leu Gln Glu Gly
1               5

<210> 163
<211> 8
<212> PRT
<213> glycine max

<400> 163
Phe Arg Glu Gln Pro Gln Gln Asn
1               5

<210> 164
<211> 6
<212> PRT
<213> glycine max

<400> 164
Gln Pro Gln Lys Gln Gln
1               5

<210> 165
<211> 6
<212> PRT
<213> glycine max

<400> 165
Arg Gln Ile Val Arg Lys
1               5

<210> 166
<211> 6
<212> PRT
<213> glycine max

<400> 166
Arg Arg Gln Gln Ala Arg
1               5

<210> 167

<211> 6
<212> PRT
<213> glycine max

<400> 167
Glu Cys Gln Ile Gln Arg
1               5

<210> 168
<211> 7
<212> PRT
<213> glycine max

<400> 168
Ile Gln Gln Gly Ser Gly Ile
1               5

<210> 169
<211> 6
<212> PRT
<213> glycine max

<400> 169
Gln Gln Lys Gly Gln Ser
1               5

<210> 170
<211> 6
<212> PRT
<213> glycine max

<400> 170
Ile Tyr His Phe Arg Glu
1               5

<210> 171
<211> 6
<212> PRT
<213> glycine max

<400> 171
Ala Val Pro Thr Gly Phe
1               5

<210> 172
<211> 5
<212> PRT
<213> glycine max

<400> 172
Asn Ser Phe Gln Asn
1               5

<210> 173
<211> 5
<212> PRT
<213> glycine max

<400> 173
Pro Thr Glu Glu Gln
1               5

<210> 174

<211> 6
<212> PRT
<213> glycine max

<400> 174
Gln Gln Gln Arg Pro Glu
1               5

<210> 175
<211> 6
<212> PRT
<213> glycine max

<400> 175
Pro Asp Cys Asp Glu Lys
1               5

<210> 176
<211> 6
<212> PRT
<213> glycine max

<400> 176
Asp Lys His Cys Gln Ser
1               5

<210> 177
<211> 6
<212> PRT
<213> glycine max

<400> 177
Cys Gln Ser Gln Ser Arg
1               5

<210> 178
<211> 6
<212> PRT
<213> glycine max

<400> 178
Gln Ser Gln Ser Arg Asn
1               5

<210> 179
<211> 6
<212> PRT
<213> glycine max

<400> 179
His Asn Ile Gly Gln Thr
1               5

<210> 180
<211> 5
<212> PRT
<213> glycine max

<400> 180
Gln Gly Lys Gly Ala
1               5

<210> 181

<211> 6
<212> PRT
<213> glycine max

<400> 181
Val Leu Ser Gly Phe Ser
1               5

<210> 182
<211> 6
<212> PRT
<213> glycine max

<400> 182
Leu Asn Glu Cys Gln Leu
1               5

<210> 183
<211> 6
<212> PRT
<213> glycine max

<400> 183
Pro Asp His Arg Val Glu
1               5

<210> 184
<211> 6
<212> PRT
<213> glycine max

<400> 184
Arg Asn Gly Leu His Ser
1               5

<210> 185
<211> 6
<212> PRT
<213> glycine max

<400> 185
Tyr Ser Pro Tyr Pro Arg
1               5

<210> 186
<211> 6
<212> PRT
<213> glycine max

<400> 186
Val Ala Ile Pro Gly Cys
1               5

<210> 187
<211> 6
<212> PRT
<213> glycine max

<400> 187
Glu Thr Phe Glu Glu Pro
1               5

<210> 188

<211> 6
<212> PRT
<213> glycine max

<400> 188
Gln Gln Leu Gln Asp Ser
1               5

<210> 189
<211> 6
<212> PRT
<213> glycine max

<400> 189
Ile Arg His Phe Asn Glu
1               5

<210> 190
<211> 7
<212> PRT
<213> glycine max

<400> 190
Asp Ile Glu Tyr Pro Glu Thr
1               5

<210> 191
<211> 6
<212> PRT
<213> glycine max

<400> 191
Lys Gln Gly Gln His Gln
1               5

<210> 192
<211> 6
<212> PRT
<213> glycine max

<400> 192
Asp Ile Ala Glu Lys Leu
1               5

<210> 193
<211> 6
<212> PRT
<213> glycine max

<400> 193
Lys Leu Glu Ser Pro Asp
1               5

<210> 194
<211> 6
<212> PRT
<213> glycine max

<400> 194
Ser Val Ile Ser Pro Lys
1               5

<210> 195

<211> 5
<212> PRT
<213> glycine max

<400> 195
Gln Gln Asp Glu Asp
1               5

<210> 196
<211> 5
<212> PRT
<213> glycine max

<400> 196
Lys Arg Glu Gln Asp
1               5

<210> 197
<211> 6
<212> PRT
<213> glycine max

<400> 197
His Pro Pro Arg Arg Pro
1               5

<210> 198
<211> 6
<212> PRT
<213> glycine max

<400> 198
Arg Glu Gln Asp Glu Asp
1               5

<210> 199
<211> 6
<212> PRT
<213> glycine max

<400> 199
Gly Gln Asp Glu Asp Glu
1               5

<210> 200
<211> 6
<212> PRT
<213> glycine max

<400> 200
Thr Gln Pro Arg Arg Pro
1               5

<210> 201
<211> 6
<212> PRT
<213> glycine max

<400> 201
Pro Arg Gln Glu Glu Pro
1               5

<210> 202

<211> 6
<212> PRT
<213> glycine max

<400> 202
Glu Glu Pro Arg Glu Arg
1               5

<210> 203
<211> 6
<212> PRT
<213> glycine max

<400> 203
Asn Ser Gln His Pro Glu
1               5

<210> 204
<211> 6
<212> PRT
<213> glycine max

<400> 204
His Pro Glu Leu Gln Cys
1               5

<210> 205
<211> 6
<212> PRT
<213> glycine max

<400> 205
Gln Cys Ala Gly Val Thr
1               5

<210> 206
<211> 5
<212> PRT
<213> glycine max

<400> 206
Thr Val Ser Lys Arg
1               5

<210> 207
<211> 6
<212> PRT
<213> glycine max

<400> 207
Lys Gly Ala Ile Gly Phe
1               5

<210> 208
<211> 5
<212> PRT
<213> glycine max

<400> 208
Gly Ser Arg Ser Gln
1               5

<210> 209

<211> 5
<212> PRT
<213> glycine max

<400> 209
Val Thr Val Glu Gly
1               5

<210> 210
<211> 7
<212> PRT
<213> glycine max

<400> 210
Arg Thr Pro Ser Tyr Pro Pro
1               5

<210> 211
<211> 7
<212> PRT
<213> glycine max

<400> 211
Val Glu Glu Asn Ile Cys Thr
1               5

<210> 212
<211> 6
<212> PRT
<213> glycine max

<400> 212
Pro Ser Arg Ala Asp Phe
1               5

<210> 213
<211> 5
<212> PRT
<213> glycine max

<400> 213
Asn Cys Gln Gly Asn
1               5

<210> 214
<211> 6
<212> PRT
<213> glycine max

<400> 214
Arg Arg Gly Gln Leu Leu
1               5

<210> 215
<211> 5
<212> PRT
<213> glycine max

<400> 215
Pro Ala Val Ala Glu
1               5

<210> 216

<211> 6
<212> PRT
<213> glycine max

<400> 216
Ile Pro Ser Glu Val Leu
1               5

<210> 217
<211> 5
<212> PRT
<213> glycine max

<400> 217
Asn Ser Tyr Asn Leu
1               5

<210> 218
<211> 5
<212> PRT
<213> glycine max

<400> 218
Cys Gln Leu Asn Asn
1               5

<210> 219
<211> 6
<212> PRT
<213> glycine max

<400> 219
Arg Val Glu Ser Glu Gly
1               5

<210> 220
<211> 6
<212> PRT
<213> glycine max

<400> 220
Tyr Leu Pro Tyr Pro Gln
1               5

<210> 221
<211> 6
<212> PRT
<213> glycine max

<400> 221
Ser His Leu Pro Ser Tyr
1               5

<210> 222
<211> 6
<212> PRT
<213> glycine max

<400> 222
Ile Gly Phe Ala Phe Pro
1               5

<210> 223

<211> 6
<212> PRT
<213> glycine max

<400> 223
Phe Pro Gly Cys Pro Glu
1               5

<210> 224
<211> 6
<212> PRT
<213> glycine max

<400> 224
Val Ile Pro Leu Gly Val
1               5

<210> 225
<211> 6
<212> PRT
<213> glycine max

<400> 225
Asp Glu Pro Val Val Ala
1               5

<210> 226
<211> 5
<212> PRT
<213> glycine max

<400> 226
Arg Val Phe Tyr Leu
1               5

<210> 227
<211> 6
<212> PRT
<213> glycine max

<400> 227
Gly Asn Pro Asp Ile Glu
1               5

<210> 228
<211> 6
<212> PRT
<213> glycine max

<400> 228
Ile Glu His Pro Glu Thr
1               5

<210> 229
<211> 5
<212> PRT
<213> glycine max

<400> 229
Gln Gly Gln His Arg
1               5

<210> 230

<211> 5
<212> PRT
<213> glycine max

<400> 230
His Arg Gln Gln Glu
1               5

<210> 231
<211> 6
<212> PRT
<213> glycine max

<400> 231
Gln Gln Glu Glu Glu Gly
1               5

<210> 232
<211> 5
<212> PRT
<213> glycine max

<400> 232
Asp Thr Ala Glu Lys
1               5

<210> 233
<211> 5
<212> PRT
<213> glycine max

<400> 233
Glu Gly Gly Leu Ser
1               5

<210> 234
<211> 6
<212> PRT
<213> glycine max

<400> 234
Lys Trp Gln Glu Gln Glu
1               5

<210> 235
<211> 6
<212> PRT
<213> glycine max

<400> 235
Gln Glu Gln Glu Asp Glu
1               5

<210> 236
<211> 6
<212> PRT
<213> glycine max

<400> 236
Asp Glu Glu Tyr Gly Arg
1               5

<210> 237

<211> 6
<212> PRT
<213> glycine max

<400> 237
Gly Lys His Glu Asp Asp
1               5

<210> 238
<211> 6
<212> PRT
<213> glycine max

<400> 238
Glu Glu Glu Asp Gln Pro
1               5

<210> 239
<211> 6
<212> PRT
<213> glycine max

<400> 239
Pro Arg Pro Asp His Pro
1               5

<210> 240
<211> 6
<212> PRT
<213> glycine max

<400> 240
His Pro Pro Gln Arg Pro
1               5

<210> 241
<211> 6
<212> PRT
<213> glycine max

<400> 241
Gln Arg Pro Ser Arg Pro
1               5

<210> 242
<211> 6
<212> PRT
<213> glycine max

<400> 242
Pro Glu Gln Gln Glu Pro
1               5

<210> 243
<211> 6
<212> PRT
<213> glycine max

<400> 243
Gln Gln Glu Pro Arg Gly
1               5

<210> 244

<211> 5
<212> PRT
<213> glycine max

<400> 244
Leu Arg Arg Gly Gln
1               5

<210> 245
<211> 6
<212> PRT
<213> glycine max

<400> 245
Gly Asn Ala Val Phe Asp
1               5

<210> 246
<211> 6
<212> PRT
<213> glycine max

<400> 246
Thr His His Asn Ala Val
1               5

<210> 247
<211> 6
<212> PRT
<213> glycine max

<400> 247
Ser Asn Ser Tyr Asn Leu
1               5

<210> 248
<211> 6
<212> PRT
<213> glycine max

<400> 248
Leu Gly Gln Ser Gln Val
1               5

<210> 249
<211> 5
<212> PRT
<213> glycine max

<400> 249
Pro Leu Val Asn Pro
1               5

<210> 250
<211> 5
<212> PRT
<213> glycine max

<400> 250
Gln Gly Asn Ser Gly
1               5

<210> 251

<211> 5
<212> PRT
<213> glycine max

<400> 251
Leu Gly Gly Ser Leu
1               5

<210> 252
<211> 6
<212> PRT
<213> glycine max

<400> 252
Asp Asp Cys Cys Ala Leu
1               5

<210> 253
<211> 6
<212> PRT
<213> glycine max

<400> 253
Asn Ser Cys Gly Arg Ser
1               5

<210> 254
<211> 5
<212> PRT
<213> glycine max

<400> 254
Gly Asn Glu Arg Cys
1               5

<210> 255
<211> 6
<212> PRT
<213> glycine max

<400> 255
Arg Ala Ala Pro Thr Gly
1               5

<210> 256
<211> 6
<212> PRT
<213> glycine max

<400> 256
Leu Cys Val Gly Ile Pro
1               5

<210> 257
<211> 5
<212> PRT
<213> glycine max

<400> 257
Thr Glu Trp Ser Val
1               5

<210> 258

<211> 6
<212> PRT
<213> glycine max

<400> 258
Val Ile Thr Gln Val Lys
1               5

<210> 259
<211> 6
<212> PRT
<213> glycine max

<400> 259
Lys Tyr Gln Gly Gly Cys
1               5

<210> 260
<211> 5
<212> PRT
<213> glycine max

<400> 260
Lys Pro Glu Glu Ile
1               5

<210> 261
<211> 6
<212> PRT
<213> glycine max

<400> 261
Phe Pro Gln Phe Lys Pro
1               5

<210> 262
<211> 5
<212> PRT
<213> glycine max

<400> 262
Glu Pro Gly Ser Leu
1               5

<210> 263
<211> 7
<212> PRT
<213> glycine max

<400> 263
Gln Gly Glu Pro Gly Ala Val
1               5

<210> 264
<211> 6
<212> PRT
<213> glycine max

<400> 264
Glu Arg Leu Gly Asp Tyr
1               5

<210> 265

<211> 6
<212> PRT
<213> glycine max

<400> 265
Ala Tyr Val Asp Asp His
1               5

<210> 266
<211> 8
<212> PRT
<213> glycine max

<400> 266
Asn His Leu Thr His Ala Ala Ile
1               5

<210> 267
<211> 6
<212> PRT
<213> glycine max

<400> 267
Ile Ile Gly Gln Asp Gly
1               5

<210> 268
<211> 6
<212> PRT
<213> glycine max

<400> 268
Gly Ser Val Trp Leu Gln
1               5

<210> 269
<211> 5
<212> PRT
<213> glycine max

<400> 269
Thr Pro Gly Gln Cys
1               5

<210> 270
<211> 5
<212> PRT
<213> glycine max

<400> 270
Ile Tyr Asp Glu Pro
1               5

<210> 271
<211> 6
<212> PRT
<213> glycine max

<400> 271
Cys Asp Ile Glu Gly Asn
1               5

<210> 272

<211> 6
<212> PRT
<213> glycine max

<400> 272
Gly Ser Val Trp Ala Gln
1               5

<210> 273
<211> 5
<212> PRT
<213> glycine max

<400> 273
Val Val Glu Arg Pro
1               5

<210> 274
<211> 5
<212> PRT
<213> glycine max

<400> 274
Ile Asp Gln Gly Tyr
1               5

<210> 275
<211> 5
<212> PRT
<213> glycine max

<400> 275
Ser Ile Asp Glu Ala
1               5

<210> 276
<211> 6
<212> PRT
<213> glycine max

<400> 276
Asn Leu Gly Tyr Ser Tyr
1               5

<210> 277
<211> 7
<212> PRT
<213> glycine max

<400> 277
Val Glu Asn Val Glu Gly Asn
1               5

<210> 278
<211> 6
<212> PRT
<213> glycine max

<400> 278
Glu Asp Glu Ile Asn Ser
1               5

<210> 279

<211> 6
<212> PRT
<213> glycine max

<400> 279
Asn Ser Pro Val Ala Pro
1               5

<210> 280
<211> 6
<212> PRT
<213> glycine max

<400> 280
Pro Asp Thr Ala Glu Lys
1               5

<210> 281
<211> 5
<212> PRT
<213> glycine max

<400> 281
Glu Lys Ile Thr Phe
1               5

<210> 282
<211> 7
<212> PRT
<213> glycine max

<400> 282
Gly Ser Ala Gly Lys Leu Thr
1               5

<210> 283
<211> 5
<212> PRT
<213> glycine max

<400> 283
Glu Thr Lys Gly Asp
1               5

<210> 284
<211> 5
<212> PRT
<213> glycine max

<400> 284
Ile Gly Ala Ser Ala
1               5

<210> 285
<211> 9
<212> PRT
<213> glycine max

<400> 285
Ala Thr Val Gln Glu Lys Ala Glu Arg
1               5

<210> 286

<211> 6
<212> PRT
<213> glycine max

<400> 286
Ala Arg Asp Pro Val Gln
1               5

<210> 287
<211> 6
<212> PRT
<213> glycine max

<400> 287
Glu Leu Ala Thr Gln Lys
1               5

<210> 288
<211> 5
<212> PRT
<213> glycine max

<400> 288
Gln His Asn Thr Ala
1               5

<210> 289
<211> 6
<212> PRT
<213> glycine max

<400> 289
Gly His Gly His His Thr
1               5

<210> 290
<211> 6
<212> PRT
<213> glycine max

<400> 290
Gly Glu Tyr Gly Gln Pro
1               5

<210> 291
<211> 5
<212> PRT
<213> glycine max

<400> 291
His Gln Thr Ser Ala
1               5

<210> 292
<211> 9
<212> PRT
<213> glycine max

<400> 292
His Gly Thr Gly Gln Pro Thr Gly His
1               5

<210> 293

<211> 6
<212> PRT
<213> glycine max

<400> 293
His Val Thr Glu Gly Val
1               5

<210> 294
<211> 7
<212> PRT
<213> glycine max

<400> 294
Val Gly Ser His Pro Ile Gly
1               5

<210> 295
<211> 9
<212> PRT
<213> glycine max

<400> 295
Leu Phe Cys Ile Ala His Thr Cys Ser
1               5

<210> 296
<211> 7
<212> PRT
<213> glycine max

<400> 296
Ser Ala Ser Lys Trp Gln His
1               5

<210> 297
<211> 6
<212> PRT
<213> glycine max

<400> 297
Gln Gln Asp Ser Cys Arg
1               5

<210> 298
<211> 6
<212> PRT
<213> glycine max

<400> 298
Ser Cys Arg Lys Gln Leu
1               5

<210> 299
<211> 7
<212> PRT
<213> glycine max

<400> 299
Lys Gln Leu Gln Gly Val Asn
1               5

<210> 300

<211> 7
<212> PRT
<213> glycine max

<400> 300
Asn Leu Thr Pro Cys Glu Lys
1               5

<210> 301
<211> 5
<212> PRT
<213> glycine max

<400> 301
Gln Gly Arg Gly Asp
1               5

<210> 302
<211> 7
<212> PRT
<213> glycine max

<400> 302
Glu Asp Glu Glu Glu Glu Gly
1               5

<210> 303
<211> 5
<212> PRT
<213> glycine max

<400> 303
Gln Lys Cys Cys Thr
1               5

<210> 304
<211> 6
<212> PRT
<213> glycine max

<400> 304
Thr Glu Met Ser Glu Leu
1               5

<210> 305
<211> 6
<212> PRT
<213> glycine max

<400> 305
Cys Lys Ala Leu Gln Lys
1               5

<210> 306
<211> 9
<212> PRT
<213> glycine max

<400> 306
Asn Gln Ser Glu Glu Leu Glu Glu Lys
1               5

<210> 307

<211> 6
<212> PRT
<213> glycine max

<400> 307
Met Cys Arg Phe Gly Pro
1               5

<210> 308
<211> 6
<212> PRT
<213> glycine max

<400> 308
Ile Gln Cys Asp Leu Ser
1               5

<210> 309
<211> 8
<212> PRT
<213> glycine max

<400> 309
Cys Ala Ser Lys Trp Gln Gln His
1               5

<210> 310
<211> 6
<212> PRT
<213> glycine max

<400> 310
Gln His Gln Gln Glu Ser
1               5

<210> 311
<211> 7
<212> PRT
<213> glycine max

<400> 311
Glu Ser Cys Arg Glu Gln Leu
1               5

<210> 312
<211> 6
<212> PRT
<213> glycine max

<400> 312
Asn Pro Cys Glu His Ile
1               5

<210> 313
<211> 6
<212> PRT
<213> glycine max

<400> 313
Glu Lys Ile Gln Ala Gly
1               5

<210> 314

<211> 7
<212> PRT
<213> glycine max

<400> 314
Asp Gly Ser Asp Glu Asp His
1               5

<210> 315
<211> 5
<212> PRT
<213> glycine max

<400> 315
Glu Gly Lys Glu Glu
1               5

<210> 316
<211> 6
<212> PRT
<213> glycine max

<400> 316
Lys Glu Glu Glu Glu Glu
1               5

<210> 317
<211> 5
<212> PRT
<213> glycine max

<400> 317
Ser Glu Met Ser Glu
1               5

<210> 318
<211> 7
<212> PRT
<213> glycine max

<400> 318
Ser Pro Ile Cys Gln Cys Lys
1               5

<210> 319
<211> 10
<212> PRT
<213> glycine max

<400> 319
Asn Gln Ser Glu Gln Leu Glu Gly Lys Glu
1               5                   10

<210> 320
<211> 6
<212> PRT
<213> glycine max

<400> 320
Ala Ile Arg Cys Arg Leu
1               5

<210> 321

<211> 5
<212> PRT
<213> glycine max

<400> 321
Ile Gly Cys Asp Leu
1               5

<210> 322
<211> 8
<212> PRT
<213> glycine max

<400> 322
Asn Gly Ala Cys Ser Pro Phe Glu
1               5

<210> 323
<211> 7
<212> PRT
<213> glycine max

<400> 323
Pro Pro Cys Arg Ser Arg Asp
1               5

<210> 324
<211> 5
<212> PRT
<213> glycine max

<400> 324
Arg Asp Cys Arg Cys
1               5

<210> 325
<211> 7
<212> PRT
<213> glycine max

<400> 325
Arg Cys Val Pro Ile Gly Leu
1               5

<210> 326
<211> 6
<212> PRT
<213> glycine max

<400> 326
Ala Gly Phe Cys Ile His
1               5

<210> 327
<211> 6
<212> PRT
<213> glycine max

<400> 327
Gly Leu Ser Ser Val Ala
1               5

<210> 328

<211> 8
<212> PRT
<213> glycine max

<400> 328
His Pro Asn Leu Cys Gln Ser Asp
1               5

<210> 329
<211> 6
<212> PRT
<213> glycine max

<400> 329
Gln Ser Asp Asp Glu Cys
1               5

<210> 330
<211> 8
<212> PRT
<213> glycine max

<400> 330
Gly Asn Phe Cys Ala Arg Tyr Pro
1               5

<210> 331
<211> 6
<212> PRT
<213> glycine max

<400> 331
Pro Pro Cys Arg Ser Ser
1               5

<210> 332
<211> 5
<212> PRT
<213> glycine max

<400> 332
Val Asp Glu His Pro
1               5

<210> 333
<211> 5
<212> PRT
<213> glycine max

<400> 333
Ser Asp Ser Glu Ala
1               5

<210> 334
<211> 9
<212> PRT
<213> glycine max

<400> 334
Glu Gly Thr Ser Ser Ala Lys Leu Thr
1               5

<210> 335

<211> 6
<212> PRT
<213> glycine max

<400> 335
Arg Cys Val Pro Ile Ala
1               5

<210> 336
<211> 6
<212> PRT
<213> glycine max

<400> 336
Leu Ala Pro Ile Asp Thr
1               5

<210> 337
<211> 5
<212> PRT
<213> glycine max

<400> 337
Thr Lys Pro Gln Thr
1               5

<210> 338
<211> 6
<212> PRT
<213> glycine max

<400> 338
Lys Pro Gln Thr His Ala
1               5

<210> 339
<211> 7
<212> PRT
<213> glycine max

<400> 339
Asp Pro Pro Asn Pro His Ile
1               5

<210> 340
<211> 5
<212> PRT
<213> glycine max

<400> 340
Asp Thr Phe Arg Asn
1               5

<210> 341
<211> 6
<212> PRT
<213> glycine max

<400> 341
Asn Leu Pro His Ala Ser
1               5

<210> 342

<211> 6
<212> PRT
<213> glycine max

<400> 342
Glu Ile His Val Glu Lys
1               5

<210> 343
<211> 6
<212> PRT
<213> glycine max

<400> 343
Lys His Arg Val Pro Lys
1               5

<210> 344
<211> 6
<212> PRT
<213> glycine max

<400> 344
Asp His Ala Gly Lys Ala
1               5

<210> 345
<211> 6
<212> PRT
<213> glycine max

<400> 345
Lys Glu Ser Gln Arg Glu
1               5

<210> 346
<211> 5
<212> PRT
<213> glycine max

<400> 346
Ala Asn Val Val Gly
1               5

<210> 347
<211> 6
<212> PRT
<213> glycine max

<400> 347
Val Ala Glu Lys Gly Arg
1               5

<210> 348
<211> 5
<212> PRT
<213> glycine max

<400> 348
Gly Arg Glu Thr Glu
1               5

<210> 349

<211> 6
<212> PRT
<213> glycine max

<400> 349
Ala His Val Val Glu Gly
1               5

<210> 350
<211> 5
<212> PRT
<213> glycine max

<400> 350
Glu Tyr Thr Ala Lys
1               5

<210> 351
<211> 7
<212> PRT
<213> glycine max

<400> 351
Glu Ala Gln Arg Glu Leu Glu
1               5

<210> 352
<211> 6
<212> PRT
<213> glycine max

<400> 352
Gln Pro Gln Glu Ala Glu
1               5

<210> 353
<211> 5
<212> PRT
<213> glycine max

<400> 353
Gly Glu Ser Glu Gly
1               5

<210> 354
<211> 7
<212> PRT
<213> glycine max

<400> 354
Arg Ala Lys His Glu Glu Gly
1               5

<210> 355
<211> 15
<212> PRT
<213> glycine max

<400> 355
Val Phe Ala Ser Gln Val Val Val Gln Thr Glu Gly Arg Val Cys
1               5                   10                  15

<210> 356

<211> 15
<212> PRT
<213> glycine max

<400> 356
Val Phe Ala Ser Gln Val Val Val Gln Thr Glu Gly Arg Val Cys
1               5                   10                  15

<210> 357
<211> 15
<212> PRT
<213> glycine max

<400> 357
Val Phe Ala Ser Gln Val Val Val Gln Thr Glu Gly Arg Val Cys
1               5                   10                  15

<210> 358
<211> 15
<212> PRT
<213> glycine max

<400> 358
Gln Val Val Val Gln Thr Glu Gly Arg Val Cys Glu Ser Gln Ser
1               5                   10                  15

<210> 359
<211> 15
<212> PRT
<213> glycine max

<400> 359
Gln Thr Glu Gly Arg Val Cys Glu Ser Gln Ser His Gly Phe His
1               5                   10                  15

<210> 360
<211> 16
<212> PRT
<213> glycine max

<400> 360
Arg Val Cys Glu Ser Gln Ser His Gly Phe His Gly Leu Cys Asn Arg
1               5                   10                  15

<210> 361
<211> 15
<212> PRT
<213> glycine max

<400> 361
Ser Gln Ser His Gly Phe His Gly Leu Cys Asn Arg Asp His Asn
1               5                   10                  15

<210> 362
<211> 16
<212> PRT
<213> glycine max

<400> 362
Gly Phe His Gly Leu Cys Asn Arg Asp His Asn Cys Ala Leu Val Cys
1               5                   10                  15

<210> 363
<211> 15
<212> PRT
<213> glycine max

<400> 363
Leu Cys Asn Arg Asp His Asn Cys Ala Leu Val Cys Arg Asn Glu
1               5                   10                  15

<210> 364
<211> 15
<212> PRT
<213> glycine max

<400> 364
Arg Cys Lys Arg Ser Arg Arg Cys Phe Cys Thr Arg Ile Cys Gly
1               5                   10                  15

<210> 365
<211> 15
<212> PRT
<213> glycine max

<400> 365
Pro Lys His Asn Lys Cys Leu Gln Ser Cys Asn Ser Glu Arg Asp
1               5                   10                  15

<210> 366
<211> 15
<212> PRT
<213> glycine max

<400> 366
Pro Lys His Asn Lys Cys Leu Gln Ser Cys Asn Ser Glu Arg Asp
1               5                   10                  15

<210> 367
<211> 15
<212> PRT
<213> glycine max

<400> 367
Ser Cys Asn Ser Glu Arg Asp Ser Tyr Arg Asn Gln Ala Cys His
1               5                   10                  15

<210> 368
<211> 16
<212> PRT
<213> glycine max

<400> 368
Ser Cys Asn Ser Glu Arg Asp Ser Tyr Arg Asn Gln Ala Cys His Ala
1               5                   10                  15

<210> 369
<211> 15
<212> PRT
<213> glycine max

<400> 369
Glu Arg Asp Ser Tyr Arg Asn Gln Ala Cys His Ala Arg Cys Asn
1               5                   10                  15

<210> 370
<211> 15
<212> PRT
<213> glycine max

<400> 370
Leu Lys Val Glu Lys Glu Glu Cys Glu Glu Gly Glu Ile Pro Arg
1               5                   10                  15

<210> 371
<211> 15
<212> PRT
<213> glycine max

<400> 371
Lys Glu Glu Cys Glu Glu Gly Glu Ile Pro Arg Pro Arg Pro Arg
1               5                   10                  15

<210> 372
<211> 15
<212> PRT
<213> glycine max

<400> 372
Lys Glu Glu Cys Glu Glu Gly Glu Ile Pro Arg Pro Arg Pro Arg
1               5                   10                  15

<210> 373
<211> 16
<212> PRT
<213> glycine max

<400> 373
Ile Pro Arg Pro Arg Pro Arg Pro Gln His Pro Glu Arg Glu Pro Gln
1               5                   10                  15

<210> 374
<211> 16
<212> PRT
<213> glycine max

<400> 374
Arg Pro Arg Pro Gln His Pro Glu Arg Glu Pro Gln Gln Pro Gly Glu
1               5                   10                  15

<210> 375
<211> 15
<212> PRT
<213> glycine max

<400> 375
Gln His Pro Glu Arg Glu Pro Gln Gln Pro Gly Glu Lys Glu Glu
1               5                   10                  15

<210> 376
<211> 15
<212> PRT
<213> glycine max

<400> 376
Gln His Pro Glu Arg Glu Pro Gln Gln Pro Gly Glu Lys Glu Glu
1               5                   10                  15

<210> 377
<211> 15
<212> PRT
<213> glycine max

<400> 377
Gln Pro Gly Glu Lys Glu Glu Asp Glu Asp Glu Gln Pro Arg Pro
1               5                   10                  15

<210> 378
<211> 15
<212> PRT
<213> glycine max

<400> 378
Glu Asp Glu Gln Pro Arg Pro Ile Pro Phe Pro Arg Pro Gln Pro
1               5                   10                  15

<210> 379
<211> 16
<212> PRT
<213> glycine max

<400> 379
Pro Arg Pro Ile Pro Phe Pro Arg Pro Gln Pro Arg Gln Glu Glu Glu
1               5                   10                  15

<210> 380
<211> 15
<212> PRT
<213> glycine max

<400> 380
Pro Gln Pro Arg Gln Glu Glu Glu His Glu Gln Arg Glu Glu Gln
1               5                   10                  15

<210> 381
<211> 15
<212> PRT
<213> glycine max

<400> 381
Trp Pro Arg Lys Glu Glu Lys Arg Gly Glu Lys Gly Ser Glu Glu
1               5                   10                  15

<210> 382
<211> 15
<212> PRT
<213> glycine max

<400> 382
Arg Pro Pro His Gln Lys Glu Glu Arg Asn Glu Glu Glu Asp Glu

1                   5                   10                  15

<210> 383
<211> 16
<212> PRT
<213> glycine max

<400> 383
Gln Lys Glu Glu Arg Asn Glu Glu Glu Asp Glu Asp Glu Glu Gln Gln
1                   5                   10                  15

<210> 384
<211> 15
<212> PRT
<213> glycine max

<400> 384
Arg Asn Glu Glu Glu Asp Glu Asp Glu Glu Gln Gln Arg Glu Ser
1                   5                   10                  15

<210> 385
<211> 15
<212> PRT
<213> glycine max

<400> 385
Glu Asp Glu Asp Glu Glu Gln Gln Arg Glu Ser Glu Glu Ser Glu
1                   5                   10                  15

<210> 386
<211> 15
<212> PRT
<213> glycine max

<400> 386
Lys Phe Glu Glu Ile Asn Lys Val Leu Phe Ser Arg Glu Glu Gly
1                   5                   10                  15

<210> 387
<211> 15
<212> PRT
<213> glycine max

<400> 387
Ile Asn Lys Val Leu Phe Ser Arg Glu Glu Gly Gln Gln Gln Gly
1                   5                   10                  15

<210> 388
<211> 15
<212> PRT
<213> glycine max

<400> 388
Ser Glu Asp Lys Pro Phe Asn Leu Arg Ser Arg Asp Pro Ile Tyr
1                   5                   10                  15

<210> 389
<211> 16
<212> PRT
<213> glycine max

<400> 389
Leu Ser Ile Val Asp Met Asn Glu Gly Ala Leu Leu Leu Pro His Phe
1               5                   10                  15

<210> 390
<211> 15
<212> PRT
<213> glycine max

<400> 390
Glu Leu Val Gly Leu Lys Glu Gln Gln Gln Glu Gln Gln Gln Glu
1               5                   10                  15

<210> 391
<211> 15
<212> PRT
<213> glycine max

<400> 391
Glu Leu Val Gly Leu Lys Glu Gln Gln Gln Glu Gln Gln Gln Glu
1               5                   10                  15

<210> 392
<211> 16
<212> PRT
<213> glycine max

<400> 392
Leu Lys Glu Gln Gln Gln Glu Gln Gln Gln Glu Glu Gln Pro Leu Glu
1               5                   10                  15

<210> 393
<211> 15
<212> PRT
<213> glycine max

<400> 393
Leu Leu Lys Asn Gln Arg Glu Ser Tyr Phe Val Asp Ala Gln Pro
1               5                   10                  15

<210> 394
<211> 15
<212> PRT
<213> glycine max

<400> 394
Leu Leu Lys Asn Gln Arg Glu Ser Tyr Phe Val Asp Ala Gln Pro
1               5                   10                  15

<210> 395
<211> 15
<212> PRT
<213> glycine max

<400> 395
Pro Phe Leu Phe Gly Ser Asn Arg Phe Glu Thr Leu Phe Lys Asn
1               5                   10                  15

<210> 396

<211> 16
<212> PRT
<213> glycine max

<400> 396
Arg Phe Glu Thr Leu Phe Lys Asn Gln Tyr Gly Arg Ile Arg Val Leu
1               5                   10                  15

<210> 397
<211> 16
<212> PRT
<213> glycine max

<400> 397
Tyr Arg Ile Leu Glu Phe Asn Ser Lys Pro Asn Thr Leu Leu Leu Pro
1               5                   10                  15

<210> 398
<211> 16
<212> PRT
<213> glycine max

<400> 398
Thr Thr Tyr Tyr Val Val Asn Pro Asp Asn Asn Glu Asn Leu Arg Leu
1               5                   10                  15

<210> 399
<211> 15
<212> PRT
<213> glycine max

<400> 399
Leu Arg Leu Ile Thr Leu Ala Ile Pro Val Asn Lys Pro Gly Arg
1               5                   10                  15

<210> 400
<211> 16
<212> PRT
<213> glycine max

<400> 400
Ile Pro Val Asn Lys Pro Gly Arg Phe Glu Ser Phe Phe Leu Ser Ser
1               5                   10                  15

<210> 401
<211> 15
<212> PRT
<213> glycine max

<400> 401
Ser Arg Asn Ile Leu Glu Ala Ser Tyr Asp Thr Lys Phe Glu Glu
1               5                   10                  15

<210> 402
<211> 17

<212> PRT
<213> glycine max

<400> 402
Leu Phe Ser Arg Glu Glu Gly Gln Gln Gln Gly Glu Gln Arg Leu Gln
1               5                   10                  15
Glu

<210> 403
<211> 15
<212> PRT
<213> glycine max

<400> 403
Gly Glu Gln Arg Leu Gln Glu Ser Val Ile Val Glu Ile Ser Lys
1               5                   10                  15

<210> 404
<211> 16
<212> PRT
<213> glycine max

<400> 404
Leu Ser Lys Arg Ala Lys Ser Ser Ser Arg Lys Thr Ile Ser Ser Glu
1               5                   10                  15

<210> 405
<211> 16
<212> PRT
<213> glycine max

<400> 405
Phe Phe Glu Ile Thr Pro Glu Lys Asn Pro Gln Leu Arg Asp Leu Asp
1               5                   10                  15

<210> 406
<211> 16
<212> PRT
<213> glycine max

<400> 406
Ser Glu Gln Asp Ile Phe Val Ile Pro Ala Gly Tyr Pro Val Val Val
1               5                   10                  15

<210> 407
<211> 15
<212> PRT
<213> glycine max

<400> 407
Gln Val Gln Glu Leu Ala Phe Pro Gly Ser Ala Gln Ala Val Glu
1               5                   10                  15

<210> 408
<211> 15
<212> PRT

<213> glycine max

<400> 408
Arg Cys Asn Leu Leu Lys Val Glu Glu Glu Glu Glu Cys Glu Glu
1               5                   10                  15

<210> 409
<211> 15
<212> PRT
<213> glycine max

<400> 409
Gln Ile Pro Arg Pro Arg Pro Gln His Pro Glu Arg Glu Arg Gln
1               5                   10                  15

<210> 410
<211> 16
<212> PRT
<213> glycine max

<400> 410
Pro Arg Pro Gln His Pro Glu Arg Glu Arg Gln Gln His Gly Glu Lys
1               5                   10                  15

<210> 411
<211> 16
<212> PRT
<213> glycine max

<400> 411
Glu Arg Gln Gln His Gly Glu Lys Glu Glu Asp Glu Gly Glu Gln Pro
1               5                   10                  15

<210> 412
<211> 15
<212> PRT
<213> glycine max

<400> 412
His Gly Glu Lys Glu Glu Asp Glu Gly Glu Gln Pro Arg Pro Phe
1               5                   10                  15

<210> 413
<211> 15
<212> PRT
<213> glycine max

<400> 413
Gly Glu Gln Pro Arg Pro Phe Pro Phe Pro Arg Pro Arg Gln Pro
1               5                   10                  15

<210> 414
<211> 15
<212> PRT
<213> glycine max

<400> 414
Arg Pro Phe Pro Phe Pro Arg Pro Arg Gln Pro His Gln Glu Glu
1               5                   10                  15

<210> 415
<211> 15
<212> PRT
<213> glycine max

<400> 415
Gln Glu Glu Glu His Glu Gln Lys Glu Glu His Glu Trp His Arg
1               5                   10                  15

<210> 416
<211> 15
<212> PRT
<213> glycine max

<400> 416
Gln Glu Glu Glu His Glu Gln Lys Glu Glu His Glu Trp His Arg
1               5                   10                  15

<210> 417
<211> 15
<212> PRT
<213> glycine max

<400> 417
Gly Gly Lys Gly Ser Glu Glu Glu Gln Asp Glu Arg Glu His Pro
1               5                   10                  15

<210> 418
<211> 16
<212> PRT
<213> glycine max

<400> 418
His Lys Gln Glu Lys His Gln Gly Lys Glu Ser Glu Glu Glu Glu Glu
1               5                   10                  15

<210> 419
<211> 16
<212> PRT
<213> glycine max

<400> 419
His Lys Gln Glu Lys His Gln Gly Lys Glu Ser Glu Glu Glu Glu Glu
1               5                   10                  15

<210> 420
<211> 15
<212> PRT
<213> glycine max

<400> 420
His Gln Gly Lys Glu Ser Glu Glu Glu Glu Glu Asp Gln Asp Glu
1               5                   10                  15

<210> 421
<211> 15
<212> PRT
<213> glycine max

<400> 421
Glu Ser Glu Glu Glu Glu Glu Asp Gln Asp Glu Asp Glu Glu Gln
1               5                   10                  15

<210> 422
<211> 15
<212> PRT
<213> glycine max

<400> 422
Lys Glu Ser Gln Glu Ser Glu Gly Ser Glu Ser Gln Arg Glu Pro
1               5                   10                  15

<210> 423
<211> 15
<212> PRT
<213> glycine max

<400> 423
Lys Glu Ser Gln Glu Ser Glu Gly Ser Glu Ser Gln Arg Glu Pro
1               5                   10                  15

<210> 424
<211> 15
<212> PRT
<213> glycine max

<400> 424
Asn Lys Arg Ser Gln Gln Leu Gln Asn Leu Arg Asp Tyr Arg Ile
1               5                   10                  15

<210> 425
<211> 15
<212> PRT
<213> glycine max

<400> 425
Lys Pro Asn Thr Leu Leu Leu Pro His His Ala Asp Ala Asp Tyr
1               5                   10                  15

<210> 426
<211> 15
<212> PRT
<213> glycine max

<400> 426
Phe Glu Glu Ile Asn Lys Val Leu Phe Gly Arg Glu Glu Gly Gln
1               5                   10                  15

<210> 427
<211> 15
<212> PRT
<213> glycine max

<400> 427
Ala Ile Val Val Leu Val Ile Asn Glu Gly Glu Ala Asn Ile Glu
1               5                   10                  15

<210> 428
<211> 15
<212> PRT
<213> glycine max

<400> 428
Asn Ile Glu Leu Val Gly Ile Lys Glu Gln Gln Gln Arg Gln Gln
1               5                   10                  15

<210> 429
<211> 15
<212> PRT
<213> glycine max

<400> 429
Gln Asp Ile Phe Val Ile Pro Ala Gly Tyr Pro Val Met Val Asn
1               5                   10                  15

<210> 430
<211> 15
<212> PRT
<213> glycine max

<400> 430
Glu Ser Tyr Phe Val Asp Ala Gln Pro Gln Gln Lys Glu Glu Gly
1               5                   10                  15

<210> 431
<211> 15
<212> PRT
<213> glycine max

<400> 431
Glu Ser Tyr Phe Val Asp Ala Gln Pro Gln Gln Lys Glu Glu Gly
1               5                   10                  15

<210> 432
<211> 15
<212> PRT
<213> glycine max

<400> 432
Glu Ser Tyr Phe Val Asp Ala Gln Pro Gln Gln Lys Glu Glu Gly
1               5                   10                  15

<210> 433
<211> 15
<212> PRT
<213> glycine max

<400> 433
Pro Leu Leu Leu Leu Gly Val Val Phe Leu Ala Ser Val Ser Val
1               5                   10                  15

<210> 434
<211> 15
<212> PRT
<213> glycine max

<400> 434
His Phe Asn Ser Lys Arg Phe Gln Thr Leu Phe Lys Asn Gln Tyr
1               5                   10                  15

<210> 435
<211> 15
<212> PRT
<213> glycine max

<400> 435
Ile Thr Gln Arg Asn Pro Gln Leu Arg Asp Leu Asp Val Phe Leu
1               5                   10                  15

<210> 436
<211> 15
<212> PRT
<213> glycine max

<400> 436
His Pro Arg Pro His Gln Pro His Gln Lys Glu Glu Glu Lys His
1               5                   10                  15

<210> 437
<211> 15
<212> PRT
<213> glycine max

<400> 437
Leu Val Asn Asn Asp Asp Arg Asp Ser Tyr Asn Leu Gln Ser Gly
1               5                   10                  15

<210> 438
<211> 15
<212> PRT
<213> glycine max

<400> 438
Gln Ser Gly Asp Ala Leu Arg Val Pro Ala Gly Thr Thr Phe Tyr
1               5                   10                  15

<210> 439
<211> 15
<212> PRT
<213> glycine max

<400> 439
Gln Arg Phe Asn Lys Arg Ser Pro Gln Leu Glu Asn Leu Arg Asp
1               5                   10                  15

<210> 440
<211> 15
<212> PRT
<213> glycine max

<400> 440
Arg Asp Ser Tyr Asn Leu His Pro Gly Asp Ala Gln Arg Ile Pro
1               5                   10                  15

<210> 441
<211> 15
<212> PRT
<213> glycine max

<400> 441
Arg Asp Ser Tyr Asn Leu His Pro Gly Asp Ala Gln Arg Ile Pro
1               5                   10                  15

<210> 442
<211> 15
<212> PRT
<213> glycine max

<400> 442
Ser His Asn Ile Leu Glu Thr Ser Phe His Ser Glu Phe Glu Glu
1               5                   10                  15

<210> 443
<211> 15
<212> PRT
<213> glycine max

<400> 443
Ser His Asn Ile Leu Glu Thr Ser Phe His Ser Glu Phe Glu Glu
1               5                   10                  15

<210> 444
<211> 15
<212> PRT
<213> glycine max

<400> 444
Leu Glu Thr Ser Phe His Ser Glu Phe Glu Glu Ile Asn Arg Val
1               5                   10                  15

<210> 445
<211> 15
<212> PRT
<213> glycine max

<400> 445
Asn Arg Val Leu Phe Gly Glu Glu Glu Glu Gln Arg Gln Gln Glu
1               5                   10                  15

<210> 446
<211> 15
<212> PRT
<213> glycine max

<400> 446
Asn Arg Val Leu Phe Gly Glu Glu Glu Glu Gln Arg Gln Gln Glu
1               5                   10                  15

<210> 447
<211> 15
<212> PRT
<213> glycine max

<400> 447
Phe Gly Glu Glu Glu Glu Gln Arg Gln Gln Glu Gly Val Ile Val
1               5                   10                  15

<210> 448
<211> 15
<212> PRT
<213> glycine max

<400> 448
Ala Ile Val Ile Leu Val Ile Asn Glu Gly Asp Ala Asn Ile Glu
1               5                   10                  15

<210> 449
<211> 15
<212> PRT
<213> glycine max

<400> 449
Leu Leu Gly Thr Val Phe Leu Ala Ser Val Cys Val Ser Leu Lys
1               5                   10                  15

<210> 450
<211> 16
<212> PRT
<213> glycine max

<400> 450
Phe Leu Leu Phe Val Leu Ser Gly Arg Ala Ile Leu Thr Leu Val Asn
1               5                   10                  15

<210> 451
<211> 15
<212> PRT
<213> glycine max

<400> 451
Val Asn Pro His Asp His Gln Asn Leu Lys Ile Ile Lys Leu Ala
1               5                   10                  15

<210> 452
<211> 16
<212> PRT
<213> glycine max

<400> 452
Ala Ile Pro Val Asn Lys Pro Gly Arg Tyr Asp Asp Phe Phe Leu Ser
1               5                   10                  15

<210> 453
<211> 16
<212> PRT
<213> glycine max

<400> 453
Glu Gly Val Ile Val Glu Leu Ser Lys Glu Gln Ile Arg Gln Leu Ser
1               5                   10                  15

<210> 454
<211> 16
<212> PRT
<213> glycine max

<400> 454
Gly Ala Leu Leu Leu Pro His Phe Asn Ser Lys Ala Ile Val Ile Leu
1               5                   10                  15

<210> 455
<211> 16
<212> PRT
<213> glycine max

<400> 455
Gln Lys Gln Lys Gln Glu Glu Glu Pro Leu Glu Val Gln Arg Tyr Arg
1               5                   10                  15

<210> 456
<211> 16
<212> PRT
<213> glycine max

<400> 456
Asn Val Val Arg Gln Ile Glu Arg Gln Val Gln Glu Leu Ala Phe Pro
1               5                   10                  15

<210> 457
<211> 15
<212> PRT
<213> glycine max

<400> 457
Phe Ser Gly Cys Cys Phe Ala Phe Ser Ser Arg Glu Gln Pro Gln
1               5                   10                  15

<210> 458
<211> 15
<212> PRT
<213> glycine max

<400> 458
Phe Ser Gly Cys Cys Phe Ala Phe Ser Ser Arg Glu Gln Pro Gln
1               5                   10                  15

<210> 459
<211> 15
<212> PRT
<213> glycine max

<400> 459
Cys Phe Ala Phe Ser Ser Arg Glu Gln Pro Gln Gln Asn Glu Cys
1               5                   10                  15

<210> 460
<211> 15
<212> PRT
<213> glycine max

<400> 460
Arg Pro Ser Tyr Thr Asn Gly Pro Gln Glu Ile Tyr Ile Gln Gln
1               5                   10                  15

<210> 461
<211> 15
<212> PRT
<213> glycine max

<400> 461
Arg Pro Ser Tyr Thr Asn Gly Pro Gln Glu Ile Tyr Ile Gln Gln
1               5                   10                  15

<210> 462

<211> 15
<212> PRT
<213> glycine max

<400> 462
Gly Met Ile Tyr Pro Gly Cys Pro Ser Thr Phe Glu Glu Pro Gln
1               5                   10                  15

<210> 463
<211> 15
<212> PRT
<213> glycine max

<400> 463
Gly Met Ile Tyr Pro Gly Cys Pro Ser Thr Phe Glu Glu Pro Gln
1               5                   10                  15

<210> 464
<211> 15
<212> PRT
<213> glycine max

<400> 464
Ser Thr Phe Glu Glu Pro Gln Gln Pro Gln Gln Arg Gly Gln Ser
1               5                   10                  15

<210> 465
<211> 15
<212> PRT
<213> glycine max

<400> 465
Ser Thr Phe Glu Glu Pro Gln Gln Pro Gln Gln Arg Gly Gln Ser
1               5                   10                  15

<210> 466
<211> 15
<212> PRT
<213> glycine max

<400> 466
Glu Pro Gln Gln Pro Gln Gln Arg Gly Gln Ser Ser Arg Pro Gln
1               5                   10                  15

<210> 467
<211> 15
<212> PRT
<213> glycine max

<400> 467
Pro Gln Gln Arg Gly Gln Ser Ser Arg Pro Gln Asp Arg His Gln
1               5                   10                  15

<210> 468
<211> 15
<212> PRT
<213> glycine max

<400> 468
Arg Pro Gln Asp Arg His Gln Lys Ile Tyr Asn Phe Arg Glu Gly
1               5                   10                  15

<210> 469

<211> 15
<212> PRT
<213> glycine max

<400> 469
Arg Glu Gly Asp Leu Ile Ala Val Pro Thr Gly Val Ala Trp Trp
1               5                   10                  15

<210> 470
<211> 15
<212> PRT
<213> glycine max

<400> 470
Arg Glu Gly Asp Leu Ile Ala Val Pro Thr Gly Val Ala Trp Trp
1               5                   10                  15

<210> 471
<211> 15
<212> PRT
<213> glycine max

<400> 471
Asn Ser Leu Glu Asn Gln Leu Asp Gln Met Pro Arg Arg Phe Tyr
1               5                   10                  15

<210> 472
<211> 15
<212> PRT
<213> glycine max

<400> 472
Ala Gly Asn Gln Glu Gln Glu Phe Leu Lys Tyr Gln Gln Glu Gln
1               5                   10                  15

<210> 473
<211> 15
<212> PRT
<213> glycine max

<400> 473
Ala Gly Asn Gln Glu Gln Glu Phe Leu Lys Tyr Gln Gln Glu Gln
1               5                   10                  15

<210> 474
<211> 15
<212> PRT
<213> glycine max

<400> 474
Gly His Gln Ser Gln Lys Gly Lys His Gln Gln Glu Glu Glu Asn
1               5                   10                  15

<210> 475
<211> 15
<212> PRT
<213> glycine max

<400> 475
Gly His Gln Ser Gln Lys Gly Lys His Gln Gln Glu Glu Glu Asn
1               5                   10                  15

<210> 476

<211> 15
<212> PRT
<213> glycine max

<400> 476
Lys Gln Ile Ala Lys Asn Leu Gln Gly Glu Asn Glu Gly Glu Asp
1               5                   10                  15

<210> 477
<211> 15
<212> PRT
<213> glycine max

<400> 477
Gly Leu Ser Val Ile Lys Pro Pro Thr Asp Glu Gln Gln Gln Arg
1               5                   10                  15

<210> 478
<211> 15
<212> PRT
<213> glycine max

<400> 478
Ile Lys Pro Pro Thr Asp Glu Gln Gln Gln Arg Pro Gln Glu Glu
1               5                   10                  15

<210> 479
<211> 15
<212> PRT
<213> glycine max

<400> 479
Asp Glu Lys Pro Gln Cys Lys Gly Lys Asp Lys His Cys Gln Arg
1               5                   10                  15

<210> 480
<211> 15
<212> PRT
<213> glycine max

<400> 480
Asp Glu Lys Pro Gln Cys Lys Gly Lys Asp Lys His Cys Gln Arg
1               5                   10                  15

<210> 481
<211> 15
<212> PRT
<213> glycine max

<400> 481
Phe Gly Ser Leu Arg Lys Asn Ala Met Phe Val Pro His Tyr Asn
1               5                   10                  15

<210> 482
<211> 15
<212> PRT
<213> glycine max

<400> 482
Asn Asn Pro Phe Lys Phe Leu Val Pro Pro Gln Glu Ser Gln Lys
1               5                   10                  15

<210> 483

<211> 15
<212> PRT
<213> glycine max

<400> 483
Asn Asn Pro Phe Lys Phe Leu Val Pro Pro Gln Glu Ser Gln Lys
1               5                   10                  15

<210> 484
<211> 16
<212> PRT
<213> glycine max

<400> 484
Asn Asn Lys Pro Phe Gln Cys Ala Gly Val Ala Leu Ser Arg Cys Thr
1               5                   10                  15

<210> 485
<211> 16
<212> PRT
<213> glycine max

<400> 485
Lys His Cys Gln Arg Pro Arg Gly Ser Gln Ser Lys Ser Arg Arg Asn
1               5                   10                  15

<210> 486
<211> 18
<212> PRT
<213> glycine max

<400> 486
Lys Asn Ala Met Phe Val Pro His Tyr Asn Leu Asn Ala Asn Ser Ile
1               5                   10                  15
Ile Tyr

<210> 487
<211> 16
<212> PRT
<213> glycine max

<400> 487
Leu Asn Gly Arg Ala Leu Ile Gln Val Val Asn Cys Asn Gly Glu Arg
1               5                   10                  15

<210> 488
<211> 15
<212> PRT
<213> glycine max

<400> 488
Gln Asn Phe Val Val Ala Ala Arg Ser Gln Ser Asp Asn Phe Glu
1               5                   10                  15

<210> 489
<211> 15

<212> PRT
<213> glycine max

<400> 489
Ala Arg Ser Gln Ser Asp Asn Phe Glu Tyr Val Ser Phe Lys Thr
1               5                   10                  15

<210> 490
<211> 15
<212> PRT
<213> glycine max

<400> 490
Phe Ala Leu Arg Glu Gln Ala Gln Gln Asn Glu Cys Gln Ile Gln
1               5                   10                  15

<210> 491
<211> 15
<212> PRT
<213> glycine max

<400> 491
Gln Ile Gln Lys Leu Asn Ala Leu Lys Pro Asp Asn Arg Ile Glu
1               5                   10                  15

<210> 492
<211> 15
<212> PRT
<213> glycine max

<400> 492
Leu Asn Ala Leu Lys Pro Asp Asn Arg Ile Glu Ser Glu Gly Gly
1               5                   10                  15

<210> 493
<211> 15
<212> PRT
<213> glycine max

<400> 493
Phe Pro Gly Cys Pro Ser Thr Tyr Gln Glu Pro Gln Glu Ser Gln
1               5                   10                  15

<210> 494
<211> 15
<212> PRT
<213> glycine max

<400> 494
Phe Pro Gly Cys Pro Ser Thr Tyr Gln Glu Pro Gln Glu Ser Gln
1               5                   10                  15

<210> 495
<211> 15
<212> PRT
<213> glycine max

<400> 495
Glu Ser Gln Gln Arg Gly Arg Ser Gln Arg Pro Gln Asp Arg His
1               5                   10                  15

<210> 496
<211> 15

<212> PRT
<213> glycine max

<400> 496
Glu Ser Gln Gln Arg Gly Arg Ser Gln Arg Pro Gln Asp Arg His
1               5                   10                  15

<210> 497
<211> 15
<212> PRT
<213> glycine max

<400> 497
Gln Arg Pro Gln Asp Arg His Gln Lys Val His Arg Phe Arg Glu
1               5                   10                  15

<210> 498
<211> 15
<212> PRT
<213> glycine max

<400> 498
Gln Arg Pro Gln Asp Arg His Gln Lys Val His Arg Phe Arg Glu
1               5                   10                  15

<210> 499
<211> 15
<212> PRT
<213> glycine max

<400> 499
Gln Ser Gln Lys Gly Lys Gln Gln Glu Glu Glu Asn Glu Gly Ser
1               5                   10                  15

<210> 500
<211> 15
<212> PRT
<213> glycine max

<400> 500
Ala Phe Gly Val Asn Met Gln Ile Val Arg Asn Leu Gln Gly Glu
1               5                   10                  15

<210> 501
<211> 15
<212> PRT
<213> glycine max

<400> 501
Val Thr Ala Pro Ala Met Arg Lys Pro Gln Gln Glu Glu Asp Asp
1               5                   10                  15

<210> 502
<211> 15
<212> PRT
<213> glycine max

<400> 502
Pro Gln Gln Glu Glu Asp Asp Asp Asp Glu Glu Glu Gln Pro Gln
1               5                   10                  15

<210> 503
<211> 15

<212> PRT
<213> glycine max

<400> 503
Gln Asn Ile Gly Gln Asn Ser Ser Pro Asp Ile Tyr Asn Pro Gln
1               5                   10                  15

<210> 504
<211> 15
<212> PRT
<213> glycine max

<400> 504
Asn Cys Asn Gly Glu Arg Val Phe Asp Gly Glu Leu Gln Glu Gly
1               5                   10                  15

<210> 505
<211> 15
<212> PRT
<213> glycine max

<400> 505
Asn Cys Asn Gly Glu Arg Val Phe Asp Gly Glu Leu Gln Glu Gly
1               5                   10                  15

<210> 506
<211> 16
<212> PRT
<213> glycine max

<400> 506
Met Ile Phe Pro Gly Cys Pro Ser Thr Tyr Gln Glu Pro Gln Glu Ser
1               5                   10                  15

<210> 507
<211> 16
<212> PRT
<213> glycine max

<400> 507
Tyr Gln Gln Gln Gln Gln Gly Gly Ser Gln Ser Gln Lys Gly Lys Gln
1               5                   10                  15

<210> 508
<211> 15
<212> PRT
<213> glycine max

<400> 508
Asn Glu Glu Glu Asp Ser Gly Ala Ile Val Thr Val Lys Gly Gly
1               5                   10                  15

<210> 509
<211> 16
<212> PRT
<213> glycine max

<400> 509
Ile Val Thr Val Lys Gly Gly Leu Arg Val Thr Ala Pro Ala Met Arg

1               5                   10                  15

<210> 510
<211> 15
<212> PRT
<213> glycine max

<400> 510
Thr Ile Cys Thr Met Arg Leu Arg Gln Asn Ile Gly Gln Asn Ser
1               5                   10                  15

<210> 511
<211> 16
<212> PRT
<213> glycine max

<400> 511
Pro Ala Leu Trp Leu Leu Lys Leu Ser Ala Gln Tyr Gly Ser Leu Arg
1               5                   10                  15

<210> 512
<211> 16
<212> PRT
<213> glycine max

<400> 512
Pro Gln Asn Phe Ala Val Ala Ala Lys Ser Gln Ser Asp Asn Phe Glu
1               5                   10                  15

<210> 513
<211> 16
<212> PRT
<213> glycine max

<400> 513
Lys Thr Asn Asp Arg Pro Ser Ile Gly Asn Leu Ala Gly Ala Asn Ser
1               5                   10                  15

<210> 514
<211> 17
<212> PRT
<213> glycine max

<400> 514
Lys Asn Asn Asn Pro Phe Ser Phe Leu Val Pro Pro Gln Glu Ser Gln
1               5                   10                  15
Arg

<210> 515
<211> 15
<212> PRT
<213> glycine max

<400> 515

Ser Phe Arg Glu Gln Pro Gln Gln Asn Glu Cys Gln Ile Gln Arg
1               5                   10                  15

<210> 516
<211> 15
<212> PRT
<213> glycine max

<400> 516
Ile Gln Gln Gly Ser Gly Ile Phe Gly Met Ile Phe Pro Gly Cys
1               5                   10                  15

<210> 517
<211> 15
<212> PRT
<213> glycine max

<400> 517
Ser Thr Phe Glu Glu Pro Gln Gln Lys Gly Gln Ser Ser Arg Pro
1               5                   10                  15

<210> 518
<211> 15
<212> PRT
<213> glycine max

<400> 518
Ser Arg Pro Gln Asp Arg His Gln Lys Ile Tyr His Phe Arg Glu
1               5                   10                  15

<210> 519
<211> 15
<212> PRT
<213> glycine max

<400> 519
Phe Arg Glu Gly Asp Leu Ile Ala Val Pro Thr Gly Phe Ala Tyr
1               5                   10                  15

<210> 520
<211> 15
<212> PRT
<213> glycine max

<400> 520
Thr Asn Ser Phe Gln Asn Gln Leu Asp Gln Met Pro Arg Arg Phe
1               5                   10                  15

<210> 521
<211> 15
<212> PRT
<213> glycine max

<400> 521
Leu Ser Val Ile Ser Pro Pro Thr Glu Glu Gln Gln Gln Arg Pro
1               5                   10                  15

<210> 522
<211> 15
<212> PRT
<213> glycine max

<400> 522

Glu Glu Gln Gln Gln Arg Pro Glu Glu Glu Glu Lys Pro Asp Cys
1               5                   10                  15

<210> 523
<211> 15
<212> PRT
<213> glycine max

<400> 523
Pro Asp Cys Asp Glu Lys Asp Lys His Cys Gln Ser Gln Ser Arg
1               5                   10                  15

<210> 524
<211> 15
<212> PRT
<213> glycine max

<400> 524
Pro Asp Cys Asp Glu Lys Asp Lys His Cys Gln Ser Gln Ser Arg
1               5                   10                  15

<210> 525
<211> 15
<212> PRT
<213> glycine max

<400> 525
Pro Asp Cys Asp Glu Lys Asp Lys His Cys Gln Ser Gln Ser Arg
1               5                   10                  15

<210> 526
<211> 15
<212> PRT
<213> glycine max

<400> 526
His Cys Gln Ser Gln Ser Arg Asn Gly Ile Asp Glu Thr Ile Cys
1               5                   10                  15

<210> 527
<211> 15
<212> PRT
<213> glycine max

<400> 527
His Asn Ile Gly Gln Thr Ser Ser Pro Asp Ile Phe Asn Pro Gln
1               5                   10                  15

<210> 528
<211> 18
<212> PRT
<213> glycine max

<400> 528
Cys Phe Ala Phe Ser Phe Arg Glu Gln Pro Gln Gln Asn Glu Cys Gln
1               5                   10                  15
Ile Gln

<210> 529
<211> 16
<212> PRT
<213> glycine max

<400> 529
Glu Phe Leu Gln Tyr Gln Pro Gln Lys Gln Gln Gly Gly Thr Gln Ser
1               5                   10                  15

<210> 530
<211> 17
<212> PRT
<213> glycine max

<400> 530
Ala Phe Val Val Asp Arg Gln Ile Val Arg Lys Leu Gln Gly Glu Asn
1               5                   10                  15
Glu

<210> 531
<211> 16
<212> PRT
<213> glycine max

<400> 531
Gln Thr Phe Asn Leu Arg Arg Gln Gln Ala Arg Gln Val Lys Asn Asn
1               5                   10                  15

<210> 532
<211> 15
<212> PRT
<213> glycine max

<400> 532
Gln Leu Asn Asn Leu Asn Ala Leu Glu Pro Asp His Arg Val Glu
1               5                   10                  15

<210> 533
<211> 15
<212> PRT
<213> glycine max

<400> 533
Arg Asn Gly Leu His Ser Pro Ser Tyr Ser Pro Tyr Pro Arg Met
1               5                   10                  15

<210> 534
<211> 15
<212> PRT
<213> glycine max

<400> 534
Arg Asn Gly Leu His Ser Pro Ser Tyr Ser Pro Tyr Pro Arg Met
1               5                   10                  15

<210> 535
<211> 15
<212> PRT
<213> glycine max

<400> 535
Leu Gly Val Ala Ile Pro Gly Cys Pro Glu Thr Phe Glu Glu Pro

1               5                   10                  15

<210> 536
<211> 15
<212> PRT
<213> glycine max

<400> 536
Leu Gly Val Ala Ile Pro Gly Cys Pro Glu Thr Phe Glu Glu Pro
1               5                   10                  15

<210> 537
<211> 15
<212> PRT
<213> glycine max

<400> 537
Gln Gln Leu Gln Asp Ser His Gln Lys Ile Arg His Phe Asn Glu
1               5                   10                  15

<210> 538
<211> 15
<212> PRT
<213> glycine max

<400> 538
Gln Gln Leu Gln Asp Ser His Gln Lys Ile Arg His Phe Asn Glu
1               5                   10                  15

<210> 539
<211> 15
<212> PRT
<213> glycine max

<400> 539
Pro Asp Ile Glu Tyr Pro Glu Thr Met Gln Gln Gln Gln Gln Gln
1               5                   10                  15

<210> 540
<211> 15
<212> PRT
<213> glycine max

<400> 540
Gln Gln Gln Lys Ser His Gly Gly Arg Lys Gln Gly Gln His Gln
1               5                   10                  15

<210> 541
<211> 15
<212> PRT
<213> glycine max

<400> 541
Asn Glu Asp Ile Ala Glu Lys Leu Glu Ser Pro Asp Asp Glu Arg
1               5                   10                  15

<210> 542
<211> 15
<212> PRT
<213> glycine max

<400> 542
Asn Glu Asp Ile Ala Glu Lys Leu Glu Ser Pro Asp Asp Glu Arg

1               5                   10                  15

<210> 543
<211> 15
<212> PRT
<213> glycine max

<400> 543
Leu Ser Val Ile Ser Pro Lys Trp Gln Glu Gln Gln Asp Glu Asp
1               5                   10                  15

<210> 544
<211> 15
<212> PRT
<213> glycine max

<400> 544
Leu Ser Val Ile Ser Pro Lys Trp Gln Glu Gln Gln Asp Glu Asp
1               5                   10                  15

<210> 545
<211> 15
<212> PRT
<213> glycine max

<400> 545
Ser His Pro Pro Arg Arg Pro Ser His Gly Lys Arg Glu Gln Asp
1               5                   10                  15

<210> 546
<211> 15
<212> PRT
<213> glycine max

<400> 546
Ser His Pro Pro Arg Arg Pro Ser His Gly Lys Arg Glu Gln Asp
1               5                   10                  15

<210> 547
<211> 15
<212> PRT
<213> glycine max

<400> 547
Arg Arg Pro Ser His Gly Lys Arg Glu Gln Asp Glu Asp Glu Asp
1               5                   10                  15

<210> 548
<211> 15
<212> PRT
<213> glycine max

<400> 548
Gly Lys Arg Asn Lys Thr Gly Gln Asp Glu Asp Glu Asp Glu Asp
1               5                   10                  15

<210> 549
<211> 15
<212> PRT
<213> glycine max

<400> 549
Trp Arg Ser Lys Lys Thr Gln Pro Arg Arg Pro Arg Gln Glu Glu

1                   5                   10                  15

<210> 550
<211> 15
<212> PRT
<213> glycine max

<400> 550
Lys Thr Gln Pro Arg Arg Pro Arg Gln Glu Glu Pro Arg Glu Arg
1               5                   10                  15

<210> 551
<211> 15
<212> PRT
<213> glycine max

<400> 551
Lys Thr Gln Pro Arg Arg Pro Arg Gln Glu Glu Pro Arg Glu Arg
1               5                   10                  15

<210> 552
<211> 15
<212> PRT
<213> glycine max

<400> 552
His Asn Ala Val Thr Ser Tyr Leu Lys Asp Val Phe Arg Ala Ile
1               5                   10                  15

<210> 553
<211> 15
<212> PRT
<213> glycine max

<400> 553
Met Ile Ile Ile Ala Gln Gly Lys Gly Ala Leu Gly Val Ala Ile
1               5                   10                  15

<210> 554
<211> 16
<212> PRT
<213> glycine max

<400> 554
Glu Glu Gly Gly Ser Val Leu Ser Gly Phe Ser Lys His Phe Leu Ala
1               5                   10                  15

<210> 555
<211> 15
<212> PRT
<213> glycine max

<400> 555
Ser Ser Ser Lys Leu Asn Glu Cys Gln Leu Asn Asn Leu Asn Ala
1               5                   10                  15

<210> 556
<211> 15
<212> PRT
<213> glycine max

<400> 556
Cys Gln Leu Asn Asn Leu Asn Ala Leu Glu Pro Asp His Arg Val
1               5                   10                  15

<210> 557
<211> 15
<212> PRT
<213> glycine max

<400> 557
Asn Leu Asn Ala Leu Glu Pro Asp His Arg Val Glu Ser Glu Gly
1               5                   10                  15

<210> 558
<211> 15
<212> PRT
<213> glycine max

<400> 558
Asn Arg Asn Gly Ser His Leu Pro Ser Tyr Leu Pro Tyr Pro Gln
1               5                   10                  15

<210> 559
<211> 15
<212> PRT
<213> glycine max

<400> 559
Asn Arg Asn Gly Ser His Leu Pro Ser Tyr Leu Pro Tyr Pro Gln
1               5                   10                  15

<210> 560
<211> 15
<212> PRT
<213> glycine max

<400> 560
Ala Ile Gly Phe Ala Phe Pro Gly Cys Pro Glu Thr Phe Glu Lys
1               5                   10                  15

<210> 561
<211> 15
<212> PRT
<213> glycine max

<400> 561
Ala Ile Gly Phe Ala Phe Pro Gly Cys Pro Glu Thr Phe Glu Lys
1               5                   10                  15

<210> 562
<211> 15
<212> PRT
<213> glycine max

<400> 562
Phe Asn Glu Gly Asp Val Leu Val Ile Pro Leu Gly Val Pro Tyr
1               5                   10                  15

<210> 563
<211> 15
<212> PRT
<213> glycine max

<400> 563
Thr Tyr Asn Thr Gly Asp Glu Pro Val Val Ala Ile Ser Pro Leu
1               5                   10                  15

<210> 564
<211> 15
<212> PRT
<213> glycine max

<400> 564
Arg Val Phe Tyr Leu Ala Gly Asn Pro Asp Ile Glu His Pro Glu
1               5                   10                  15

<210> 565
<211> 15
<212> PRT
<213> glycine max

<400> 565
Arg Val Phe Tyr Leu Ala Gly Asn Pro Asp Ile Glu His Pro Glu
1               5                   10                  15

<210> 566
<211> 16
<212> PRT
<213> glycine max

<400> 566
Arg Val Phe Tyr Leu Ala Gly Asn Pro Asp Ile Glu His Pro Glu Thr
1               5                   10                  15

<210> 567
<211> 15
<212> PRT
<213> glycine max

<400> 567
Gln Gln Gln Lys Ser His Gly Gly Arg Lys Gln Gly Gln His Arg
1               5                   10                  15

<210> 568
<211> 15
<212> PRT
<213> glycine max

<400> 568
Ser His Gly Gly Arg Lys Gln Gly Gln His Arg Gln Gln Glu Glu
1               5                   10                  15

<210> 569
<211> 15
<212> PRT
<213> glycine max

<400> 569
Arg Lys Gln Gly Gln His Arg Gln Gln Glu Glu Glu Gly Gly Ser
1               5                   10                  15

<210> 570
<211> 15
<212> PRT

<213> glycine max

<400> 570
Glu Asp Thr Ala Glu Lys Leu Arg Ser Pro Asp Asp Glu Arg Lys
1               5                   10                  15

<210> 571
<211> 15
<212> PRT
<213> glycine max

<400> 571
Glu Gly Gly Leu Ser Val Ile Ser Pro Lys Trp Gln Glu Gln Glu
1               5                   10                  15

<210> 572
<211> 15
<212> PRT
<213> glycine max

<400> 572
Glu Gly Gly Leu Ser Val Ile Ser Pro Lys Trp Gln Glu Gln Glu
1               5                   10                  15

<210> 573
<211> 15
<212> PRT
<213> glycine max

<400> 573
Ser Val Ile Ser Pro Lys Trp Gln Glu Gln Glu Asp Glu Asp Glu
1               5                   10                  15

<210> 574
<211> 15
<212> PRT
<213> glycine max

<400> 574
Glu Gln Glu Asp Glu Asp Glu Asp Glu Asp Glu Glu Tyr Gly Arg
1               5                   10                  15

<210> 575
<211> 15
<212> PRT
<213> glycine max

<400> 575
Ser His Gly Lys His Glu Asp Asp Glu Asp Glu Asp Glu Glu Glu
1               5                   10                  15

<210> 576
<211> 15
<212> PRT
<213> glycine max

<400> 576
His Glu Asp Asp Glu Asp Glu Asp Glu Glu Glu Asp Gln Pro Arg
1               5                   10                  15

<210> 577
<211> 15
<212> PRT

<213> glycine max

<400> 577
Glu Asp Glu Asp Glu Glu Glu Asp Gln Pro Arg Pro Asp His Pro
1               5                   10                  15

<210> 578
<211> 15
<212> PRT
<213> glycine max

<400> 578
Glu Glu Glu Asp Gln Pro Arg Pro Asp His Pro Pro Gln Arg Pro
1               5                   10                  15

<210> 579
<211> 15
<212> PRT
<213> glycine max

<400> 579
Gln Pro Arg Pro Asp His Pro Pro Gln Arg Pro Ser Arg Pro Glu
1               5                   10                  15

<210> 580
<211> 15
<212> PRT
<213> glycine max

<400> 580
Asp His Pro Pro Gln Arg Pro Ser Arg Pro Glu Gln Gln Glu Pro
1               5                   10                  15

<210> 581
<211> 15
<212> PRT
<213> glycine max

<400> 581
Arg Pro Glu Gln Gln Glu Pro Arg Gly Arg Gly Cys Gln Thr Arg
1               5                   10                  15

<210> 582
<211> 15
<212> PRT
<213> glycine max

<400> 582
Cys Gln Gly Asn Ala Val Phe Asp Gly Glu Leu Arg Arg Gly Gln
1               5                   10                  15

<210> 583
<211> 15
<212> PRT
<213> glycine max

<400> 583
Cys Gln Gly Asn Ala Val Phe Asp Gly Glu Leu Arg Arg Gly Gln
1               5                   10                  15

<210> 584
<211> 15
<212> PRT

<213> glycine max

<400> 584
Thr His His Asn Ala Val Ser Ser Tyr Ile Lys Asp Val Phe Arg
1               5                   10                  15

<210> 585
<211> 15
<212> PRT
<213> glycine max

<400> 585
Val Leu Ser Asn Ser Tyr Asn Leu Gly Gln Ser Gln Val Arg Gln
1               5                   10                  15

<210> 586
<211> 15
<212> PRT
<213> glycine max

<400> 586
Val Leu Ser Asn Ser Tyr Asn Leu Gly Gln Ser Gln Val Arg Gln
1               5                   10                  15

<210> 587
<211> 15
<212> PRT
<213> glycine max

<400> 587
Lys Tyr Gln Gly Asn Ser Gly Pro Leu Val Asn Pro Gly Ser Gly
1               5                   10                  15

<210> 588
<211> 15
<212> PRT
<213> glycine max

<400> 588
Lys Tyr Gln Gly Asn Ser Gly Pro Leu Val Asn Pro Gly Ser Gly
1               5                   10                  15

<210> 589
<211> 16
<212> PRT
<213> glycine max

<400> 589
Leu Ile Glu Thr Trp Asn Ser Gln His Pro Glu Leu Gln Cys Ala Gly
1               5                   10                  15

<210> 590
<211> 16
<212> PRT
<213> glycine max

<400> 590
Thr Trp Asn Ser Gln His Pro Glu Leu Gln Cys Ala Gly Val Thr Val
1               5                   10                  15

<210> 591
<211> 16
<212> PRT
<213> glycine max

<400> 591
Gln His Pro Glu Leu Gln Cys Ala Gly Val Thr Val Ser Lys Arg Thr
1               5                   10                  15

<210> 592
<211> 15
<212> PRT
<213> glycine max

<400> 592
Gln Cys Ala Gly Val Thr Val Ser Lys Arg Thr Leu Asn Arg Asn
1               5                   10                  15

<210> 593
<211> 16
<212> PRT
<213> glycine max

<400> 593
Ile Val Val Gln Gly Lys Gly Ala Ile Gly Phe Ala Phe Pro Gly Cys
1               5                   10                  15

<210> 594
<211> 15
<212> PRT
<213> glycine max

<400> 594
Gln Ser Ser Arg Arg Gly Ser Arg Ser Gln Gln Gln Leu Gln Asp
1               5                   10                  15

<210> 595
<211> 15
<212> PRT
<213> glycine max

<400> 595
Glu Arg Lys Gln Ile Val Thr Val Glu Gly Gly Leu Ser Val Ile
1               5                   10                  15

<210> 596
<211> 17
<212> PRT
<213> glycine max

<400> 596
Asp Glu Glu Tyr Gly Arg Thr Pro Ser Tyr Pro Pro Arg Arg Pro Ser
1               5                   10                  15
His

<210> 597
<211> 17

<212> PRT
<213> glycine max

<400> 597
Gln Thr Arg Asn Gly Val Glu Glu Asn Ile Cys Thr Met Lys Leu His
1               5                   10                  15
Glu

<210> 598
<211> 16
<212> PRT
<213> glycine max

<400> 598
Glu Asn Ile Ala Arg Pro Ser Arg Ala Asp Phe Tyr Asn Pro Lys Ala
1               5                   10                  15

<210> 599
<211> 15
<212> PRT
<213> glycine max

<400> 599
Arg Val Arg Val Val Asn Cys Gln Gly Asn Ala Val Phe Asp Gly
1               5                   10                  15

<210> 600
<211> 16
<212> PRT
<213> glycine max

<400> 600
Phe Asp Gly Glu Leu Arg Arg Gly Gln Leu Leu Val Val Pro Gln Asn
1               5                   10                  15

<210> 601
<211> 15
<212> PRT
<213> glycine max

<400> 601
Val Val Pro Gln Asn Pro Ala Val Ala Glu Gln Gly Gly Glu Gln
1               5                   10                  15

<210> 602
<211> 16
<212> PRT
<213> glycine max

<400> 602
Asp Val Phe Arg Val Ile Pro Ser Glu Val Leu Ser Asn Ser Tyr Asn
1               5                   10                  15

<210> 603
<211> 15
<212> PRT

<213> glycine max

<400> 603
Ser Glu Val Leu Ser Asn Ser Tyr Asn Leu Gly Gln Ser Gln Val
1               5                   10                  15

<210> 604
<211> 15
<212> PRT
<213> glycine max

<400> 604
Ala Tyr Val Asp Asp His Leu Leu Cys Gly Ile Glu Gly Asn His
1               5                   10                  15

<210> 605
<211> 15
<212> PRT
<213> glycine max

<400> 605
Gly Asn His Leu Thr His Ala Ala Ile Ile Gly Gln Asp Gly Ser
1               5                   10                  15

<210> 606
<211> 15
<212> PRT
<213> glycine max

<400> 606
Gly Asn His Leu Thr His Ala Ala Ile Ile Gly Gln Asp Gly Ser
1               5                   10                  15

<210> 607
<211> 15
<212> PRT
<213> glycine max

<400> 607
Thr His Ala Ala Ile Ile Gly Gln Asp Gly Ser Val Trp Leu Gln
1               5                   10                  15

<210> 608
<211> 15
<212> PRT
<213> glycine max

<400> 608
Leu Ile Ile Gly Ile Tyr Asp Glu Pro Met Thr Pro Gly Gln Cys
1               5                   10                  15

<210> 609
<211> 15
<212> PRT
<213> glycine max

<400> 609
Asp Phe Pro Gln Phe Lys Pro Glu Glu Ile Thr Ala Ile Met Asn
1               5                   10                  15

<210> 610
<211> 16
<212> PRT

<213> glycine max

<400> 610
Leu Gln Ser Thr Asp Phe Pro Gln Phe Lys Pro Glu Glu Ile Thr Ala
1               5                   10                  15

<210> 611
<211> 15
<212> PRT
<213> glycine max

<400> 611
Met Asn Asp Phe Asn Glu Pro Gly Ser Leu Ala Pro Thr Gly Leu
1               5                   10                  15

<210> 612
<211> 17
<212> PRT
<213> glycine max

<400> 612
Lys Tyr Met Val Ile Gln Gly Glu Pro Gly Ala Val Ile Arg Gly Lys
1               5                   10                  15
Lys

<210> 613
<211> 16
<212> PRT
<213> glycine max

<400> 613
Cys Asn Met Val Val Glu Arg Leu Gly Asp Tyr Leu Ile Asp Gln Gly
1               5                   10                  15

<210> 614
<211> 15
<212> PRT
<213> glycine max

<400> 614
Met Val Val Glu Arg Pro Gly Asp Tyr Leu Ile Asp Gln Gly Tyr
1               5                   10                  15

<210> 615
<211> 15
<212> PRT
<213> glycine max

<400> 615
Met Val Val Glu Arg Pro Gly Asp Tyr Leu Ile Asp Gln Gly Tyr
1               5                   10                  15

<210> 616
<211> 16
<212> PRT
<213> glycine max

<400> 616

Asp Asp His Leu Leu Cys Asp Ile Glu Gly Asn His Leu Thr His Ala
1               5                   10                  15

<210> 617
<211> 16
<212> PRT
<213> glycine max

<400> 617
Ile Ile Gly Gln Asp Gly Ser Val Trp Ala Gln Ser Thr Asp Phe Pro
1               5                   10                  15

<210> 618
<211> 15
<212> PRT
<213> glycine max

<400> 618
His Lys Ile Glu Ser Ile Asp Glu Ala Asn Leu Gly Tyr Ser Tyr
1               5                   10                  15

<210> 619
<211> 15
<212> PRT
<213> glycine max

<400> 619
His Lys Ile Glu Ser Ile Asp Glu Ala Asn Leu Gly Tyr Ser Tyr
1               5                   10                  15

<210> 620
<211> 15
<212> PRT
<213> glycine max

<400> 620
Val Glu Asn Val Glu Gly Asn Gly Gly Pro Gly Thr Ile Lys Lys
1               5                   10                  15

<210> 621
<211> 16
<212> PRT
<213> glycine max

<400> 621
Gly Val Phe Thr Phe Glu Asp Glu Ile Asn Ser Pro Val Ala Pro Ala
1               5                   10                  15

<210> 622
<211> 16
<212> PRT
<213> glycine max

<400> 622
Phe Glu Asp Glu Ile Asn Ser Pro Val Ala Pro Ala Thr Leu Tyr Lys
1               5                   10                  15

<210> 623
<211> 16
<212> PRT
<213> glycine max

<400> 623
Gly Gly Ala Ala Leu Pro Asp Thr Ala Glu Lys Ile Thr Phe Asp Ser
1               5                   10                  15

<210> 624
<211> 15
<212> PRT
<213> glycine max

<400> 624
Leu Pro Asp Thr Ala Glu Lys Ile Thr Phe Asp Ser Lys Leu Val
1               5                   10                  15

<210> 625
<211> 17
<212> PRT
<213> glycine max

<400> 625
Ala Gly Pro Asn Gly Gly Ser Ala Gly Lys Leu Thr Val Lys Tyr Glu
1               5                   10                  15
Thr

<210> 626
<211> 15
<212> PRT
<213> glycine max

<400> 626
Leu Thr Val Lys Tyr Glu Thr Lys Gly Asp Ala Glu Pro Asn Gln
1               5                   10                  15

<210> 627
<211> 19
<212> PRT
<213> glycine max

<400> 627
Leu Leu Ile Ser Leu Leu Phe Cys Ile Ala His Thr Cys Ser Ala Ser
1               5                   10                  15
Lys Trp Gln

<210> 628
<211> 17
<212> PRT
<213> glycine max

<400> 628
Ile Ala His Thr Cys Ser Ala Ser Lys Trp Gln His Gln Gln Asp Ser
1               5                   10                  15
Cys

<210> 629
<211> 16
<212> PRT
<213> glycine max

<400> 629
Ser Lys Trp Gln His Gln Gln Asp Ser Cys Arg Lys Gln Leu Gln Gly
1               5                   10                  15

<210> 630
<211> 16
<212> PRT
<213> glycine max

<400> 630
Gln His Gln Gln Asp Ser Cys Arg Lys Gln Leu Gln Gly Val Asn Leu
1               5                   10                  15

<210> 631
<211> 17
<212> PRT
<213> glycine max

<400> 631
Gln Asp Ser Cys Arg Lys Gln Leu Gln Gly Val Asn Leu Thr Pro Cys
1               5                   10                  15
Glu

<210> 632
<211> 17
<212> PRT
<213> glycine max

<400> 632
Gln Leu Gln Gly Val Asn Leu Thr Pro Cys Glu Lys His Ile Met Glu
1               5                   10                  15
Lys

<210> 633
<211> 15
<212> PRT
<213> glycine max

<400> 633
Ile Met Glu Lys Ile Gln Gly Arg Gly Asp Asp Asp Asp Asp Asp
1               5                   10                  15

<210> 634
<211> 17
<212> PRT
<213> glycine max

<400> 634
Asn Glu Gly Lys Asp Glu Asp Glu Glu Glu Glu Gly His Met Gln Lys
1               5                   10                  15
Cys

<210> 635
<211> 15
<212> PRT
<213> glycine max

<400> 635
Glu Glu Gly His Met Gln Lys Cys Cys Thr Glu Met Ser Glu Leu
1               5                   10                  15

<210> 636
<211> 16
<212> PRT
<213> glycine max

<400> 636
Met Gln Lys Cys Cys Thr Glu Met Ser Glu Leu Arg Ser Pro Lys Cys
1               5                   10                  15

<210> 637
<211> 16
<212> PRT
<213> glycine max

<400> 637
Ser Pro Lys Cys Gln Cys Lys Ala Leu Gln Lys Ile Met Glu Asn Gln
1               5                   10                  15

<210> 638
<211> 18
<212> PRT
<213> glycine max

<400> 638
Gln Lys Ile Met Glu Asn Gln Ser Glu Glu Leu Glu Glu Lys Gln Lys
1               5                   10                  15
Lys Lys

<210> 639
<211> 17
<212> PRT
<213> glycine max

<400> 639
Ile Asn Leu Ala Thr Met Cys Arg Phe Gly Pro Met Ile Gln Cys Asp
1               5                   10                  15
Leu

<210> 640
<211> 14
<212> PRT
<213> glycine max

<400> 640
Arg Phe Gly Pro Met Ile Gln Cys Asp Leu Ser Ser Asp Asp
1               5                   10

<210> 641
<211> 17
<212> PRT
<213> glycine max

<400> 641
Ile Ala His Thr Cys Cys Ala Ser Lys Trp Gln Gln His Gln Gln Glu
1               5                   10                  15
Ser

<210> 642
<211> 17
<212> PRT
<213> glycine max

<400> 642
Cys Cys Ala Ser Lys Trp Gln Gln His Gln Gln Glu Ser Cys Arg Glu
1               5                   10                  15
Gln

<210> 643
<211> 17
<212> PRT
<213> glycine max

<400> 643
Gln Gln His Gln Gln Glu Ser Cys Arg Glu Gln Leu Lys Gly Ile Asn
1               5                   10                  15
Leu

<210> 644
<211> 16
<212> PRT
<213> glycine max

<400> 644
Lys Gly Ile Asn Leu Asn Pro Cys Glu His Ile Met Glu Lys Ile Gln
1               5                   10                  15

<210> 645
<211> 16
<212> PRT
<213> glycine max

<400> 645
Cys Glu His Ile Met Glu Lys Ile Gln Ala Gly Arg Arg Gly Glu Asp
1               5                   10                  15

<210> 646
<211> 17
<212> PRT
<213> glycine max

<400> 646
Gly Arg Arg Gly Glu Asp Gly Ser Asp Glu Asp His Ile Leu Ile Arg

1               5                   10                  15
Thr


<210> 647
<211> 15
<212> PRT
<213> glycine max

<400> 647
Tyr Ile Arg Lys Lys Glu Gly Lys Glu Glu Glu Glu Glu Gly His
1               5                   10                  15

<210> 648
<211> 16
<212> PRT
<213> glycine max

<400> 648
Arg Lys Lys Glu Gly Lys Glu Glu Glu Glu Glu Gly His Met Gln Lys
1               5                   10                  15



<210> 649
<211> 15
<212> PRT
<213> glycine max

<400> 649
Met Gln Lys Cys Cys Ser Glu Met Ser Glu Leu Lys Ser Pro Ile
1               5                   10                  15

<210> 650
<211> 17
<212> PRT
<213> glycine max

<400> 650
Met Ser Glu Leu Lys Ser Pro Ile Cys Gln Cys Lys Ala Leu Gln Lys
1               5                   10                  15
Ile


<210> 651
<211> 18
<212> PRT
<213> glycine max

<400> 651
Gln Lys Ile Met Asp Asn Gln Ser Glu Gln Leu Glu Gly Lys Glu Lys
1               5                   10                  15
Lys Gln


<210> 652
<211> 16
<212> PRT
<213> glycine max

<400> 652
Glu Leu Met Asn Leu Ala Ile Arg Cys Arg Leu Gly Pro Met Ile Gly
1               5                   10                  15

<210> 653
<211> 14
<212> PRT
<213> glycine max

<400> 653
Arg Leu Gly Pro Met Ile Gly Cys Asp Leu Ser Ser Asp Asp
1               5                   10

<210> 654
<211> 15
<212> PRT
<213> glycine max

<400> 654
Glu Thr Ala Thr Asn Ile Gly Ala Ser Ala Lys Ala Gly Met Glu
1               5                   10                  15

<210> 655
<211> 18
<212> PRT
<213> glycine max

<400> 655
Met Glu Lys Thr Lys Ala Thr Val Gln Glu Lys Ala Glu Arg Met Thr
1               5                   10                  15
Ala Arg

<210> 656
<211> 16
<212> PRT
<213> glycine max

<400> 656
Ala Glu Arg Met Thr Ala Arg Asp Pro Val Gln Lys Glu Leu Ala Thr
1               5                   10                  15

<210> 657
<211> 16
<212> PRT
<213> glycine max

<400> 657
Asp Pro Val Gln Lys Glu Leu Ala Thr Gln Lys Lys Glu Ala Lys Met
1               5                   10                  15

<210> 658
<211> 15
<212> PRT
<213> glycine max

<400> 658
Lys Gln Ala Ala Arg Gln His Asn Thr Ala Ala Lys Gln Ser Ala
1               5                   10                  15

<210> 659
<211> 16
<212> PRT
<213> glycine max

<400> 659
Thr Ala Gly His Met Gly His Gly His His Thr Thr Gly Thr Gly Thr
1               5                   10                  15

<210> 660
<211> 16
<212> PRT
<213> glycine max

<400> 660
Thr Tyr Ser Thr Thr Gly Glu Tyr Gly Gln Pro Met Gly Ala His Gln
1               5                   10                  15

<210> 661
<211> 15
<212> PRT
<213> glycine max

<400> 661
Gln Pro Met Gly Ala His Gln Thr Ser Ala Met Pro Gly His Gly
1               5                   10                  15

<210> 662
<211> 17
<212> PRT
<213> glycine max

<400> 662
Ser Ala Met Pro Gly His Gly Thr Gly Gln Pro Thr Gly His Val Thr
1               5                   10                  15
Glu

<210> 663
<211> 16
<212> PRT
<213> glycine max

<400> 663
Gly Gln Pro Thr Gly His Val Thr Glu Gly Val Val Gly Ser His Pro
1               5                   10                  15

<210> 664
<211> 17
<212> PRT
<213> glycine max

<400> 664
Val Thr Glu Gly Val Val Gly Ser His Pro Ile Gly Thr Asn Arg Gly
1               5                   10                  15
Pro

<210> 665
<211> 18
<212> PRT
<213> glycine max

<400> 665
Glu Ala Ala Asp Cys Asn Gly Ala Cys Ser Pro Phe Glu Met Pro Pro
1               5                   10                  15
Cys Arg

<210> 666
<211> 17
<212> PRT
<213> glycine max

<400> 666
Ser Pro Phe Glu Met Pro Pro Cys Arg Ser Arg Asp Cys Arg Cys Val
1               5                   10                  15
Pro

<210> 667
<211> 15
<212> PRT
<213> glycine max

<400> 667
Pro Pro Cys Arg Ser Arg Asp Cys Arg Cys Val Pro Ile Gly Leu
1               5                   10                  15

<210> 668
<211> 17
<212> PRT
<213> glycine max

<400> 668
Arg Ser Arg Asp Cys Arg Cys Val Pro Ile Gly Leu Val Ala Gly Phe
1               5                   10                  15
Cys

<210> 669
<211> 16
<212> PRT
<213> glycine max

<400> 669
Pro Ile Gly Leu Val Ala Gly Phe Cys Ile His Pro Thr Gly Leu Ser
1               5                   10                  15

<210> 670
<211> 16
<212> PRT
<213> glycine max

<400> 670
Cys Ile His Pro Thr Gly Leu Ser Ser Val Ala Lys Met Ile Asp Glu
1               5                   10                  15

<210> 671
<211> 18
<212> PRT
<213> glycine max

<400> 671
Lys Met Ile Asp Glu His Pro Asn Leu Cys Gln Ser Asp Asp Glu Cys
1               5                   10                  15
Met Lys

<210> 672
<211> 16
<212> PRT
<213> glycine max

<400> 672
His Pro Asn Leu Cys Gln Ser Asp Asp Glu Cys Met Lys Lys Gly Ser
1               5                   10                  15

<210> 673
<211> 18
<212> PRT
<213> glycine max

<400> 673
Met Lys Lys Gly Ser Gly Asn Phe Cys Ala Arg Tyr Pro Asn Asn Tyr
1               5                   10                  15
Ile Asp

<210> 674
<211> 16
<212> PRT
<213> glycine max

<400> 674
Ser Pro Phe Glu Val Pro Pro Cys Arg Ser Ser Asp Cys Arg Cys Val
1               5                   10                  15

<210> 675
<211> 15
<212> PRT
<213> glycine max

<400> 675
Ser Val Ala Lys Met Val Asp Glu His Pro Asn Leu Cys Gln Ser
1               5                   10                  15

<210> 676
<211> 15
<212> PRT
<213> glycine max

<400> 676
Gly Trp Cys Phe Asp Ser Asp Ser Glu Ala Leu Lys Gly Phe Leu
1               5                   10                  15

<210> 677
<211> 16
<212> PRT
<213> glycine max

<400> 677
Met Gln Glu Gly Thr Ser Ser Ala Lys Leu Thr Thr His Leu Asn Lys
1               5                   10                  15

<210> 678
<211> 16
<212> PRT
<213> glycine max

<400> 678
Arg Ser Ser Asp Cys Arg Cys Val Pro Ile Ala Leu Phe Val Gly Phe
1               5                   10                  15

<210> 679
<211> 15
<212> PRT
<213> glycine max

<400> 679
Glu Ile His Val Glu Lys His Arg Val Pro Lys Met Ala Thr His
1               5                   10                  15

<210> 680
<211> 15
<212> PRT
<213> glycine max

<400> 680
Glu Ile His Val Glu Lys His Arg Val Pro Lys Met Ala Thr His
1               5                   10                  15

<210> 681
<211> 15
<212> PRT
<213> glycine max

<400> 681
Lys Asp His Ala Gly Lys Ala Met Gly Asp Ile Gly Gly Arg Gly
1               5                   10                  15

<210> 682
<211> 15
<212> PRT
<213> glycine max

<400> 682
His Ala Ala Ala Asn Val Val Gly Asn Lys Glu Ser Gln Arg Glu
1               5                   10                  15

<210> 683
<211> 15
<212> PRT
<213> glycine max

<400> 683
His Ala Ala Ala Asn Val Val Gly Asn Lys Glu Ser Gln Arg Glu
1               5                   10                  15

<210> 684
<211> 15
<212> PRT
<213> glycine max

<400> 684
Glu Ser Gly Gly Gln Val Val Ala Glu Lys Gly Arg Glu Thr Glu
1               5                   10                  15

<210> 685
<211> 15
<212> PRT
<213> glycine max

<400> 685
Glu Ser Gly Gly Gln Val Val Ala Glu Lys Gly Arg Glu Thr Glu
1               5                   10                  15

<210> 686
<211> 15
<212> PRT
<213> glycine max

<400> 686
Ala Ala Ala His Val Val Glu Gly Ala Ala Gly Tyr Ala Gly His
1               5                   10                  15

<210> 687
<211> 15
<212> PRT
<213> glycine max

<400> 687
Glu Tyr Thr Ala Lys Lys Lys Glu Glu Ala Gln Arg Glu Leu Glu
1               5                   10                  15

<210> 688
<211> 15
<212> PRT
<213> glycine max

<400> 688
Glu Tyr Thr Ala Lys Lys Lys Glu Glu Ala Gln Arg Glu Leu Glu
1               5                   10                  15

<210> 689
<211> 15
<212> PRT
<213> glycine max

<400> 689
Gln Pro Gln Glu Ala Glu Glu Arg Pro Ser Glu Gly Ile Gly Glu
1               5                   10                  15

<210> 690
<211> 15
<212> PRT
<213> glycine max

<400> 690
Asn Thr Met Gly Gly Glu Ser Glu Gly Gly Gly Gly Lys Glu Glu
1               5                   10                  15

<210> 691
<211> 15
<212> PRT
<213> glycine max

<400> 691
Val Leu Glu Thr Arg Val Thr Gly Arg Ala Lys His Glu Glu Gly
1               5                   10                  15

<210> 692
<211> 15
<212> PRT
<213> glycine max

<400> 692
Gly Leu Ala Phe Phe Leu Ala Pro Ile Asp Thr Lys Pro Gln Thr
1               5                   10                  15

<210> 693
<211> 15
<212> PRT
<213> glycine max

<400> 693
Gly Leu Ala Phe Phe Leu Ala Pro Ile Asp Thr Lys Pro Gln Thr
1               5                   10                  15

<210> 694
<211> 15
<212> PRT
<213> glycine max

<400> 694
Phe Leu Ala Pro Ile Asp Thr Lys Pro Gln Thr His Ala Gly Tyr
1               5                   10                  15

<210> 695
<211> 15
<212> PRT
<213> glycine max

<400> 695
Phe Asp Thr Phe Arg Asn Ser Trp Asp Pro Pro Asn Pro His Ile
1               5                   10                  15

<210> 696
<211> 15
<212> PRT
<213> glycine max

<400> 696
Phe Asp Thr Phe Arg Asn Ser Trp Asp Pro Pro Asn Pro His Ile
1               5                   10                  15

<210> 697
<211> 15
<212> PRT
<213> glycine max

<400> 697
Ala Ser Asn Leu Pro His Ala Ser Ser Asn Ile Asp Pro Leu Asp
1               5                   10                  15

<210> 698
<211> 18
<212> PRT
<213> glycine max

<400> 698
Gly Gly Tyr Val Pro Cys Arg Gln Glu Glu Asp Glu Glu Leu His His
1               5                   10                  15
Lys Cys


<210> 699
<211> 15
<212> PRT
<213> glycine max

<400> 699
Gly Val Ile Thr Gln Val Lys Tyr Gln Gly Gly Cys Gly Arg Gly
1               5                   10                  15

<210> 700
<211> 15
<212> PRT
<213> glycine max

<400> 700
Ala Phe Gly Gly Ile Arg Ala Ala Pro Thr Gly Asn Glu Arg Cys
1               5                   10                  15

<210> 701
<211> 15
<212> PRT
<213> glycine max

<400> 701
Ala Phe Gly Gly Ile Arg Ala Ala Pro Thr Gly Asn Glu Arg Cys
1               5                   10                  15

<210> 702
<211> 15
<212> PRT
<213> glycine max

<400> 702
Ala Val Ile Met Leu Cys Val Gly Ile Pro Thr Glu Trp Ser Val
1               5                   10                  15

<210> 703
<211> 15
<212> PRT
<213> glycine max

<400> 703
Ala Val Ile Met Leu Cys Val Gly Ile Pro Thr Glu Trp Ser Val
1               5                   10                  15

<210> 704
<211> 15

<212> PRT
<213> glycine max

<400> 704
Ile Leu Gly Gly Ser Leu Gly Thr Val Asp Asp Cys Cys Ala Leu
1               5                   10                  15

<210> 705
<211> 15
<212> PRT
<213> glycine max

<400> 705
Ile Leu Gly Gly Ser Leu Gly Thr Val Asp Asp Cys Cys Ala Leu
1               5                   10                  15

<210> 706
<211> 17
<212> PRT
<213> glycine max

<400> 706
Gly Ile Glu Pro Cys Ser Lys Tyr Ser Gln His Gln Gln His Val Gln
1               5                   10                  15
Asn

<210> 707
<211> 17
<212> PRT
<213> glycine max

<400> 707
Gly Glu Ile Met Cys Trp Arg Glu Val Val Thr Gln His Gln Gln His
1               5                   10                  15
Ala

<210> 708
<211> 15
<212> PRT
<213> glycine max

<400> 708
Val Phe Ala Ser Gln Val Val Val Gln Thr Glu Gly Arg Val Cys
1               5                   10                  15

<210> 709
<211> 17
<212> PRT
<213> glycine max

<400> 709
Gly Gly Phe Ala Ser Gln Val Phe Ala Asp Cys Cys Lys Leu Gln Ser
1               5                   10                  15
Asp

<210> 710
<211> 17
<212> PRT
<213> glycine max

<400> 710
Gly Glu Phe Ala Ser Gln Val Glu His Ile Arg Pro Val Leu Cys Ala
1               5                   10                  15
Tyr


<210> 711
<211> 17
<212> PRT
<213> glycine max

<400> 711
Gly Asp Phe Ala Ser Gln Val Asn Gln Leu Asn Trp Phe Val Phe Pro
1               5                   10                  15
Asn


<210> 712
<211> 17
<212> PRT
<213> glycine max

<400> 712
Gly Ile Phe Ala Ser Gln Val Arg Ile Ile Ala Ile His Tyr Pro Asn
1               5                   10                  15
Phe


<210> 713
<211> 17
<212> PRT
<213> glycine max

<400> 713
Gly Gln Phe Ala Ser Gln Val Leu Val Glu Phe Cys Asp Gln Ser Asn
1               5                   10                  15
Cys


<210> 714
<211> 17
<212> PRT
<213> glycine max

<400> 714
Gly Pro Phe Ala Ser Gln Val Leu Lys Lys Leu Cys Ala Gln Tyr Ala
1               5                   10                  15
Phe


<210> 715
<211> 17
<212> PRT
<213> glycine max

<400> 715
Gly Val Phe Ala Ser Gln Val Ser Gln Gly Gln Gln Gln Asp Gln Phe
1               5                   10                  15
Asp


<210> 716
<211> 17

<212> PRT
<213> glycine max

<400> 716
Gly Phe Phe Ser Cys Phe Ala Ser Gln Val Ile Cys Tyr Gly Gly Lys
1               5                   10                  15
Asp


<210> 717
<211> 17
<212> PRT
<213> glycine max

<400> 717
Gly Trp Asp His Cys Glu Ala Ser Gln Val Val Lys Phe Asn Tyr Leu
1               5                   10                  15
Cys


<210> 718
<211> 17
<212> PRT
<213> glycine max

<400> 718
Gly Ile Trp Ala Ser Gln Val Val Trp Lys Phe Gln Thr Gly Pro Tyr
1               5                   10                  15
Phe


<210> 719
<211> 17
<212> PRT
<213> glycine max

<400> 719
Gly His Ser Ala Ser Gln Val Val His Asn Val Arg Glu Gly Phe Ser
1               5                   10                  15
Tyr


<210> 720
<211> 17
<212> PRT
<213> glycine max

<400> 720
Gly Gln Tyr Ala Ser Gln Val Val His Ser Lys Glu His Pro Asp Thr
1               5                   10                  15
Asn


<210> 721
<211> 17
<212> PRT
<213> glycine max

<400> 721
Gly Tyr Asn Met Ser Lys Tyr His Pro Ala Ser Gln Val Val Thr Tyr
1               5                   10                  15
Tyr

<210> 722
<211> 17
<212> PRT
<213> glycine max

<400> 722
Gly Tyr Leu Ala Ser Gln Val Val Ile Tyr Pro Glu Ser Glu Glu Gln
1               5                   10                  15
Phe

<210> 723
<211> 17
<212> PRT
<213> glycine max

<400> 723
Gly Asn Tyr Ala Ser Gln Val Val Gln Val Phe Gly Thr Asn Asp Phe
1               5                   10                  15
Asn

<210> 724
<211> 17
<212> PRT
<213> glycine max

<400> 724
Gly Val Pro Ala Ser Gln Val Val Asp Asn Lys Gln Thr Leu Val Tyr
1               5                   10                  15
Phe

<210> 725
<211> 17
<212> PRT
<213> glycine max

<400> 725
Gly Leu Tyr Ala Ser Gln Val Val Ser Val Arg Tyr Tyr Asp Tyr Ser
1               5                   10                  15
Cys

<210> 726
<211> 17
<212> PRT
<213> glycine max

<400> 726
Gly Gln Pro Ala Ser Gln Val Val Ile Ala Gln Glu Leu Phe Phe Glu
1               5                   10                  15
Cys

<210> 727
<211> 17
<212> PRT
<213> glycine max

<400> 727
Gly Phe Asn Ala Ser Gln Val Val Asn Lys Pro Glu Cys Asp Val Glu

1               5                   10                  15
Tyr


<210> 728
<211> 17
<212> PRT
<213> glycine max

<400> 728
Gly His Arg Ala Ser Gln Val Val Trp Ala Tyr Glu Tyr Ala His His
1               5                   10                  15
Ala


<210> 729
<211> 17
<212> PRT
<213> glycine max

<400> 729
Gly Asp Tyr Ala Ser Gln Val Val Gln Pro Gln Gly Glu Phe Gln Pro
1               5                   10                  15
Cys


<210> 730
<211> 17
<212> PRT
<213> glycine max

<400> 730
Gly Gln Gln Ala Ser Gln Val Val Ile Gln Lys Cys Asp Gln Pro Asp
1               5                   10                  15
Tyr


<210> 731
<211> 17
<212> PRT
<213> glycine max

<400> 731
Gly Val Glu Ala Ser Gln Val Val Ala Phe Gly Ile Cys Lys Gln Val
1               5                   10                  15
Ala


<210> 732
<211> 17
<212> PRT
<213> glycine max

<400> 732
Gly Thr Glu Ala Ser Gln Val Val Val Ser Ala Cys His Gln Cys Ile
1               5                   10                  15
Ile


<210> 733
<211> 17
<212> PRT
<213> glycine max

<400> 733
Gly Trp Asp His Cys Glu Ala Ser Gln Val Val Lys His Val Arg Glu
1               5                   10                  15
Ile